# EUROPÄISCHE PATENTSCHRIFT

(11) **EP 1 590 324 B1**
(45) Veröffentlichungstag und Bekanntmachung des Hinweises auf die Patenterteilung: **11.03.2009**
(21) Anmeldenummer: 04708768.9
(22) Anmeldetag: 06.02.2004
(51) Int. Cl.: C07D 207/16, C07D 521/00, C07D 209/08, C07D 231/56, C07D 207/34, C07D 209/42, C07D 209/12, A61K 31/40, A61K 31/404, A61K 31/415, A61P 7/02, A61P 17/00, A61P 29/00

(54) **NEUE HETEROARYLSUBSTITUIERTE ACETONDERIVATE ALS HEMMSTOFFE DER PHOSPHOLIPASE A sb 2 /sb**
NOVEL HETEROARYL-SUBSTITUTED ACETONE DERIVATIVES AS INHIBITORS OF PHOSPHOLIPASE A sb 2 /sb
NOUVEAUX DERIVES D'ACETONE A SUBSTITUTION HETEROARYLE SERVANT D'INHIBITEURS DE PHOSPHOLIPASE A sb 2 /sb

(30) Priorität: 07.02.2003 DE 10305089
(43) Veröffentlichungstag der Anmeldung: 02.11.2005
(73) Patentinhaber: MERCKLE GMBH, 89143 Blaubeuren (DE)
(72) Erfinder: LEHR, Matthias, 48329 Havixbeck (DE); LUDWIG, Joachim, 34243 Kassel (DE)
(74) Vertreter: Teipel, Stephan
(86) Internationale Anmeldenummer: PCT/EP2004/001128
(87) Internationale Veröffentlichungsnummer: WO 2004/069797

(56) Entgegenhaltungen:
- EP-A- 0 193 853
- WO-A-01/53257
- WO-A-98/05637
- CONNOLLY, STEPHEN ET AL: "Design and Synthesis of a Novel and Potent Series of Inhibitors of Cytosolic Phospholipase A2 Based on a 1,3-Disubstituted Propan -2-one Skeleton" JOURNAL OF MEDICINAL CHEMISTRY ( 2002 ), 45(6), 1348-1362 CODEN: JMCMAR; ISSN: 0022-2623, 2. August 2002 (2002-08-02), XP002287473 in der Anmeldung erwähnt

## Beschreibung

Die vorliegende Erfindung betrifft neuartige heteroarylsubstituierte Acetonderivate, welche das Enzym Phospholipase A₂ hemmen. Diese Verbindungen sind geeignet als Arzneimittel zur Prävention und zur Behandlung von Erkrankungen, die durch eine erhöhte Aktivität dieses Enzyms verursacht bzw. mitverursacht werden, wie z.B. Entzündungen, Schmerz, Fieber, Allergien, Asthma, Psoriasis und Endotoxinschock. Die Erfindung betrifft ferner Methoden zur Synthese dieser Verbindungen sowie pharmazeutische Mittel, die diese Verbindungen enthalten.

Unter der Bezeichnung "Phospholipase A₂" fasst man die große und diverse Gruppe von Enzymen zusammen, die Phospholipide an der *sn*-2-Position unter Bildung von freien Fettsäuren und Lysophospholipiden spalten. Handelt es sich bei der freigesetzten Fettsäure um Arachidonsäure, so kann diese über den Cyclooxygenase-Weg zu den Prostaglandinen und Thromboxanen sowie über die Lipoxygenase-Wege zu den Leukotrienen und anderen hydroxylierten Fettsäuren metabolisiert werden. Die Prostaglandine sind an der Entstehung des Schmerzes und des Fiebers sowie an entzündlichen Reaktionen wesentlich beteiligt. Leukotriene sind wichtige Mediatoren bei Entzündungsprozessen und bei anaphylaktischen und allergischen Vorgängen (Forth. et al., Allgemeine und Spezielle Pharmakologie und Toxikologie Spektrum Akademischer Verlag Heidelberg, Berlin, Oxford, 1998).

Die durch die Phospholipase A₂ gebildeten Lysophospholipide besitzen zellschädigende Eigenschaften. Lysophosphatidylserin führt zur Freisetzung des an allergischen Prozessen beteiligten Histamins (Moreno et al., Agents Actions 1992, 36, 258). Lysophosphatidylcholin wird darüber hinaus zum plättchenaktivierenden Faktor (PAF) metabolisiert, der ebenfalls ein wichtiger Mediator z.B. bei Entzündungen ist.

Verschiedene Phospholipase A₂ - Formen sind bekannt. Zu diesen zählen Ca²⁺-abhängige niedermolekulare sekretorische Phospholipasen A₂ (sPLA₂), Ca²⁺-unabhängige hochmolekulare Phospholipasen A₂ (iPLA₂), Ca²⁺-abhängige hochmolekulare cytosolische Phospholipasen A₂ (cPLA₂) und Ca²⁺-unabhängige Lipoprotein-assozierte Phospholipasen A₂ (LP-PLA₂), früher auch PAF-Acetylhydrolase genannt (Six et al., Biochim. Biophys. Acta. 2000, 1488, 1-19). Nach dem derzeitgen Kenntnisstand spielt die cytosolische Phospholipase A₂ (cPLA₂) die Schlüsselrolle bei der Biosynthese der Prostaglandine, Leukotriene, des PAFs und der Lysophospholipide. Dies wurde unter anderem durch Untersuchungen an cPLA₂ knock-out Mäusen, d.h. Mäusen, die dieses Enzym nicht mehr besitzen, nachgewiesen (Uozumi et al., Nature 1997, 390, 618-622; Bonventre et al., Nature 1997, 390, 622-625, Dennis et al., Journal of Experimental Medicine 2002, 196, 349-357).

Übermäßige Stimulation dieses Enzyms kann deshalb zu einer Reihe von chronischen und akuten Erkrankungen führen, wie Asthma, cerebrale Ischämie (Clemens et. al, Stroke 1996, 27, 527-535), Alzheimersche Erkrankung (Stephenson et al. Neurobiology of Stroke 1996, 3, 51-63), rheumatoide Arthritis (Huang et al., Mediators of Inflammation 1994, 3, 307-308), chronische Hauterkrankungen und Schädigung der Haut durch UV-Strahlen (Gresham et al., American Journal of Physiology 1996, 270 C1037-C1050).

Hemmstoffe der cPLA₂ können deshalb von Nutzen sein bei einer Reihe von entzündlichen Erkrankungen.

In der Literatur wurden bereits Hemmstoffe der cytosolischen Phospholipase A₂ beschrieben (Lehr, Drugs of the Future 2000, 25, 823-832).

So sind 1,3-disubstituierte Propan-2-on-Verbindungen der Firma AstraZeneca bekannt (Connolly et al., Journal of Medicinal Chemistry 2002, 45, 1348-1362). WO 00/34254 offenbart Verbindungen mit Hemmwirkung auf die cytosolische Phospholipase A₂. In den Patentschriften US 6,414,179, US 2002/0037875 und US 2002/0065246 werden Alpha Amino-, Thio- und Oxo-substituierte Ketone sowie alpha- und beta-substituierte Trifluormethylketone als Hemmstoffe der cytosolischen Phospholipase A₂ offenbart. Ferner offenbart EP 976 748 bestimmte Pyrrolidinderivate als Hemmstoffe der cytosolischen Phospholipase A₂.

Es besteht jedoch weiterhin ein Bedürfnis nach neuartigen Verbindungen, die die Phospholipase A₂ und insbesondere die cytosolische Phospholipase A₂ hemmen. Es wurde nun überraschend gefunden, daß bestimmte Heteroaryl-substituierte Acetonderivate diese Aufgabe lösen. Die vorliegende Erfindung betrifft somit Verbindungen der Formel I 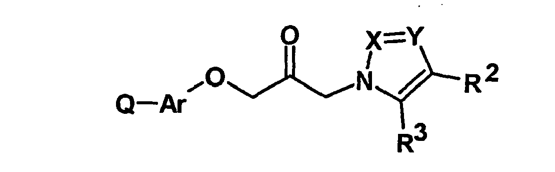 worin
Q für R¹, OR¹, SR¹, SOR¹, SO₂R¹, NR⁹R¹ oder einen geradkettigen C₁₋₃₁-Alkyl- oder C₂₋₃₁-Alkenyl- oder -Alkinylrest steht, der mit 1 oder 2 Resten, unabhängig ausgewählt aus O, S, SO, SO₂, NR⁹ und Aryl, das mit 1 oder 2 substituenten R⁴ substituiert sein kann, unterbrochen sein kann, und der mit 1-4 C₁₋₆Alkylresten und/oder 1 oder 2 Arylresten substituiert sein kann, wobei die Arylreste mit 1 oder 2 Substituenten R⁴ substituiert sein können;
Ar für einen Arylrest steht, der mit 1 oder 2 Substituenten R⁴ substituiert sein kann;
X für N oder CR⁵ steht;
Y für N oder CR⁶ steht;
R¹ für H oder einen Arylrest steht, der mit 1 oder 2 Substituenten R⁴ substituiert sein kann;
R² und R³
   a) unabhängig voneinander für H, C₁₋₆Alkyl, C₂₋₆-Alkenyl, C₂₋₆-Alkinyl oder R⁷-W stehen, oder
   b) zusammen mit den Kohlenstoffatomen, an die sie gebunden sind, für einen 5-oder 6-gliedrigen aromatischen oder heteroaromatischen Ring stehen, der mit 1 oder 2 Substituenten R⁴ substituiert sein kann;
R⁴ für C₁₋₆-Alkyl, Halogen, CF₃, CN, NO₂, OR⁹, S(O)ₒR⁹, COR⁹, COOR⁹, CONR⁹R¹⁰, SO₃R⁹, SO₂NR⁹R¹⁰,Tetrazolyl oder R⁷-W steht;
R⁵ für H oder R⁴ steht;
R⁶ für H, C₁₋₆-Alkyl, Halogen, CF₃, CN, NO₂, OR⁹, SR⁹, COR⁹, COOR⁹, CONR⁹R¹⁰, SO₃R⁹, SO₂NR⁹R¹⁰,Tetrazolyl oder R⁸-W steht;
R⁷ für C₁₋₆-Alkyl, C₂₋₆Alkenyl oder C₂₋₆-Alkinyl steht;
R⁸ für C₂₋₆-Alkyl, C₂₋₆-Alkenyl oder C₂₋₆Alkinyl steht;
R⁹ für H, C₁₋₆-Alkyl oder Aryl steht;
R¹⁹ für H oder C₁₋₆-Alkyl steht;
W für COOH, SO₃H oder Tetrazolyl steht; und
o für 0, 1 oder 2 steht.
sowie deren pharmazeutisch verträglichen Salze und Ester.

Die pharmazeutisch verträglichen Salze können Basenadditionssalze sein. Dazu zählen Salze der Verbindungen mit anorganischen Basen, wie Alkalihydroxiden, Erdalkalihydroxiden oder mit organischen Basen, wie Mono-, Di- oder Triethanolamin. Auch Säureadditionssalze sind umfasst.

Zu den pharmazeutisch verträglichen Estern der Verbindungen zählen insbesondere physiologisch leicht hydrolisierbare Ester, beispielsweise Alkyl-, Pivaloyloxymethyl-, Acetoxymethyl-, Phthalidyl-, Indanyl- und Methoxymethylenester.

Der Ausdruck "Alkyl" umfasst, wenn nicht anders angegeben geradkettige, verzweigte oder cyclische Alkylgruppen, wie Methyl, Ethyl, Propyl, Butyl, Pentyl, Neopentyl, Undecyl, Dodecyl, Pentadecyl, Hexadecyl, Heptadecyl, Octadecyl, Cyclohexyl etc.

Der Ausdruck "Alkenyl" umfasst geradkettige, verzweigte oder cyclische Alkenylgruppen, wie Ethenyl, Propenyl, Butenyl, Decenyl, Heptadecenyl, Cyclohexenyl etc.

Der Ausdruck "Alkinyl" umfasst geradkettige oder verzweigte Alkinylgruppen, wie Ethinyl, Propinyl, Butinyl, Decinyl, Heptadecinyl etc.

Der Ausdruck "Aryl" umfasst Phenyl, Naphthyl, Biphenyl sowie 5- oder 6-gliedrige heterocyclische Ringe, die 1 bis 3 Atome ausgewählt aus O, N oder S enthalten und gegebenenfalls mit einem Benzolring anelliert sind. Bevorzugt werden Phenyl und Indolyl, insbesondere Phenyl.

Der Ausdruck "Halogen" umfasst ein Fluor-, Chlor-, Brom- oder Jodatom, wobei insbesondere Fluor- oder Chloratom bevorzugt sind.

Wenn in einer Verbindung Reste wie R⁴, R⁷, R⁹ und/oder R¹⁰ mehrfach auftreten, können diese jeweils unabhängig voneinander gewählt werden.

Der geradkettige C₁₋₃₁-Alkyl- oder C₂₋₃₁-Alkenyl- oder -Alkinylrest, für den in der Formel I Q steht, kann mit 1 oder 2 Resten, unabhängig ausgewählt aus O, S, SO, SO₂, NR⁹ und Aryl unterbrochen sein. Unter "unterbrochen" wird vorliegend verstanden, daß der Rest, zusätzlich zu den Kohlenstoffatomen seiner Kette sowohl an einer beliebigen Stelle innerhalb der Kette als auch am Ende der Kette, d.h. zwischen der Kohlenstoffkette und Ar einen solchen Rest enthalten kann. Die zusätzlich gegebenenfalls vorhandenen Substituenten in Form von 1-4 C₁₋₆Alkylresten und/oder 1 oder 2 Arylresten können mit jedem beliebigen Kohlenstoffatom der Kette verbunden sein.

Vorzugsweise steht in den vorstehend beschriebenen Verbindungen der Formel I Q für

R¹-(CHR¹¹)ₚ-A-Z²-B-Z¹-

worin A für eine Bindung oder einen geradkettigen C₁₋ₘ-Alkyl- oder C₂₋ₘ-Alkenyl- oder Alkinylrest steht, B für eine Bindung oder einen geradkettigen C₁₋ₙ-Alkyl- oder C₂₋ₙ-Alkenyl- oder -Alkinylrest steht, R¹ und R¹¹ unabhängig voneinander für H oder einen Arylrest stehen, der mit 1 oder 2 Substituenten R⁴ substituiert sein kann, und Z¹ und Z² unabhängig voneinander für eine Bindung, O, S, SO, SO₂, NR⁹, CR⁹R¹⁰ oder einen Arylrest stehen, wobei der Arylrest mit 1 oder 2 Substituenten R⁴ substituiert sein kann p steht hierin für 0 oder 1, m für eine ganze Zahl von 0 bis 12 und n für eine ganze Zahl von 0 bis 16. Vorzugsweise ist die Summe aus m und n nicht größer als 17, besonders bevorzugt nicht größer als 10.

Q ist bevorzugt:

R¹-(CHR¹¹)ₚ(CH₂)ₘ-Z²-(CH₂)ₙ-Z¹-

worin R¹, R¹¹, Z², Z¹, p, m und n wie vorstehend definiert sind.

Besonders bevorzugt ist Q ausgewählt aus:

R¹-B-Z¹-

R¹-CHR¹¹-B-Z¹-

R¹-A-Z²-B-Z¹- und

R¹-CHR¹¹-A-Z²-B-Z¹-,

insbesondere

R¹-(CH₂)ₙ-Z¹-

R¹-CHR¹¹-(CH₂)ₙ-Z¹-

R¹-(CH₂)ₘ-Z²-(CH₂)ₙ-Z¹- und

R¹-CHR¹¹-(CH₂)ₘ -Z²-(CH₂)ₘ-Z¹-.

R¹ und R¹¹ stehen vorzugsweise unabhängig voneinander für H oder einen Phenylrest, insbesondere für einen nicht substituierten Phenylrest. Z¹ und Z² sind, wenn sie für einen Arylrest stehen, vorzugsweise Phenyl, insbesondere nicht substituiertes Phenyl.

In einer bevorzugten Ausführungsform steht Q in den erfindungsgemäßen Verbindungen der Formel I für Phenyl oder C₅₋₁₂-Alkyl oder -Alkoxy, besonders bevorzugt für C₇₋₁₀-Alkyl oder Alkoxy und am bevorzugtesten für C₈-Alkyl.

In den erfindungsgemäßen Verbindungen der Formel I steht Ar für einen Arylrest und vorzugsweise für einen wie vorstehend definierten Arylrest. Besonders bevorzugt steht Ar für einen Phenylrest, der vorzugsweise die benachbarten Gruppen Q und O in paraStellung miteinander verbindet.

Wenn R² und R³ zusammen mit den Kohlenstoffatomen, an die sie gebunden sind, einen 5-oder 6-gliedrigen aromatischen oder heteroaromatischen Ring bilden, handelt es sich hierbei vorzugsweise um einen Benzo-Ring oder einen 6-gliedrigen aromatischen heterocyclischen Ring mit 1-3 Stickstoffatomen. Diese Ringe können mit 1 oder 2 Substituenten R⁴ substituiert sein, wobei ein Substituent R⁴ und insbesondere COOH, CH₃, Cl, OCH₃, CN, CHO, COOCH₃ oder CONH₂ bevorzugt ist.

In dem Heteroarylrest der erfindungsgemäßen Acetonderivate steht Y vorzugsweise für CR⁶. R⁵ und R⁶ werden vorzugsweise aus H, COOH, t-Butyl, Cl, CHO, COCH₃ oder COOCH₃ ausgewählt.

Besonders geeignete Heteroarylreste für die erfindungsgemäßen Acetonderivate sind Pyrrolyl, Pyrazolyl, Indolyl, Indazolyl, Pyrrolyl-2-carbonsäure, Pyrrolyl-3-carbonsäure, Indolyl-2-carbonsäure, Indolyl-3-carbonsäure, Indolyl-4-carbonsäure, Indolyl-5-carbonsäure, Indolyl-6-carbonsäure, 5-Methylindolyl, 5-Chlorindolyl, 5-Methoxyindolyl, Indolyl-5-carbonitril, Indolyl-5-carbaldehyd, Indolyl-5-carbonsäuremethylester, 3-tert-Butylindolyl-5-carbonsäure, 3-Chlorindolyl-5-carbonsäure, 3-Formylindolyl-5-carbonsäure, 3-Acetylindolyl-5-carbonsäure, 3-Methoxycarbonylindolyl-5-carbonsäure, 3-tert-Butylindolyl-6-carbonsäure und Indolyl-5-carbamid.

Die erfindungsgemäßen Verbindungen haben sich als potente Phospholipase A₂-Hemmer erwiesen. Die Verbindungen sind daher brauchbar als Arzneimittel zur Prävention und zur Behandlung von Erkrankungen, die durch Produkte bzw. Folgeprodukte dieses Enzyms verursacht bzw. mitverursacht werden, wie zum Beispiel zur Behandlung des rheumatischen Formenkreises und zur Prävention und Behandlung von allergisch induzierten Erkrankungen. Die erfindungsgemäßen Verbindungen stellen somit u.a. wirksame Analgetika, Antiphlogistika, Antipyretika, Antiallergika und Broncholytika dar und sind zur Thromboseprophylaxe und zur Prophylaxe des anaphylaktischen Schocks sowie zur Behandlung dermatologischer Erkrankungen, wie Psoriasis, Urtikaria, akute und chronische Exantheme allergischer und nicht-allergischer Genese, brauchbar.

Die vorliegende Erfindung betrifft daher auch pharmazeutische Mittel, die eine Verbindung der allgemeinen Formel I oder ein pharmazeutisch verträgliches Salz oder Ester davon umfassen.

Die Verbindungen der Formel I eignen sich insbesondere zur Herstellung eines pharmazeutischen Mittels zur Prävention oder Behandlung von Erkrankungen, die durch eine erhöhte Aktivität der Phospholipase A₂, vorzugsweise der cytosolischen Phospholipase A₂, verursacht oder mitverursacht werden. Hierbei handelt es sich z.B. um Erkrankungen, ausgewählt aus Entzündungen, Schmerz, Fieber, Allergien, Asthma, Psoriasis, cerebrale Ischämie, Alzheimersche Erkrankung, chronische Hauterkrankungen, Schädigung der Haut durch UV-Strahlen, rheumatische Erkrankungen, Thrombose, anaphylaktischer Schock, Urtikuria, akute und chronische Exantheme und Endotoxinschock.

Die erfindungsgemäßen Verbindungen können entweder als einzelne therapeutische Wirkstoffe oder als Mischungen mit anderen therapeutischen Wirkstoffen verabreicht werden. Sie können alleine verabreicht werden, im allgemeinen werden sie jedoch in Form pharmazeutischer Mittel verabreicht, d.h. als Mischungen der Wirkstoffe mit geeigneten pharmazeutischen Trägern oder Verdünnungsmitteln. Die Verbindungen oder Mittel können oral, parenteral, durch Inhalation, rektal oder topisch (einschließlich dermal, transdermal, buccal und sublingual) verabreicht werden.

Die Art des pharmazeutischen Mittels und des pharmazeutischen Trägers bzw. Verdünnungsmittels hängt von der gewünschten Verabreichungsart ab. Orale Mittel können beispielsweise als Tabletten oder Kapseln, auch in retardierter Form, vorliegen und können übliche Exzipienzien enthalten, wie Bindemittel (z.B. Sirup Akazia, Gelatine, Sorbit, Tragant oder Polyvinylpyrrolidon), Füllstoffe (z.B. Lactose, Zucker, Maisstärke, Calciumphosphat, Sorbit oder Glycin), Gleitmittel (z.B. Magnesiumstearat, Talkum, Polyethylenglykol oder Siliciumdioxid), desintegrierende Mittel (z.B. Stärke) oder Netzmittel (z.B. Natriumlaurylsulfat). Orale flüssige Präparate können in Form wässriger oder öliger Suspensionen, Lösungen, Emulsionen, Sirupen, Elixieren oder Sprays usw. vorliegen oder können als Trockenpulver zur Rekonstitution mit Wasser oder einem anderen geeigneten Träger vorliegen. Derartige flüssige Präparate können übliche Additive, beispielsweise Suspendiermittel, Geschmacksstoffe, Verdünnungsmittel oder Emulgatoren enthalten. Für die parenterale Verabreichung kann man Lösungen oder Suspensionen mit üblichen pharmazeutischen Trägern einsetzen. Für die Verabreichung durch Inhalation können die Verbindungen in pulverförmige, wässriger oder teilweise wässriger Lösung vorliegen, die in Form eines Aerosols angewendet werden kann. Mittel für die topische Anwendung können z.B. als pharmazeutisch verträgliche Puder, Lotionen, Salben, Cremes, Gele oder als therapeutische Systeme vorliegen, die therapeutisch wirksame Mengen der erfindungsgemäßen Verbindungen enthalten.

Die erforderliche Dosierung ist abhängig von der Form des angewendeten pharmazeutischen Mittels, von der Art der Anwendung, der Schwere der Symptome und dem speziellen Subjekt (Mensch oder Tier), das behandelt wird. Die Behandlung wird üblicherweise mit einer Dosis begonnen, die unterhalb der optimalen Dosis liegt. Danach wird die Dosis erhöht, bis der für die gegebenen Bedingungen optimale Effekt erzielt wird. Im allgemeinen werden die erfindungsgemäßen Verbindungen am besten in Konzentrationen verabreicht, mit welchen sich effektive Wirkungen erzielen lassen, ohne dass schädliche oder nachteilige Wirkungen auftreten. Sie können in einer Einzeldosis oder in mehreren Dosen verabreicht werden.

Die Wirksamkeit der erfindungsgemäßen Verbindungen lässt sich anhand der Hemmung der cytosolischen Phospholipase A₂ bestimmen. Dazu wird in intakten humanen Thrombocyten die cytosolische Phospholipase A₂ mit Calcium lonophor A23187 stimuliert und dadurch die Freisetzung von Arachidonsäure aus den Membranphospholipiden ausgelöst. Um die Metabolisierung des Enzymproduktes Arachidonsäure über den Cyclooxygenase-Weg und den 12-Lipoxygenase-Weg zu verhindern, wird dabei der duale Cyclooxygenase/12-Lipoxygenase-Inhibitor 5,8,11,14-Eikosatetrainsäure zugesetzt. Nach Reinigung mittels Festphasenextraktion wird die freigesetzte Arachidonsäure durch reversed phase-HPLC mit UV-Detektion bestimmt. Die Hemmung des Enzyms durch eine Testsubstanz ergibt sich aus dem Verhältnis von der in Anwesenheit bzw. in Abwesenheit der Testsubstanz gebildeten Arachidonsäuremengen. Nähere Angaben zum Testsystem erfolgen in dem Beispiel 10.

Die vorliegende Erfindung betrifft ferner ein Verfahren zur Herstellung einer Verbindung der Formel I, worin eine Verbindung der Formel II 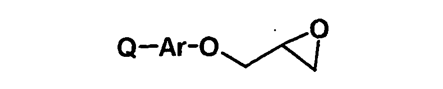 oder eine Verbindung der Formel III 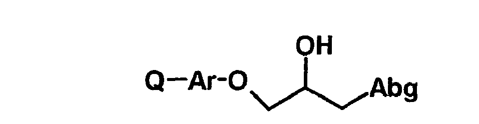 mit einer Verbindung der Formel IV 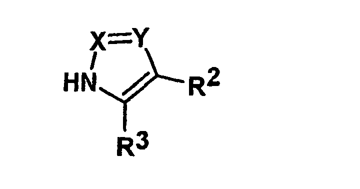 umgesetzt und der gebildete Alkohol zum gewünschten Keton oxidiert wird, wobei Q, Ar, X, Y, R² und R³ wie vorstehend definiert sind und Abg für eine Abgangsgruppe wie Halogen, insbesondere Brom steht.

Die nachfolgenden Beispiele erläutern die Erfindung, ohne sie auf die konkreten Verbindungen einzuschränken.

Alle Ansätze wurden unter Stickstoffschutzgasatmosphäre durchgeführt. Zur säulenchromatographischen Reinigung wurde Kieselgel 60 der Fa. Merck, Darmstadt, Partikelgröße 63-200 µm oder 15-40 µm (= Flash-Chromatographie) verwendet.

### Beispiel 1

### 1-(4-Octylphenoxy)-3(pyrrol-1-yl)propan-2-on

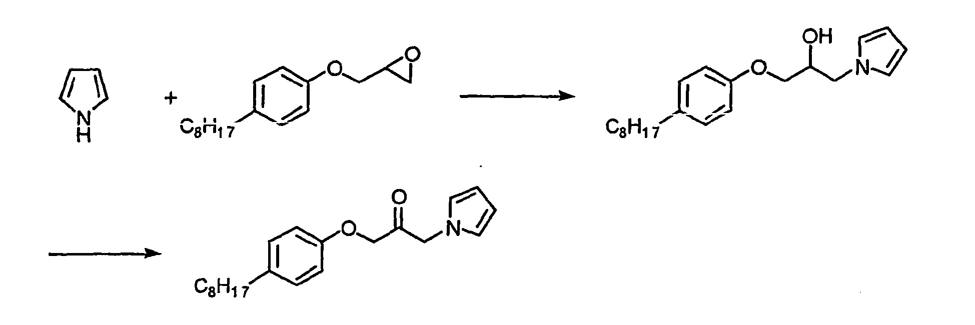

### A. 1-(4-Octylphenoxy)-3-(pyrrol-1-yl)propan-2-ol

0.048 g (1.200 mmol) Natriumhydrid als 60%ige Dispersion in Mineralöl werden in 10 ml absol. DMF suspendiert, 10 min bei Raumtemperatur gerührt und mit 0.077 g (1.15 mmol) Pyrrol versetzt. Nach einstündigem Rühren wird eine Lösung von 0.300 g (1.14 mmol) 2-(4-Octylphenoxymethyl)oxiran (Kuliev et al. Uch. Zap. Azerb. Gos. Univ. Ser. Khim Nauk. 1964, 4, 97; Chem. Abstr. 1966, 65, 640c) in 10 ml absol. DMF hinzugetropft. Es wird neunzehn Stunden gerührt, mit halbgesättigter NaCl-Lösung hydrolysiert und viermal mit Diethylether extrahiert. Die vereinigten organischen Phasen werden am Rotationsverdampfer auf die Hälfte des Volumens eingeengt und dreimal mit gesättigter NaCl-Lösung gewaschen. Trocknen über Natriumsulfat, Filtrieren und erneutes Einengen am Rotationsverdampfer hinterlassen als Rohprodukt ein rotes Öl, das, säulenchromatographisch an Kieselgel gereinigt (Fließmittel: Petrolether/Ethylacetat 95:5), das Produkt in Form eines gelblichen Öls liefert.
Ausbeute: 0.229 g (0.695 mmol); 61%
C₂₁H₃₁NO₂ (329.5)
¹H-NMR (CDCl₃): δ (ppm) = 0.89 (t, J = 7 Hz, 3H), 1.31 (m, 10H), 1.58 (m, 2H), 2.55 (t, J = 8 Hz, 2H), 3.83-3.92 (m, 2H), 4.06-4.24 (m, 3H), 6.17 (t, J = 2 Hz, 2H), 6.70 (t, J = 2 Hz, 2H), 6.82 (d, J = 9 Hz, 2H), 7.10 (d, J = 9 Hz, 2H)

### B. 1-(4-Octylphenoxy)-3-(pyrrol-1-yl)propan-2-on

1.24 g (12.1 mmol) Essigsäureanhydrid werden mit 10 ml absol. DMSO versetzt, 10 min bei Raumtemperatur gerührt und zu einer Lösung von 0.100 g (0.304 mmol) 1-(4-Octylphenoxy)-3-(pyrrol-1-yl)propan-2-ol in 10 ml absol. DMSO getropft. Nach neunzehnstündigem Rühren wird in eine Mischung aus 5%iger Natriumhydrogencarbonat- und gesättigter NaCl-Lösung (1:1, v/v) gegossen und 10 min lang hydrolysiert. Viermalige Extraktion mit Diethylether, Vereinigung der organischen Phasen, Einengen am Rotationsverdampfer auf die Hälfte des Volumens, dreimaliges Waschen mit gesättigter NaCl-Lösung, Trocknen über Natriumsulfat, Filtrieren und erneutes Einengen am Rotationsverdampfer hinterlassen ein bräunliches Öl, das, säulenchromatographisch an Kieselgel gereinigt (Fließmittel: Petrolether/Ethylacetat 97:3), das Produkt in Form eines weißen Feststoffes liefert.
Ausbeute: 0.087 g (0.266 mmol); 88%
Schmp.: 58-59 °C
C₂₁H₂₉NO₂ (327.5)
¹H-NMR (CDCl₃): δ (ppm) = 0.89 (t, J = 7 Hz, 3H), 1.26 (m, 10H), 1.58 (m, 2H), 2.56 (t, J = 8 Hz, 2H), 4.59 (s, 2H), 4.97 (s, 2H), 6.24 (t, J = 2 Hz, 2H), 6.61 (t, J = 2 Hz, 2H), 6.80 (d, J = 9 Hz, 2H), 7.12 (d, J = 9 Hz, 2H)

### Beispiel 2

### 1-(4-Octylphenoxy)-3-(pyrazol-1-yl)propan-2-on

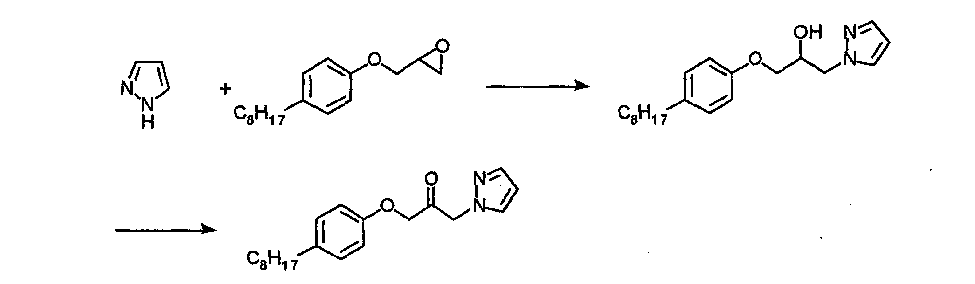

### A. 1-(4-Octylphenoxy)-3-(pyrazol-1-yl)propan-2-ol

0.091 g (3.96 mmol) Natrium werden mit 5 ml absol. THF und mit einer Lösung von 0.268 g (3.94 mmol) Pyrazol in 15 absol. THF versetzt. Nach zweistündigem Rühren bei Raumtemperatur wird eine Lösung von 0.690 g (2.63 mmol) 2-(4-Octylphenoxymethyl)oxiran in 10 ml THF hinzugetropft und acht Stunden lang unter Rückfluss erhitzt. Die abgekühlte Reaktionsmischung wird in halbgesättigte NaCl-Lösung gegossen und viermal mit Diethylether extrahiert. Die vereinigten organischen Phasen werden am Rotationsverdampfer auf die Hälfte des Volumens eingeengt. Waschen mit halbgesättigter NaCl-Lösung, Trocknen über Natriumsulfat, Filtrieren und erneutes Einengen am Rotationsverdampfer hinterlassen als Rohprodukt einen gelben Feststoff, der, säulenchromatographisch an Kieselgel gereinigt (Fließmittel: Petrolether/Ethylacetat 85:15), und umkristallisiert aus Petrolether, das Produkt in Form eines weißen Feststoffes liefert.
Ausbeute: 0.716 g (2.17 mmol); 82%
Schmp.: 88 °C
C₂₀H₃₀N₂O₂ (330.5)
¹H-NMR (CDCl₃): δ (ppm) = 0.89 (t, J = 6 Hz, 3H), 1.28 (m, 10H), 1.58 (m, 2H), 2.55 (t, J = 8 Hz, 2H), 3.71-3.78 (m, 1H), 3.92-4.02 (m, 2H), 4.23-4.48 (m, 3H), 6.27 (t, J = 2 Hz, 1H), 6.83 (d, J = 9 Hz, 2H), 7.09 (d, J = 9 Hz, 2H), 7.45 (d, J = 2 Hz, 1H), 7.55 (d, J = 2 Hz, 1 H)

### B. 1-(4-Octylphenoxy)-3-(pyrazol-1-yl)propan-2-on

0.154 g (1.51 mmol) Essigsäureanhydrid werden mit 5 ml absol. DMSO versetzt, 10 min bei Raumtemperatur gerührt und zu einer Lösung von 0.050 g (0.152 mmol) 1-(4-Octylphenoxy)-3-(pyrazol-1-yl)propan-2-ol in 10 ml absol. DMSO getropft. Nach achtzehnstündigem Rühren bei Raumtemperatur wird in eine Mischung aus 5%iger Natriumhydrogencarbonat- und gesättigter NaCl-Lösung (1:1, v/v) gegossen und 10 min lang hydrolysiert. Viermalige Extraktion mit Diethylether, Vereinigung der organischen Phasen, Einengen am Rotationsverdampfer auf die Hälfte des Volumens, dreimaliges Waschen mit gesättigter NaCl-Lösung, Trocknen über Natriumsulfat, Filtrieren und erneutes Einengen am Rotationsverdampfer hinterlassen als Rohprodukt einen gelblichen Feststoff, der, säulenchromatographisch an Kieselgel gereinigt (Fließmittel: Petrolether/Ethylacetat 90:10), das Produkt in Form eines weißen Feststoffes liefert.
Ausbeute: 0.048 g (0.146 mmol); 96%
Schmp.: 85-87 °C
C₂₀H₂₈N₂O₂ (328.5)
¹H-NMR (CDCl₃): δ (ppm) = 0.88 (t, J = 7 Hz, 3H), 1.28 (m, 10H), 1.57 (m, 2H), 2.55 (t, J = 8 Hz, 2H), 4.63 (s, 2H), 5.26 (s, 2H), 6.36 (t, J = 2 Hz, 1H), 6.81 (d, J = 9 Hz, 2H), 7.11 (d, J = 9 Hz, 2H), 7.43 (d, J = 2 Hz, 1 H), 7.59 (d, J = 2 Hz, 1 H)

### Beispiel 3

### 1-(Indol-1-yl)-3-(4-octylphenoxy)propan-2-on

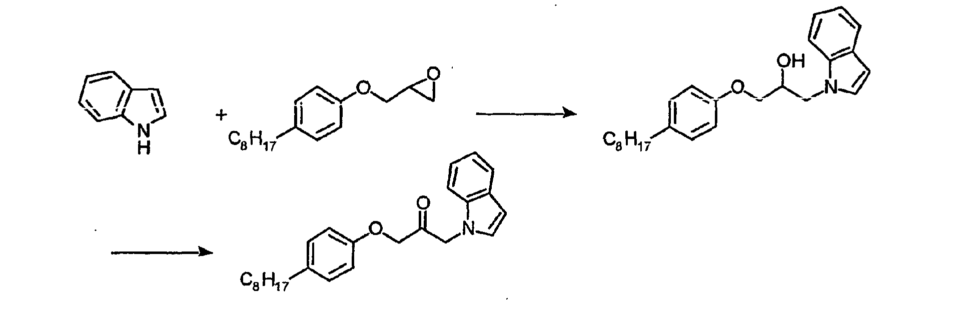

### A. 1-(Indol-1-yl)-3-(4-octylphenoxy)propan-2-ol

0.048 g (1.20 mmol) Natriumhydrid als 60%ige Dispersion in Mineralöl werden in 10 ml absol. DMF suspendiert, 10 min bei Raumtemperatur gerührt und mit einer Lösung von 0.134 g (1.14 mmol) Indol in 10 ml absol. DMF versetzt. Nach eineinhalbstündigem Rühren wird eine Lösung von 0.300 g (1.14 mmol) 2-(4-Octylphenoxymethyl)oxiran in 10 ml absol. DMF hinzugetropft. Es wird sechzehn Stunden gerührt, mit halbgesättigter NaCl-Lösung hydrolysiert und viermal mit Diethylether extrahiert. Die vereinigten organischen Phasen werden am Rotationsverdampfer auf die Hälfte des Volumens eingeengt und dreimal mit gesättigter NaCl-Lösung gewaschen. Trocknen über Natriumsulfat, Filtrieren und erneutes Einengen am Rotationsverdampfer hinterlassen als Rohprodukt ein oranges Öl, das, säulenchromatographisch an Kieselgel gereinigt (Fließmittel: Petrolether/Ethylacetat 95:5), das Produkt in Form eines gelben Öls liefert.
Ausbeute: 0.356 g (0.938 mmol); 82%
C₂₅H₃₃NO₂ (379.5)
¹H-NMR (CDCl₃): δ (ppm) = 0.93 (t, J = 7 Hz, 3H), 1.31 (m, 10H), 1.62 (m, 2H), 2.44 (s breit, 1H), 2.58 (t, J = 8 Hz, 2H), 3.84-3.94 (m, 2H), 4.29-4.45 (m, 3H), 6.52 (d, J = 3 Hz, 1H), 6.82 (d, J = 9 Hz, 2H), 7.11 (d, J = 9 Hz, 2H), 7.13-7.15 (m, 2H), 7.20-7.25 (m, 1H), 7.40 (d, J = 8 Hz, 1 H), 7.64 (d, J = 8 Hz, 1 H)

### B. 1-(indol-1-yl)-3-(4-octylphenoxy)propan-2-on

1.77 g (17.3 mmol) Essigsäureanhydrid werden mit 10 ml absol. DMSO versetzt, 10 min bei Raumtemperatur gerührt und zu einer Lösung von 0.164 g (0.432 mmol) 1-(Indol-1-yl)-3-(4-octylphenoxy)propan-2-ol in 10 ml absol. DMSO getropft. Nach sechsstündigem Rühren wird in eine Mischung aus 5%iger Natriumhydrogencarbonat- und gesättigter NaCl-Lösung (1:1, v/v) gegossen und 10 min lang hydrolysiert. Viermalige Extraktion mit Diethylether, Vereinigung der organischen Phasen, Einengen am Rotationsverdampfer auf die Hälfte des Volumens, dreimaliges Waschen mit gesättigter NaCl-Lösung, Trocknen über Natriumsulfat, Filtrieren und erneutes Einengen am Rotationsverdampfer hinterlassen als Rohprodukt ein gelbes Öl, das, säulenchromatographisch an Kieselgel gereinigt (Fließmittel: Petrolether/Ethylacetat 95:5) und aus Petrolether umkristallisiert, das Produkt in Form eines weißen Feststoffes liefert.
Ausbeute: 0.106 g (0.281 mmol); 65%
Schmp.: 65 °C
C₂₅H₃₁NO₂ (377.5)
¹H-NMR (CDCl₃): δ (ppm) = 0.90 (t, J = 7 Hz, 3H), 1.30 (m, 10H), 1.59 (m, 2H), 2.57 (t, J = 8 Hz, 2H), 4.59 (s, 2H), 5.16 (s, 2H), 6.60 (d, J = 3 Hz, 1H). 6.81 (d, J = 9 Hz, 2H), 7.05 (d, J = 3 Hz, 1H), 7.12-7.16 (m, 4H), 7.19-7.23 (m, 1H), 7.65 (d, J = 6 Hz, 1 H)

### Beispiel 4

### 1-(Indazol-1-yl)-3-(4-octylphenoxy)propan-2-on

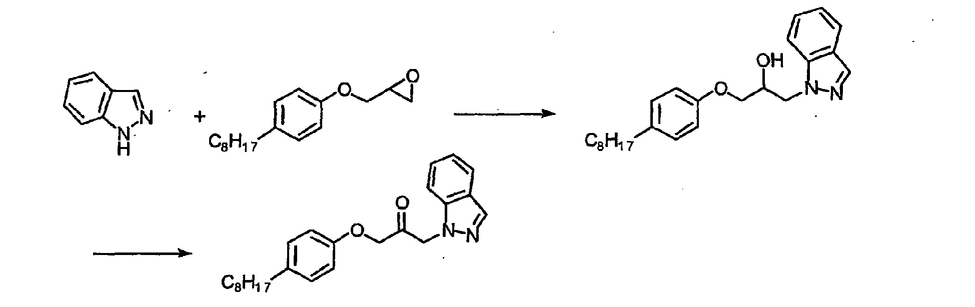

### A. 1-(Indazol-1-yl)-3-(4-octylphenoxy)propan-2-ol

0.048 g (1.20 mmol) Natriumhydrid als 60%ige Dispersion in Mineralöl werden in 10 ml absol. DMF suspendiert, 10 min bei Raumtemperatur gerührt und mit einer Lösung von 0.135 g (1.14 mmol) Indazol versetzt. Nach einstündigem Rühren wird eine Lösung von 0.300 g (1.14 mmol) 2-(4-Octylphenoxymethyl)oxiran in 10 ml absol. DMF hinzugetropft. Es wird vierzig Stunden gerührt, mit halbgesättigter NaCl- Lösung hydrolysiert und viermal mit Diethylether extrahiert. Die vereinigten organischen Phasen werden am Rotationsverdampfer auf die Hälfte des Volumens eingeengt und dreimal mit gesättigter NaCl-Lösung gewaschen. Trocknen über Natriumsulfat, Filtrieren und erneutes Einengen am Rotationsverdampfer hinterlassen einen weißen Feststoff, der aus Petrolether/Ethylacetat (80:20) umkristallisiert wird.

### B. 1-(Indazol-1-yl)-3-(4-octylphenoxy)propan-2-on

1.07 g (10.5 mmol) Essigsäureanhydrid werden mit 10 ml absol. DMSO versetzt, 10 min bei Raumtemperatur gerührt und zu einer Lösung von 0.100 g (0.263 mmol) 1-(Indazol-1-yl)-3-(4-octylphenoxy)propan-2-ol in 10 ml absol. DMSO getropft. Nach zwanzigstündigem Rühren wird in eine Mischung aus 5%iger Natriumhydrogencarbonat- und gesättigter NaCl-Lösung (1:1, v/v) gegossen und 10 min lang hydrolysiert. Viermalige Extraktion mit Diethylether, Vereinigung der organischen Phasen, Einengen am Rotationsverdampfer auf die Hälfte des Volumens, dreimaliges Waschen mit gesättigter NaCl-Lösung, Trocknen über Natriumsulfat, Filtrieren und erneutes Einengen am Rotationsverdampfer hinterlassen als Rohprodukt ein gelbes Öl, das, säulenchromatographisch an Kieselgel gereinigt (Fließmittel: Petrolether/Ethylacetat 90:10), das Produkt in Form eines weißen Feststoffes liefert.
Ausbeute: 0.062 g (0.164 mmol)
Schmp.: 66-67 °C
C₂₄H₃₀N₂O₂ (378.5)
¹H-NMR (CDCl₃): δ (ppm) = 0.88 (t, J = 7 Hz, 3H), 1.26 (m, 10H), 1.57 (m, 2H), 2.55 (t, J = 8 Hz, 2H), 4.63 (s, 2H), 5.46 (s, 2H), 6.79 (d, J = 9 Hz, 2H), 7.11 (d, J = 9 Hz, 2H), 7.16-7.20 (m, 2H), 7.37-7.41 (m, 1 H), 7.75-7.77 (m, 1 H), 8.08 (d, J = 1 Hz, 1 H)

### Beispiel 5

### 1-[3-(4-Octylphenoxy)-2-oxopropyl]pyrrol-2-carbonsäure

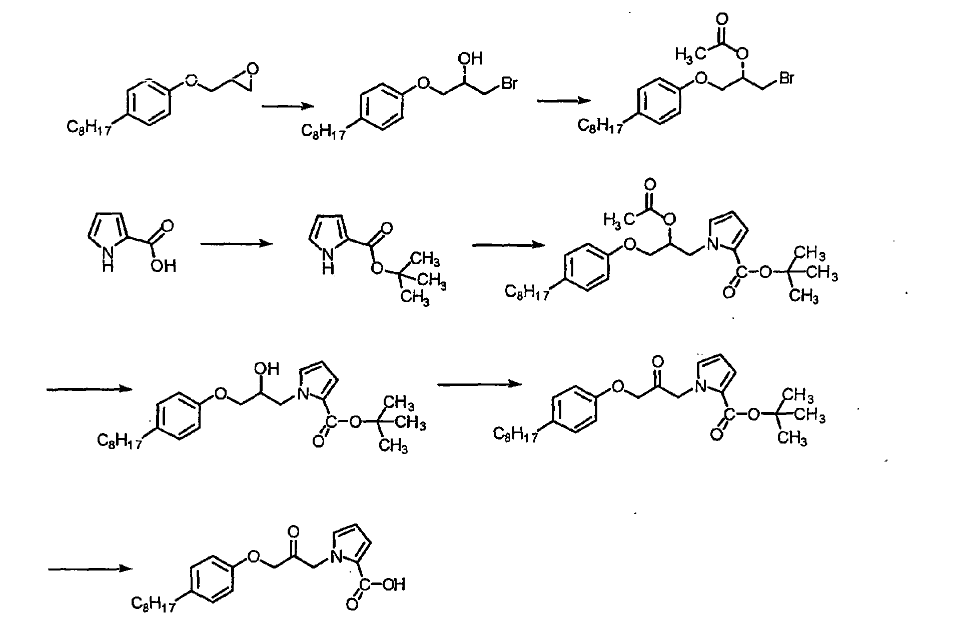

### A. 1-Brom-3-(4-octylphenoxy)propan-2-ol

1.17 g (4.46 mmol) 2-(4-Octyl-phenoxymethyl)oxiran werden in 10 ml absol. Dichlormethan gelöst und mit 1.34 g (22.3 mol) Kieselgel sowie mit 1.16 g (13.4 mmol) Lithiumbromid versetzt. Die Suspension wird am Rotationsverdampfer bis fast zur Trockne eingeengt und drei Stunden bei Raumtemperatur stehen gelassen. Der Reaktionsansatz wird mit Dichlormethan versetzt, über Watte filtriert und am Rotationsverdampfer eingeengt. Aufnehmen der Suspension in Diethylether, Waschen mit Wasser, Trocknen über Natriumsulfat, Filtrieren und erneutes Einengen am Rotationsverdampfer liefern das Produkt in Form eines gelblichen Öls.
Ausbeute: 1.52 g (4.42 mmol); 99%
C₁₇H₂₇BrO₂ (343.3)
¹H-NMR (CDCl₃): δ (ppm) = 0.89 (t, J = 6 Hz, 3H), 1.28 (m, 10H), 1.58 (m, 2H), 2.55 (t, J = 8 Hz, 2H), 3.55-3.71 (m, 2H), 4.04-4.21 (m, 3H), 6.84 (d, J = 9 Hz, 2H), 7.11 (d, J = 9 Hz, 2H)

### B. [1-Brom-3-(4-octylphenoxy)propan-2-yl]acetat

0.41 g (5.18 mmol) absol. Pyridin werden in 10 ml absol. Dichormethan gelöst, auf 0°C gekühlt und mit 0.41 g (5.22 mmol) Acetylchlorid versetzt, wobei *N*-Acetylpyridiniumchlorid ausfällt. Nach dreißigminütigem Rühren werden 0.50 g (1.46 mmol) 1-Brom-3-(4-octylphenoxy)propan-2-ol in 10 ml absol. Dichlormethan gelöst und hinzugetropft. Der Reaktionsansatz wird weitere drei Stunden bei 0°C gerührt, mit Dichlormethan verdünnt und zweimal mit 5%iger Natriumhydrogencarbonat-Lösung gewaschen. Die vereinigten wässrigen Phasen werden noch einmal mit Dichlormethan extrahiert, die vereinigten organischen Phasen mit Wasser gewaschen. Nach Trocknen über Natriumsulfat, Filtrieren, Einengen am Rotationsverdampfer und dreimaligem Versetzen mit Toluol sowie jeweiligem Einengen am Rotationsverdampfer wird als Rohprodukt ein gelbes Öl erhalten, das, säulenchromatographisch an Kieselgel gereinigt (Fließmittel: Petrolether/Ethylacetat 95:5), das Produkt in Form eines gelblichen Öls liefert.
Ausbeute: 0.56 g (1.45 mmol); 100%
C₁₉H₂₉BrO₃ (385.3)
¹H-NMR (CDCl₃): δ (ppm) = 0.89 (t, J = 7 Hz, 3H), 1.28 (m, 10H), 1.59 (m, 2H), 2.13 (s, 3H), 2.55 (t, J = 8 Hz, 2H), 3.59-3.76 (m, 2H), 4.11-4.18 (m, 2H), 5.31 (m, 1 H), 6.86 (d, J = 9 Hz, 2H), 7.10 (d, J = 9 Hz, 2H)

### C. tert-Butylpyrrol-2-carboxylat

0.50 g (4.50 mmol) Pyrrol-2-carbonsäure werden in 15 ml absol. Benzol suspendiert und unter Rückfluss erhitzt. Eine Lösung von 4.07 g (18.0 mmol) 90%igem *N,N-*Dimethylformamiddi-*tert*-butylacetal in 15 ml absol. Benzol wird innerhalb von 30 min hinzugetropft. Nach erneutem dreißigminütigem Erhitzen unter Rückfluss und anschließendem Abkühlen wird die Reaktionsmischung mit Diethylether verdünnt, mit 5%iger Natriumcarbonat- sowie mit gesättigter NaCl-Lösung gewaschen. Trocknen über Natriumsulfat, Filtrieren und Einengen am Rotationsverdampfer hinterlassen als Rohprodukt einen rötlichen Feststoff, der säulenchromatographisch an Kieselgel gereinigt (Fließmittel: Petrolether/Ethylacetat 95:5), das Produkt in Form eines weißen Feststoffes liefert.
Ausbeute: 0.566 g (3.38 mmol); 75%
Schmp.: 87-89 °C
C₉H₁₃NO₂ (167.2)
¹H-NMR (CDCl₃): δ (ppm) = 1.57 (s, 9H), 6.23 (m, 1 H), 6.84 (m, 1 H), 6.91 (m, 1 H), 9.23 (s, breit, 1 H)

### D. tert-Butyl-1-[2-acetoxy-3-(4-octylphenoxy)propyl]pyrrol-2-carboxylat

0.145 g (0.867 mmol) *tert-*Butylpyrrol-2-carboxylat werden in 10 ml absol. DMSO gelöst, mit 0.107 g (0.952 mmol) Kalium-*tert-*butylat versetzt und 15 min bei 110°C gerührt. Eine Lösung von 0.333 g (0.864 mmol) [1-Brom-3-(4-octylphenoxy)propan-2-yl]acetat- in 10 ml absol. DMSO wird hinzugetropft. Nach dreißigminütigem Erhitzen bei 110°C und anschließendem Abkühlen erfolgt Hydrolyse in gesättigter - NaCl-Lösung Viermalige Extraktion mit Diethylether, Vereinigung der organischen Phasen, Einengen am Rotationsverdampfer auf die Hälfte des Volumens, dreimaliges Waschen mit gesättigter NaCl-Lösung, Trocknen über Natriumsulfat, Filtrieren und erneutes Einengen am Rotationsverdampfer hinterlassen als Rohprodukt ein gelbes Öl, das, flashsäulenchromatographisch an Kieselgel gereinigt (Fließmittel: Petrolether/Ethylacetat 80:20), das Produkt in Form eines farblosen Öls liefert.
Ausbeute: 0.169 g (0.358 mmol); 41 %
C₂₈H₄₁NO₅ (471.6)
¹H-NMR (CDCl₃): ö (ppm) = 0.88 (t, J = 7 Hz, 3H), 1.28 (m, 10H), 1.55 (s, 9H), 1.60 (m, 2H), 2.01 (s, 3H), 2.54 (t, J = 8 Hz, 2H), 4.01 (dd, J = 11 Hz und J = 4 Hz, 1H), 4.13 (dd, J = 11 Hz und J = 4 Hz, 1 H), 4.49 (dd, J = 14 Hz und J = 8 Hz, 1H), 4.86 (dd, J = 14 Hz und J = 4 Hz, 1H), 5.43-5.48 (m, 1H), 6.07 (dd, J = 4 Hz und J = 2 Hz, 1H), 6.79-6.83 (m, 3H), 6.88 (dd, J = 4 Hz und J = 2 Hz, 1H), 7.08 (d, J = 9 Hz, 2H)

### E. tert-Butyl-1-[2-hydroxy-3-(4-octylphenoxy)propyl]pyrrol-2-carboxylat

0.150 g (0.318 mmol) *tert*-Butyl-1-[2-acetoxy-3-(4-octylphenoxy)propyl]pyrrol-2-carboxylat werden in 10 ml absol. Methanol gelöst, mit 1.26 ml (0.630 mmol) einer 0.5M Natriummethanolat-Lösung versetzt und 15 min bei Raumtemperatur gerührt. Nach Einengen am Rotationsverdampfer auf die Hälfte des Volumens wird der Ansatz mit Diethylether verdünnt. Waschen der organischen Phase mit halbgesättigter sowie mit gesättigter NaCl-Lösung, Trocknen über Natriumsulfat, Filtrieren und erneutes Einengen am Rotationsverdampfer liefern das Produkt in Form eines gelblichen Öls.
Ausbeute: 0.133 g (0.310 mmol); 98%
C₂₆H₃₉NO₄ (429.6)
¹H-NMR (CDCl₃): δ (ppm) = 0.89 (t, J = 7 Hz, 3H), 1.28 (m, 10H), 1.56 (s, 9H), 1.60 (m, 2H), 2.55 (t, J = 8 Hz, 2H), 3.57 (d, J = 4 Hz, 1 H), 3.93 (d, J = 6 Hz, 2H), 4.30 (m, 1 H), 4.46 (dd, J = 14 Hz und J = 6 Hz, 1H), 4.66 (dd, J = 14 Hz und J = 4 Hz, 1H), 6.11 (dd, J = 4 Hz und J = 2 Hz, 1H), 6.84 (m, 3H), 6.90 (dd, J = 4 Hz und J = 2 Hz, 1 H), 7.08 (d, J = 9 Hz, 2H)

### F. tert-Butyl-1-[3-(4-octylphenoxy)-2-oxopropyl]pyrrol-2-carboxylat

1.14 g (11.2 mmol) Essigsäureanhydrid werden mit 10 ml absol. DMSO versetzt, 10 min bei Raumtemperatur gerührt und zu einer Lösung von 0.121 g (0.282 mmol) *tert-*Butyl-1-[2-hydroxy-3-(4-octylphenoxy)propyl]pyrrol-2-carboxylat in 10 ml absol. DMSO getropft. Nach sechzehnstündigem Rühren wird in eine Mischung aus 5%iger Natriumhydrogencarbonat- und gesättigter NaCl-Lösung (1:1, v/v) gegossen und 10 min lang hydrolysiert. Viermalige Extraktion mit Diethylether, Vereinigung der organischen Phasen, Einengen am Rotationsverdampfer auf die Hälfte des Volumens, dreimaliges Waschen mit gesättigter NaCl-Lösung, Trocknen über Natriumsulfat, Filtrieren und erneutes Einengen am Rotationsverdampfer hinterlassen als Rohprodukt ein gelbes Öl, das, säulenchromatographisch an Kieselgel gereinigt (Fließmittel: Petrolether/Ethylacetat 98:2), das Produkt in Form eines gelblichen Öls liefert.
Ausbeute: 0.096 g (0.225 mmol); 80%
C₂₆H₃₇NO₄ (427.6)
¹H-NMR (CDCl₃): δ (ppm) = 0.89 (t, J = 7 Hz, 3H), 1.29 (m, 10H), 1.52 (s, 9H), 1.60 (m, 2H), 2.55 (t, J = 8 Hz, 2H), 4.73 (s, 2H), 5.29 (s, 2H), 6.18 (m, 1H), 6.72 (m, 1H), 6.86 (d, J = 8 Hz, 2H), 6.93 (m, 1H), 7.11 (d, J = 8 Hz, 2H)

### G. 1-[3-(4-Octylphenoxy)-2-oxopropyl]pyrrol-2-carbonsäure

0.045 g (0.105 mmol) *tert*-Butyl-1-[3-(4-octylphenoxy)-2-oxopropyl]pyrrol-2-carboxylat werden in 10 ml absol. Dichlormethan gelöst und mit 0.900 g (7.89 mmol) Trifluoressigsäure versetzt. Nach einstündigem Rühren bei Raumtemperatur wird am Rotationsverdampfer bis zur Trockne eingeengt. Zweimaliges Lösen in Toluol sowie jeweiliges Einengen am Rotationsverdampfer hinterlassen als Rohprodukt einen bräunlichen Feststoff, der, umkristallisiert aus Methanol und wenigen Tropfen Wasser, das Produkt in Form eines beigen Feststoffes liefert.
Ausbeute: 0.016 g (0.043 mmol); 41%
Schmp.: 157 °C (Zersetzung)
C₂₂H₂₉NO₄ (371.5)
¹H-NMR (DMSO-*d*₆): δ (ppm) = 0.84 (t, J = 7 Hz, 3H), 1.24 (m, 10H), 1.50 (s, 2H), 2.49 (t, J = 8 Hz, 2H), 4.86 (s, 2H), 5.27 (s, 2H), 6.11 (m, 1 H), 6.81 (m, 1 H), 6.83 (d, J = 8 Hz, 2H), 7.03 (m, 1 H), 7.06 (d, J = 8 Hz, 2H)

### Beispiel 6

### 1-[3-(4-Octylphenoxy)-2-oxopropyl]pyrrol-3-carbonsäure

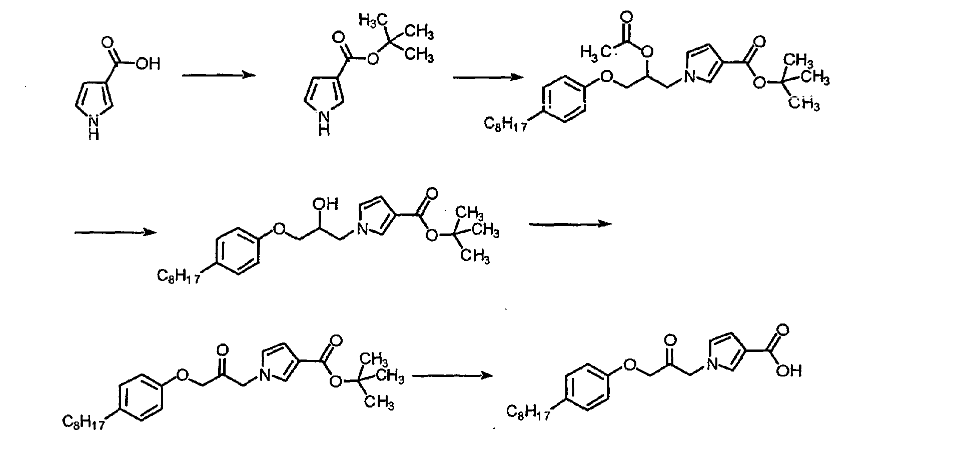

### A. tert-Butylpyrrol-3-carboxylat

0.50 g (4.50 mmol) Pyrrol-3-carbonsäure werden in 15 ml absol. Benzol suspendiert und unter Rückfluss erhitzt. Eine Lösung 4.07 g (18.0 mmol) 90%igem *N,N*-Dimethylformamiddi-*tert-*butylacetal in 15 ml absol. Benzol wird innerhalb von 30 min hinzugetropft. Nach erneutem dreißigminütigem Erhitzen unter Rückfluss und anschließendem Abkühlen wird die Reaktionsmischung mit Diethylether verdünnt, mit 5%iger Natriumcarbonat- sowie mit gesättigter NaCl-Lösung gewaschen. Trocknen über Natriumsulfat, Filtrieren und Einengen am Rotationsverdampfer hinterlassen als Rohprodukt einen gelben Feststoff, der, säulenchromatographisch an Kieselgel gereinigt (Fließmittel: Petrolether/Ethylacetat 90:10), das Produkt in Form eines weißen Feststoffes liefert.
Ausbeute: 0.433 g (2.59 mmol); 58%
Schmp.: 82-84 °C
C₉H₁₃NO₂ (167.2)
¹H-NMR (CDCl₃): δ (ppm) = 1.55 (s, 9H), 6.59 (m, 1 H), 6.72 (m, 1H), 7.34 (m, 1H), 8.75 (s, breit, 1H)

### B. tert-Butyl-1-[2-acetoxy-3-(4-octylphenoxy)propyl]pyrrol-3-carboxylat

0.145 g (0.867 mmol) *tert*-Butylpyrrol-3-carboxylat werden in 10 ml absol. DMSO gelöst, mit 0.107 g (0.952 mmol) Kalium-*tert-*butylat versetzt und 15 min bei 110°C gerührt. Eine Lösung von 0.333 g (0.864 mmol) [1-Brom-3-(4-octylphenoxy)propan-2-yl]acetat in 10 ml absol. DMSO wird hinzugetropft. Nach dreißigminütigem Erhitzen bei 110°C und anschließendem Abkühlen erfolgt Hydrolyse in gesättigter NaCl-Lösung. Viermalige Extraktion mit Diethylether, Vereinigung der organischen Phasen, Einengen am Rotationsverdampfer auf die Hälfte des Volumens, dreimaliges Waschen mit gesättigter NaCl-Lösung, Trocknen über Natriumsulfat, Filtrieren und erneutes Einengen am Rotationsverdampfer hinterlassen als Rohprodukt ein gelbes Öl, das, flashsäulenchromatographisch an Kieselgel gereinigt (Fließmittel: Petrolether/Ethylacetat 93:7), das Produkt in Form eines farblosen Öls liefert.
Ausbeute: 0.104 g (0.221 mmol); 26%
C₂₈H₄₁NO₅ (471.6)
¹H-NMR (CDCl₃): δ (ppm) = 0.88 (t, J = 7 Hz, 3H), 1.26 (m, 10H), 1.52 (s, 9H), 1.59 (m, 2H), 2.09 (s, 3H), 2.54 (t, J = 8 Hz, 2H), 3.89 (dd, J = 10 Hz und J = 6 Hz, 1H), 3.95 (dd, J = 10 Hz und J = 4 Hz, 1 H), 3.89-4.25 (m, 2H), 5.30 (m, 1 H), 6.50 (dd, J = 3 Hz und J = 2 Hz, 1 H), 6.57 (m, 1 H), 6.81 (d, J = 9 Hz, 2H), 7.09 (d, J = 9 Hz, 2H), 7.20 (m, 1 H)

### C. tert-Butyl-1-[2-hydroxy-3-(4-octylphenoxy)propyl]pyrrol-3-carboxylat

0.104 g (0.221 mmol) *tert*-Butyl-1-[2-acetoxy-3-(4-octylphenoxy)propyl]pyrrol-3-carboxylat werden in 10 ml absol. Methanol gelöst, mit 0.88 ml (0.440 mmol) einer 0.5M Natriummethanolat-Lösung versetzt und 15 min bei Ramtemperatur gerührt. Nach Einengen am Rotationsverdampfer auf die Hälfte des Volumens wird der Ansatz mit Diethylether verdünnt. Waschen der organischen Phase mit halbgesättigter sowie mit gesättigter NaCl-Lösung, Trocknen über Natriumsulfat, Filtrieren und erneutes Einengen am Rotationsverdampfer liefern das Produkt in Form eines gelblichen Öls.
Ausbeute: 0.091 g (0.212 mmol); 96%
C₂₆H₃₉NO₄ (429.6)
¹H-NMR (CDCl₃): δ (ppm) = 0.89 (t, J = 7 Hz, 3H), 1.30 (m, 10H), 1.55 (s, 9H), 1.62 (m, 2H), 2.46 (s breit, 1H), 2.54 (t, J = 8 Hz, 2H), 3.86 (dd, J = 10 Hz und J = 6 Hz), 3.92 (dd, J = 9 Hz und J = 5 Hz), 4.07 (dd, J = 14 Hz und J = 7 Hz), 4.16 (dd, J = 14 Hz und J = 5 Hz), 4.27 (m, 1H), 6.55 (dd, J = 3 Hz und J = 2 Hz, 1H), 6.63 (m, 1H), 6.82 (d, J = 9 Hz, 2H), 7.11 (d, J = 9 Hz, 2H), 7.27 (m, 1H)

### D. tert-Butyl-1-[3-(4-octylphenoxy)-2-oxopropyl]pyrrol-3-carboxylat

0.817 g (8.00 mmmol) Essigsäureanhydrid werden mit 10 ml absol. DMSO versetzt, 10 min bei Raumtemperatur gerührt und zu einer Lösung von 0.086 g (0.200 mmol) *tert*-Butyl-1-[2-hydroxy-3-(4-octylphenoxy)propyl]pyrrol-3-carboxylat in 10 ml absol. DMSO getropft. Nach neunzehnstündigem Rühren wird in eine Mischung aus 5%iger Natriumhydrogencarbonat- und gesättigter NaCl-Lösung (1:1. v/v) gegossen und 10 min lang hydrolysiert. Viermalige Extraktion mit Diethylether, Vereinigung der organischen Phasen, Einengen am Rotations-verdampfer auf die Hälfte des Volumens, dreimaliges Waschen mit gesättigter NaCl-Lösung, Trocknen über Natriumsulfat, Filtrieren und erneutes Einengen am Rotationsverdampfer hinterlassen als Rohprodukt ein braunes Öl, das, flashsäulenchromatographisch an Kieselgel gereinigt (Fließmittel: Petrolether/Ethylacetat 90:10), das Produkt in Form eines gelblichen Öls liefert.
Ausbeute: 0.065 g (0.152 mmol); 76%
C₂₆H₃₇NO₄ (427.6)
¹H-NMR (CDCl₃): δ (ppm) = 0.88 (t, J = 7 Hz, 3H), 1.26 (m, 10H), 1.53 (s, 9H), 1.59 (m, 2H), 2.56 (t, J = 8 Hz, 2H), 4.63 (s, 2H), 4.99 (s, 2H), 6.50 (m, 1H), 6.59 (dd, J = 3 Hz und J = 2 Hz, 1H), 6.82 (d, J = 9 Hz, 2H), 7.13 (m, 3H)

### E. 1-[3-(4-Octylphenoxy)-2-oxopropyl]pyrrol-3-carbonsäure

0.049 g (0.115 mmol) *tert*-Butyl-1-[3-(4-octylphenoxy)-2-oxopropyl]pyrrol-3-carboxylat werden in 10 ml absol. Dichlormethan gelöst und mit 0.988 g (8.67 mmol) Trifluoressigsäure versetzt. Nach eineinhalbstündigem Rühren bei Raumtemperatur wird am Rotationsverdampfer bis zur Trockene eingeengt. Zweimaliges Lösen in Toluol sowie jeweiliges Einengen am Rotationsverdampfer hinterlassen als Rohprodukt einen grünlichen Feststoff, der in Diethylether gelöst und mit Petrolether gefällt wird. Zwanzigminütiges Zentrifugieren bei 3000 U/min, Abdekantieren des Überstandes, Verdrängen der Lösungsmittelreste im Stickstoffstrom und Trocknen im Vakuumexsiccator liefern das Produkt in Form eines beigen Feststoffes.
Ausbeute: 0.018 g (0.048 mmol); 42%
Schmp.: 125 °C (Zersetzung)
C₂₂H₂₉NO₄ (371.5)
¹H-NMR (CDCl₃): δ (ppm) = 0.88 (t, J = 7 Hz, 3H), 1.28 (m, 10H), 1.57 (m, 2H), 2.56 (t, J = 8 Hz, 2H), 4.65 (s, 2H), 5.04 (s, 2H), 6.55 (m, 1 H), 6.68 (m, 1 H), 6.82 (d, J = 8 Hz, 2H), 7.14 (d, J = 8 Hz, 2H), 7.30 (m, 1H)

### Beispiel 7

### 1-[3-(4-Octylphenoxy)-2-oxopropyl]indol-2-carbonsäure

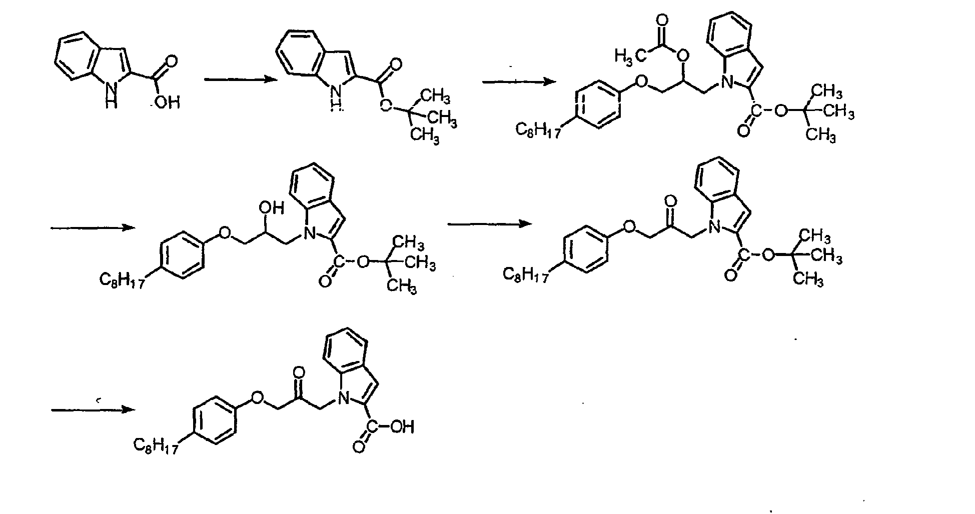

### A. tert-Butylindol-2-carboxylat

1.00 g (6.21 mmol) Indol-2-carbonsäure werden in 15 ml absol. THF gelöst, mit 1.01 g (6.23 mmol) *N*,*N*'-Carbonyldiimidazol versetzt und eine Stunde bei Raumtemperatur gerührt. Anschließend werden 0.77 g (6.86 mmol) Kalium-*tert-*butylat sowie 9.12 g (123 mmol) *tert-*Butanol hinzugefügt. Nach sechsstündigem Erhitzen unter Rückfluss, Quenchen mit 5 ml Wasser, Abfiltrieren, Spülen mit THF, Trocknen über Natriumsulfat und Einengen am Rotationsverdampfer wird als Rohprodukt ein bräunlicher Feststoff erhalten. Säulenchromatographische Vorreinigung an Kieselgel über eine kurze Säule mit Petrolether/Ethylacetat 95:5, hinterlässt einen weißen Feststoff, der, säulenchromatographisch an Kieselgel gereinigt (Fließmittel: Petrolether/Ethylacetat 98:2), das Produkt in Form eines weißen Feststoffes liefert.
Ausbeute: 0.325 g (1.496 mmol); 24%
Schmp.: 104-105 °C
C₁₃H₁₅NO₂ (217.3)
¹H-NMR (CDCl₃): δ (ppm) = 1.65 (s, 9H), 7.14 (m, 2H), 7.31 (m, 1 H), 7.43 (dd, J = 8 Hz und J = 1 Hz, 1 H), 7.68 (dd, J = 8 Hz und J = 1 Hz, 1H), 9.12 (s, breit, 1 H)

### B. tert-Butyl-1-[2-acetoxy-3-(4-octylphenoxy)propyl]indol-2-carboxylat

0.325 g (1.50 mmol) *tert-*Butylindol-2-carboxylat werden in 15 ml absol. DMSO gelöst, mit 0.184 g (1.64 mmol) Kalium-*tert-*butylat versetzt und 15 min bei 110°C gerührt. Eine Lösung von 0.576 g (1.50 mmol) [1-Brom-3-(4-Octylphenoxy)propan-2-yl]acetat in 15 ml absol. DMSO wird hinzugetropft. Nach dreißigminütigem Erhitzen bei 110°C und anschließendem Abkühlen erfolgt Hydrolyse in gesättigter NaCl-Lösung. Viermalige Extraktion mit Diethylether, Vereinigung der organischen Phasen, Einengen am Rotationsverdampfer auf die Hälfte des Volumens, dreimaliges Waschen mit gesättigter NaCl-Lösung, Trocknen über Natriumsulfat, Filtrieren und erneutes Einengen am Rotationsverdampfer hinterlassen als Rohprodukt ein gelbes Öl, das, flashsäulenchromatographisch an Kieselgel gereinigt (Fließmittel: Petrolether/Ethylacetat 99:1), das Produkt in Form eines farblosen Öls liefert. Ausbeute: 0.246 g (0.472 mmol); 32%
C₃₂H₄₃NO₅ (521.7)
¹H-NMR (CDCl₃): δ (ppm) = 0.89 (t, J = 7 Hz, 3H), 1.30 (m, 10H), 1.59 (m, 2H), 1.62 (s, 9H), 1.81 (s, 3H), 2.56 (t, J = 8 Hz, 2H), 4.17 (d, J = 4 Hz, 2H), 4.83 (dd, J = 15 Hz und J = 8 Hz, 1 H), 5.07 (dd, J = 15 Hz und J = 5 Hz, 1H), 5.57 (m, 1H), 6.84 (d, J = 9 Hz, 2H), 7.09 (d, J = 9 Hz, 2H), 7.13 (m, 1 H), 7.26 (s, 1 H), 7.32 (m, 1 H), 7.57 (d, J = 8 Hz, 1 H), 7.64 (d, J = 8 Hz, 1 H)

### C. tert-Butyl-1-[2-hydroxy-3-(4-octylphenoxy)propyl]indol-2-carboxylat

0.213 g (0.408 mmol) *tert*-Butyl-1-[2-acetoxy-3-(4-octylphenoxy)propyl]indol-2-carboxylat werden in 10 ml absol. Methanol gelöst, mit 1.64 ml (0.82 mmol) einer 0.5M Natriummethanolat-Lösung versetzt und 15 min bei Raumtemperatur gerührt. Nach Einengen am Rotationsverdampfer auf die Hälfte des Volumens wird der Ansatz mit Diethylether verdünnt. Waschen der organischen Phase mit halbgesättigter sowie mit gesättigter NaCl-Lösung, Trocknen über Natriumsulfat, Filtrieren und erneutes Einengen am Rotationsverdampfer hinterlassen als Rohprodukt ein gelbliches Öl, das säulenchromatographisch an Kieselgel gereinigt (Fließmittel: Petrolether/Ethylacetat 95:5), das Produkt in Form eines gelblichen Öls liefert.
Ausbeute: 0.152 g (0.317 mmol); 78%
C₃₀H₄₁NO₄ (479.7)
¹H-NMR (CDCl₃): δ (ppm) = 0.89 (t, J = 7 Hz, 3H), 1.29 (m, 10H), 1.59 (m, 2H), 1.63 (s, 9H), 2.55 (t, J = 8 Hz, 2H), 3.62 (s breit, 1H), 3.99 (dd, J = 10 Hz und J = 6 Hz, 1 H), 4.04 (dd, J = 10 Hz und J = 5 Hz, 1H), 4.42 (m, 1H), 4.76 (dd, J = 15 Hz und J = 7 Hz, 1 H), 4.81 (dd, J = 15 Hz und J = 5 Hz, 1H), 6.86 (d, J = 9 Hz, 2H), 7.10 (d, J = 9 Hz, 2H), 7.14 (m, 1H), 7.29 (m, 2H), 7.48 (d, J = 8 Hz, 1H), 7.69 (d, J = 8 Hz, 1H)

### D. tert-Butyl-1-[3-(4-octylphenoxy)-2-oxopropyl)indol-2-carboxylat

1.02 g (10.0 mmol) Essigsäureanhydrid werden mit 10 ml absol. DMSO versetzt, 10 min bei Raumtemperatur gerührt und zu einer Lösung von 0.120 g (0.250 mmol) *tert-*Butyl-1-[2-hydroxy-3-(4-octylphenoxy)propyl]indol-2-carboxylat in 10 ml absol. DMSO getropft. Nach achtstündigem Rühren wird in eine Mischung aus 5%iger Natriumhydrogencarbonat- und gesättigter NaCl-Lösung (1:1, v/v) gegossen und 10 min lang hydrolysiert. Viermalige Extraktion mit Diethylether, Vereinigung der organischen Phasen, Einengen am Rotationsverdampfer auf die Hälfte des Volumens, dreimaliges Waschen mit gesättigter NaCl-Lösung, Trocknen über Natriumsulfat, Filtrieren und erneutes Einengen am Rotationsverdampfer hinterlassen als Rohprodukt ein gelbes Öl, das, säulenchromatographisch an Kieselgel gereinigt (Fließmittel: Petrolether/Ethylacetat 98:2), das Produkt in Form eines gelblichen Öls liefert.
Ausbeute: 0.113 g (0.237 mmol); 95%
C₃₀H₃₉NO₄ (477.6)
¹H-NMR (CDCl₃): δ (ppm) = 0.89 (t, J = 7 Hz, 3H), 1.29 (m, 10H), 1.58 (s, 9H), 1.61 (m, 2H), 2.57 (t, J = 8 Hz, 2H), 4.73 (s, 2H), 5.57 (s, 2H), 6.87 (d, J = 9 Hz, 2H), 7.11-7.14 (m, 4H), 7.30 (m, 2H), 7.68 (d, J = 8 Hz, 1 H)

### E. 1-[3-(4-Octylphenoxy)-2-oxopropyl]indol-2-carbonsäure

0.046 g (0.096 mmol) *tert*-Butyl-1-[3-(4-octylphenoxy)-2-oxopropyl]indol-2-carboxylat werden in 10 ml absol. Dichlormethan gelöst und mit 1.49 g (13.6 mmol) Trifluoressigsäure versetzt. Nach eineinhalbstündigem Rühren bei Raumtemperatur wird am Rotationsverdampfer bis zur Trockne eingeengt. Zweimaliges Lösen in Toluol sowie jeweiliges Einengen am Rotationsverdampfer hinterlassen als Rohprodukt einen gelblichen Feststoff, der, umkristallisiert aus Petrolether/Ethylacetat (67:33), das Produkt in Form eines weißen Feststoffes liefert.
Ausbeute: 0.031 g (0.074 mmol); 77%
Schmp.: 173 °C
C₂₆H₃₁NO₄ (421.5)
¹H-NMR (DMSO-*d*₆): δ (ppm) = 0.83 (t, J = 7 Hz, 3H), 1.23 (m, 10H), 1.50 (m, 2H), 2.48 (t, J = 8 Hz, 2H), 4.99 (s, 2H), 5.60 (s, 2H), 6.86 (d, J = 9 Hz, 2H), 7.07 (d, J = 9 Hz, 2H), 7.12 (m, 1 H), 7.26 (s, 1 H), 7.29 (m, 1H), 7.54 (d, J = 8 Hz, 1 H), 7.68 (d, J = 8 Hz, 1 H)

### Beispiel 8

### 1-[3-(4-Octylphenoxy)-2-oxopropyl]indol-3-cärbonsäure

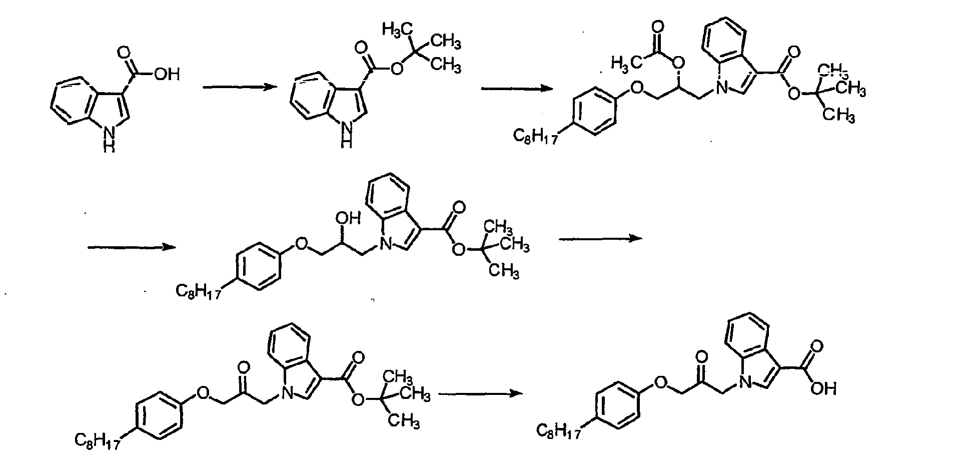

### A. tert-Butylindol-3-carboxylat

1.00 g (6.21 mmol) Indol-3-carbonsäure werden in 150 ml absol. THF gelöst, mit 0.05 g (0.68 mmol) absol. DMF sowie mit 1.48 g (12.5 mmol) Thionylchlorid versetzt und eine Stunde bei Raumtemperatur gerührt. Nach Zutropfen von 2.51 g (24.8 mmol) Triethylamin sowie von 9.20 g (124 mmol) *tert-*Butanol und zweistündigem Erhitzen unter Rückfluss erfolgt Hydrolyse der abkühlten Reaktionsmischung in Wasser. Dreimalige Extraktion mit Diethylether, Einengen am Rotationsverdampfer auf die Hälfte des Volumens, Waschen mit 5%iger Natriumcarbonat-Lösung sowie mit gesättigter NaCl-Lösung, Trocknen über Natriumsulfat, Filtrieren und erneutes Einengen am Rotationsverdampfer hinterlassen als Rohprodukt ein braunes Öl. das, säulenchromatographisch an Kieselgel gereinigt (Fließmittel: Petrolether/Ethylacetat 90:10), das Produkt in Form eines gelben Öls liefert.
Ausbeute: 1.05 g (4.83 mmol); 78%
C₁₃H₁₅NO₂ (217.3)
¹H-NMR (CDCl₃): δ (ppm) = 1.65 (s, 9H), 7.25 (m, 2H), 7.39 (m, 1H), 7.85 (d, J = 3 Hz, 1H), 8.15 (m, 1 H), 8.84 (s, breit, 1 H)

### B. tert-Butyl-1-[2-acetoxy-3-(4-octylphenoxy)propyl]indol-3-carboxylat

0.50 g (2.30 mmol) *tert-*Butylindol-3-carboxylat werden in 15 ml absol. DMSO gelöst, mit 0.28 g (2.50 mmol) Kalium-*tert-*butylat versetzt und 15 min bei 110°C gerührt. Eine Lösung von 0.89 g (2.31 mmol) [1-Brom-3-(4-octylphenoxy)propan-2-yl]acetat in 15 ml absol. DMSO wird hinzugetropft. Nach dreißigminütigem Erhitzen bei 110°C und anschließendem Abkühlen erfolgt Hydrolyse in gesättigter NaCl-Lösung. Viermalige Extraktion mit Diethylether, Vereinigung der organischen Phasen, Einengen am Rotationsverdampfer auf die Hälfte des Volumens, dreimaliges Waschen mit gesättigter NaCl-Lösung, Trocknen über Natriumsulfat, Filtrieren und erneutes Einengen am Rotationsverdampfer hinterlassen als Rohprodukt ein gelbes Öl, das, flashsäulenchromatographisch an Kieselgel gereinigt (Fließmittel: Petrolether/Ethylacetat 95:5), das Produkt in Form eines gelblichen Öls liefert.
Ausbeute: 0.56 g (1.07 mmol); 47%
C₃₂H₄₃NO₅ (521.7)
¹H-NMR (CDCl₃): δ (ppm) = 0.89 (t, J = 7 Hz, 3H), 1.29 (m, 10H), 1.57 (m, 2H), 1.61 (s, 9H), 2.05 (s, 3H), 2.55 (t, J = 8 Hz, 2H), 3.97 (d, J = 5 Hz, 2H), 4.51 (dd, J = 9 Hz und J = 6 Hz, 1H), 4.56 (dd, J = 9 Hz und J = 6 Hz, 1 H), 5.43 (m, 1 H), 6.83 (d, J = 9 Hz, 2H), 7.10 (d, J = 9 Hz, 2H), 7.24 (m, 2H), 7.45 (m, 1 H), 7.75 (s, 1H), 8.14 (m, 1 H)

### C. tert-Butyl-1-[2-hydroxy-3-(4-octylphenoxy)propyl]indol-3-carboxylat

0.293 g (0.562 mmol) *tert*-Butyl-1-[2-acetoxy-3-(4-octylphenoxy)propyl]indol-3-carboxylat in 20 ml absol. Methanol gelöst, mit 2.24 ml (1.12 mmol) einer 0.5M Natriummethanolat-Lösung versetzt und 15 min bei Raumtemperatur gerührt. Nach Einengen am Rotationsverdampfer auf die Hälfte des Volumens wird der Ansatz mit Diethylether verdünnt. Waschen der organischen Phase mit halbgesättigter sowie mit gesättigter NaCl-Lösung, Trocknen über Natriumsulfat, Filtrieren und erneutes Einengen am Rotationsverdampfer liefern das Produkt in Form eines gelblichen Öls.
Ausbeute: 0.269 g (0.561 mmol); 100%
C₃₀H₄₁NO₄ (479.7)
¹H-NMR (CDCl₃): δ (ppm) = 0.90 (t, J = 7 Hz, 3H), 1.30 (m, 10H), 1.59 (m, 2H), 1.63 (s, 9H), 2.56 (t, J = 8 Hz, 2H), 2.66 (s, breit, 1H), 3.89 (dd, J = 10 Hz und J = 5 Hz, 1H), 3.95 (dd, J = 10 Hz und J = 5 Hz, 1H), 4.30 (dd, J = 14 Hz und J = 6 Hz, 1 H), 4.37 (m, 1H), 4.45 (dd, J = 14 Hz und J = 4 Hz, 1 H), 6.81 (d, J = 8 Hz, 2H), 7.09 (d, J = 8 Hz, 2H), 7.25 (m, 2H), 7.40 (m, 1 H), 7.98 (s, 1H), 8.16 (m, 1H)

### D. tert-Butyl-1-[3-(4-octylphenoxy)-2-oxopropyl]indol-3-carboxylat

2.02 g (19.8 mmol) Essigsäureanhydrid werden mit 10 ml absol. DMSO versetzt, 10 min bei Raumtemperatur gerührt und zu einer Lösung von 0.237 g (0.494 mmol) *tert-*Butyl-1-[2-hydroxy-3-(4-octylphenoxy)propyl]indol-3-carboxylat in 10 ml absol. DMSO getropft. Nach zweistündigem Rühren wird in eine Mischung aus 5%iger Natriumhydrogencarbonat- und gesättigter NaCl-Lösung (1:1, v/v) gegossen und 10 min lang hydrolysiert. Viermalige Extraktion mit Diethylether, Vereinigung der organischen Phasen, Einengen am Rotationsverdampfer auf die Hälfte des Volumens, dreimaliges Waschen mit gesättigter NaCl-Lösung, Trocknen über Natriumsulfat, Filtrieren und erneutes Einengen am Rotationsverdampfer hinterlassen als Rohprodukt ein gelbes Öl, das, flashsäulenchromatographisch gereinigt an Kieselgel (Fließmittel: Petrolether/Ethylacetat 95:5), das Produkt in Form eines gelblichen Öls liefert.
Ausbeute: 0.160 g (0.335 mmol); 68%
C₃₀H₃₉NO₄ (477.6)
¹H-NMR (CDCl₃): δ (ppm) = 0.89 (t, J = 7 Hz, 3H), 1.30 (m, 10H), 1.59 (m, 2H), 1.62 (s, 9H), 2.58 (t, J = 8 Hz, 2H), 4.66 (s, 2H), 5.22 (s, 2H), 6.85 (d, J = 9 Hz, 2H), 7.08 (dd, J = 7 Hz und J = 2 Hz, 1 H), 7.15 (d, J = 9 Hz, 2H), 7.24 (m, 2H), 7.71 (s, 1H), 8.17 (dd, J = 6 Hz und J = 2 Hz, 1H)

### E. 1-[3-(4-Octylphenoxy)-2-oxopropyl]indol-3-carbonsäure

0.072 g (0.151 mmol) *tert-*Butyl-1-[3-(4-octylphenoxy)-2-oxopropyl]indol-3-carboxylat werden in 10 ml absol. Dichlormethan gelöst und mit 1.56 g (13.7 mmol) Trifluoressigsäure versetzt. Nach einstündigem Rühren bei Raumtemperatur wird am Rotationsverdampfer bis zur Trockene eingeengt. Zweimaliges Lösen in Toluol sowie jeweiliges Einengen am Rotationsverdampfer hinterlassen als Rohprodukt einen rötlichen Feststoff, der, umkristallisiert aus Petrolether/Ethylacetat (67:33), das Produkt in Form eines weißen Feststoffes liefert.
Ausbeute: 0.055 g (0.130 mmol); 86%
Schmp.: 182-183 °C
C₂₆H₃₁NO₄ (421.5)
¹H-NMR (DMSO-*d*₆): δ (ppm) = 0.84 (t, J = 7 Hz, 3H), 1.24 (m, 10H), 1.51 (m, 2H), 2.49 (t, J = 8 Hz, 2H), 5.00 (s, 2H), 5.46 (s, 2H), 6.88 (d, J = 9 Hz, 2H), 7.10 (d, J = 9 Hz, 2H), 7.18 (m, 2H), 7.44 (m, 1H), 7.97 (s, 1H), 8.00 (m, 1H)

### Beispiel 9

### 1-[3-(4-Octylphenoxy)-2-oxopropyl]indol-5-carbonsäure

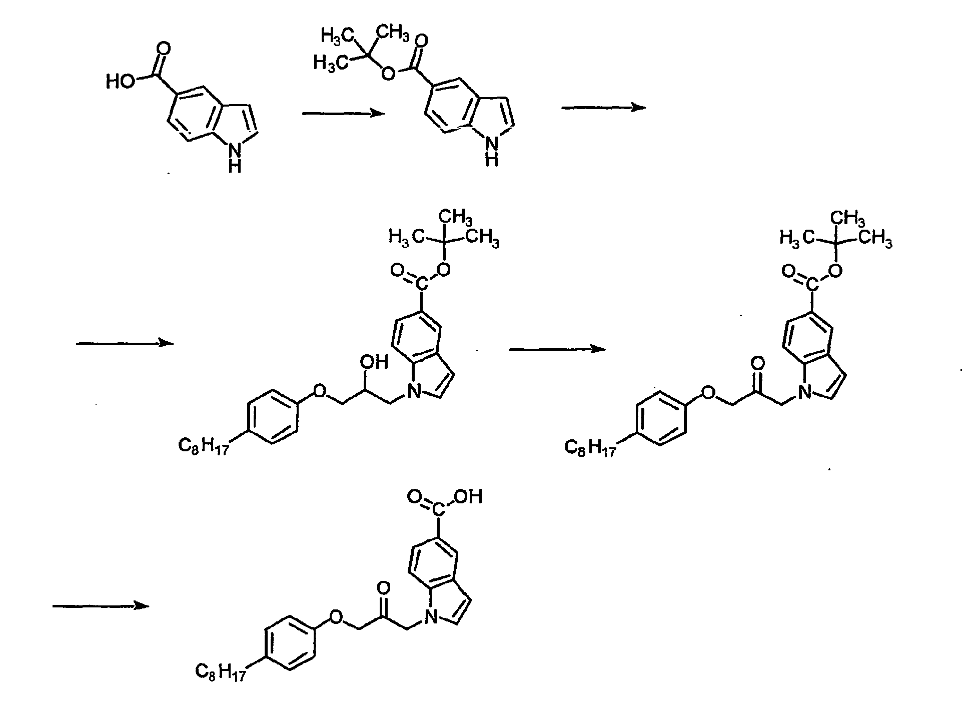

### A. tert-Butylindol-5-carboxylat

0.50 g (3.10 mmol) Indol-5-carbonsäure in 20 ml absol. Benzol suspendiert und unter Rückfluss erhitzt. Eine Lösung von 2.80 g (12.39 mmol) 90%igem *N*,*N-*Dimethylformamiddi-*tert*-butylacetal in 20 ml absol. Benzol wird innerhalb von 30 min hinzugetropft. Nach erneutem dreißigminütigem Erhitzen unter Rückfluss und anschließendem Abkühlen wird die Reaktionsmischung mit Diethylether verdünnt, mit 5%iger Natriumcarbonat- sowie mit gesättigter NaCl-Lösung gewaschen. Trocknen über Natriumsulfat, Filtrieren und Einengen am Rotationsverdampfer hinterlassen als Rohprodukt ein gelbes Öl, das säulenchromatographisch an Kieselgel gereinigt (Fließmittel: Petrolether/Ethylacetat 95:5), das Produkt in Form eines weißen Feststoffes liefert.
Ausbeute: 0.214 g (0.98 mmol); 32%
Schmp.: 91-93 °C
C₁₃H₁₅NO₂ (217.3)
¹H-NMR (CDCl₃): δ (ppm) = 1.63 (s, 9H), 6.64 (m, 1H), 7.26 (m, 1H), 7.38 (d, J = 9 Hz, 1H), 7.87 (dd, 1 H, J = 9 Hz und J = 2 Hz), 8.36 (m, 1H), 8.40 (s, breit, 1 H)

### B. tert-Butyl-1-[2-hydroxy-3-(4-octylphenoxy)propyl]indol-5-carboxylat

0.077 g (1.93 mmol) Natriumhydrid als 60%ige Dispersion in Mineralöl werden in 15 ml absol. DMF suspendiert, 10 min bei Raumtemperatur gerührt. mit einer Lösung von 0.400 g (1.84 mmol) *tert*-Butylindol-5-carboxylat in 15 ml absol. DMF versetzt und eine Stunde bei Raumtemperatur gerührt. Eine Lösung von 0.483 g (1.84 mmol) 2-(4-Octylphenoxymethyl)oxiran in 15 ml absol. DMF wird hinzugetropft. Nach vierstündigem Erhitzen bei 60°C und anschließendem Abkühlen erfolgt Hydrolyse in halbgesättigter NaCl-Lösung. Viermalige Extraktion mit Diethylether, Vereinigung der organischen Phasen, Einengen am Rotationsverdampfer auf die Hälfte des Volumens, dreimaliges Waschen mit gesättigter NaCl-Lösung, Trocknen über Natriumsulfat, Filtrieren und erneutes Einengen am Rotationsverdampfer hinterlassen als Rohprodukt ein gelbes Öl, das, flashsäulenchromatographisch an Kieselgel gereinigt (Fließmittel: Petrolether/Ethylacetat 90:10), das Produkt in Form eines farblosen Öls liefert.
Ausbeute: 0.765 g (1.60 mmol); 87%
C₃₀H₄₁NO₄ (479.7)
¹H-NMR (CDCl₃): δ (ppm) = 0.89 (t, J = 7 Hz, 3H), 1.29 (m, 10H), 1.58 (m, 2H), 1.62 (s, 9H), 2.55 (t, J = 8 Hz, 2H), 3.83 (dd, J = 10 Hz und J = 5 Hz, 1H), 3.93 (dd, J = 10 Hz und J = 4 Hz, 1 H), 4.33 (m, 2H), 4.42 (m, 1H), 6.60 (dd, J = 3 Hz und J = 1 Hz, 1H), 6.80 (d, J = 9 Hz, 2H), 7.08 (d, J = 9 Hz, 2H), 7.20 (d, J = 3 Hz, 1 H), 7.37 (d, J = 9 Hz, 1H), 7.86 (dd, J = 9 Hz und J = 2 Hz, 1H), 8.33 (d, J = 2 Hz, 1H)

### C. tert-Butyl-1-[3-(4-octylphenoxy)-2-oxopropyl]indol-5-carboxylat

5.33 g (52.2 mmol) Essigsäureanhydrid werden mit 30 ml absol. DMSO versetzt, 10 min bei Raumtemperatur gerührt und zu einer Lösung von 0.626 g (1.31 mmol) *tert-*Butyl-1-[2-hydroxy-3-(4-octylphenoxy)propyl]indol-5-carboxylat in 20 ml absol. DMSO getropft. Nach siebzehnstündigem Rühren wird in eine Mischung aus 5%iger Natriumhydrogencarbonat- und gesättigter NaCl-Lösung (1:1, v/v) gegossen und 10 min lang hydrolysiert. Viermalige Extraktion mit Diethylether, Vereinigung der organischen Phasen, Einengen am Rotationsverdampfer auf die Hälfte des Volumens, dreimaliges Waschen mit gesättigter NaCl-Lösung, Trocknen über Natriumsulfat, Filtrieren und erneutes Einengen am Rotationsverdampfer hinterlassen als Rohprodukt ein gelbes Öl, das flashsäulenchromatographisch an Kieselgel gereinigt (Fließmittel: Petrolether/Ethylacetat 95:5), das Produkt in Form eines farblosen Öls liefert.
Ausbeute: 0.232 g (0.486 mmol); 37 %
C₃₀H₃₉NO₄ (477.6)
¹H-NMR (CDCl₃): δ (ppm) = 0.89 (t, J = 7 Hz, 3H), 1.30 (m, 10H), 1.61 (m, 2H), 1.63 (s, 9H), 2.59 (t, J = 8 Hz, 2H), 4.64 (s, 2H), 5.21 (s, 2H), 6.68 (dd, J = 3 Hz und J = 1 Hz, 1 H), 6.84 (d, J = 9 Hz, 2H), 7.09 (d, J = 9 Hz, 1H und d, J = 3 Hz, 1H), 7.15 (d, J = 9 Hz, 2H), 7.87 (dd, J = 9 Hz und J = 2 Hz, 1 H), 8.35 (dd, J = 2 Hz und J = 1 Hz, 1H)

### D. 1-[3-(4-Octylphenoxy)-2-oxopropyl]indol-5-carbonsäure

0.222 g (0.465 mmol) *tert-*Butyl-1-[3-(4-octylphenoxy)-2-oxopropyl]indol-5-carboxylat werden in 60 ml absol. Dichlormethan gelöst und mit 3.98 g (34.9 mmol) Trifluoressigsäure versetzt. Nach vierstündigem Rühren bei Raumtemperatur wird am Rotationsverdampfer bis zur Trockene eingeengt. Zweimaliges Versetzen mit Hexan sowie jeweiliges Einengen am Rotationsverdampfer bis zur Trockene hinterlassen als Rohprodukt einen bräunlichen Feststoff, der, chromatographisch an einer RP-HPLC-Säule gereinigt (stationäre Phase: Kromasil, mobile Phase Acetonitril/Wasser 80:20), das Produkt in Form eines weißen Feststoffes liefert.
Ausbeute: 0.134 g (0.318 mmol); 68 %
Schmp.: 122-124 °C
C₂₆H₃₁NO₄ (421.5)
¹H-NMR (CDCl₃): δ (ppm) = 0.88 (t, J = 7 Hz, 3H), 1.29 (m, 10H), 1.59 (m, 2H), 2.58 (t, J = 8 Hz, 2H), 4.66 (s, 2H), 5.25 (s, 2H), 6.71 (d, J = 3 Hz, 1H), 6.85 (d, J = 8 Hz, 2H), 7.14 (m, 4H), 7.96 (d, J = 9 Hz, 1 H), 8.49 (s, 1 H)

### Beispiel 10

### 1-[3-(4-Octylphenoxy)-2-oxopropyl]indol-4-carbonsäure

### A. tert-Butylindol-4-carboxylat

Die Darstellung erfolgt ausgehend von Indol-4-carbonsäure analog der Synthese der Stufe A von Beispiel 9.
Schmp.: 96°C
C₁₃H₁₅NO₂ (217.3)
¹H-NMR (CDCl₃): δ (ppm) = 1.68 (s, 9H), 7.17-7.19 (m, 1H), 7.21-7.25 (m, 1H), 7.33-7.34 (m, 1H), 7.57 (d, J = 8 Hz, 1 H), 7.88 (dd, J = 8 Hz und J = 1 Hz, 1H), 8.39 (s, breit, 1 H)

### B. tert-Butyl-1-[2-hydroxy-3-(4-octylphenoxy)propyl]indol-4-carboxylat

Die Darstellung erfolgt ausgehend von *tert*-Butylindol-4-carboxylat analog der Synthese der Stufe B von Beispiel 9. Davon abweichend beträgt die Reaktionszeit nur 3 h. Die chromatographische Reinigung erfolgt zunächst an Kieselgel (Fließmittel Petrolether/Ethylacetat 85:15) und danach an RP18-Material (Fließmittel Acetonitril/Wasser 80:20). Das Produkt fällt als Öl an.
C₃₀H₄₁NO₄ (479.7)
¹H-NMR (CDCl₃): δ (ppm) = 0.88 (t, J = 7 Hz, 3H), 1.27-1.31 (m, 10H), 1.56-1.64 (m, 2H), 1.67 (s, 9H), 2.38 (s, breit, 1H), 2.55 (t, J = 8 Hz, 2H), 3.83 (dd, J = 9 Hz und J = 5 Hz, 1H), 3.92 (dd, J = 9 Hz und J = 5 Hz, 1H), 4.34-4.38 (m, 2H), 4.47 (dd, J = 17 Hz und J=8Hz, 1H), 6.79 (d, J = 9 Hz, 2H), 7.08 (d, J = 8 Hz, 2H), 7.13 (d, J = 3 Hz, 1 H), 7.20-7.24 (m, 1 H), 7.27 (d, J = 3 Hz, 1 H), 7.57 (dd, J = 8 Hz und J = 1 Hz, 1 H), 7.86 (d, J = 7 Hz, 1 H)

### C. tert-Butyl-1-[3-(4-octylphenoxy)-2-oxopropyl]indol-4-carboxylat

Die Darstellung erfolgt ausgehend von *tert*-Butyl-1-[2-hydroxy-3-(4-octylphenoxy)propyl]indol-4-carboxylat analog der Synthese der Stufe C von Beispiel 9. Davon abweichend beträgt die Reaktionszeit nur 15 h. Die chromatographische Reinigung erfolgt an Kieselgel (Fließmittel Petrolether/Ethylacetat 93:7). Das Produkt fällt als Feststoff an.
Schmp.: 100°C
C₃₀H₃₉NO₄ (477.6)
¹H-NMR (CDCl₃): δ (ppm) = 0.88 (t, J = 7 Hz, 3H), 1.27-1.31 (m, 10H), 1.56-1.60 (m, 2H), 1.67 (s, 9H), 2.57 (t, J = 8 Hz, 2H), 4.61 (s, 2H), 5.22 (s, 2H), 6.82 (d, J = 9 Hz, 2H), 7.14 (d, J = 9 Hz, 2H), 7.16 (d, J = 3 Hz, 1H), 7.20-7.22 (m, 2H), 7.24-7.28 (m, 1H), 7.88 (dd, J = 7 Hz und J = 1 Hz, 1 H)

### D. 1-[3-(4-Octylphenoxy)-2-oxopropyl]indol-4-carbonsäure

63 mg (0.13 mmol) Keton werden in 15 ml absol. Dichormethan gelöst und mit 1.12 g (9.82 mmol) Trifluoressigsäure versetzt. Nach zweistündigem Rühren bei Raumtemperatur wird am Rotationsverdampfer bis zur Trockene eingeengt. Dreimaliges Versetzen mit je 10 ml einer Mischung aus Petrolether und Ethylacetat (1:2) sowie jeweiliges Einengen am Rotationsverdampfer bis zur Trockene hinterlassen als Rohprodukt einen Feststoff, der aus Petrolether/Ethylacetat (2:1) umkristallisiert wird.
Ausbeute: 48 mg (0.11 mmol); 86%
Schmp.: 160-161 °C
C₂₆H₃₁NO₄ (421.5)
¹H-NMR (CDCl₃): δ (ppm) = 0.89 (t, J = 7 Hz, 3H), 1.28-1.32 (m, 10H), 1.56-1.63 (m, 2H), 2.58 (t, J = 8 Hz, 2H), 4.64 (s, 2H), 5.26 (s, 2H), 6.84 (d, J = 9 Hz, 2H), 7.15 (d, J = 9 Hz, 2H), 7.22 (d, 1 H), 7.25-7.29 (m, 1H), 7.31 (d, J = 3 Hz, 1H), 7.35 (d, J = 8 Hz, 1H), 8.02 (d, J = 7 Hz, 1H)

### Beispiel 11

### 1-[3-(4-Octylphenoxy)-2-oxopropyl]indol-6-carbonsäure

### A. tert-Butylindol-6-carboxylat

Die Darstellung erfolgt ausgehend von lndol-6-carbonsäure analog der Synthese der Stufe A von Beispiel 9. Das Produkt wird zusätzlich aus Petrolether umkristallisiert.
Schmp.: 100-101 °C
C₁₃H₁₅NO₂ (217.3)
¹H-NMR (CDCl₃): δ (ppm) = 1.63 (s, 9H), 6.59 (m, 1H), 7.35 (m, 1H), 7.63 (d, J = 8 Hz, 1H), 7.77 (dd, J = 8 Hz und J = 1 Hz, 1 H), 8.13 (m, 1H), 8.55 (s, breit, 1H)

### B. tert-Butyl-1-[2-hydroxy-3-(4-octylphenoxy)propyl]indol-6-carboxylat

Die Darstellung erfolgt ausgehend von *tert*-Butylindol-6-carboxylat analog der Synthese der Stufe B von Beispiel 9. Davon abweichend beträgt die Reaktionszeit 5 h. Die chromatographische Reinigung erfolgt zunächst an Kieselgel (Fließmittel: Petrolether/Ethylacetat 9:1) und danach an RP18-Material (Fließmittel Acetonitril/Wasser 4:1). Das Produkt fällt als Öl an.
C₃₀H₄₁NO₄ (479.7)
¹H-NMR (CDCl₃): δ (ppm) = 0.88 (t, J = 7 Hz, 3H), 1.26-1.30 (m, 10H), 1.56-1.60 (m, 2H), 1.62 (s, 9H), 2.45 (s, breit, 1H), 2.54 (t, J = 8 Hz, 2H), 3.84 (dd, J = 10 Hz und J = 5 Hz, 1 H), 3.93 (dd, J = 10 Hz und J = 4 Hz, 1 H), 4.33-4.37 (m, 2H), 4.48 (dd, J = 17 Hz und J = 8 Hz, 1 H), 6.54 (dd, J = 3 Hz und J = 1 Hz, 1 H), 6.80 (d, J = 9 Hz, 2H), 7.08 (d, J = 9 Hz, 2H), 7.30 (d, J = 3 Hz, 1H), 7.61 (d, J = 8 Hz, 1 H), 7.75 (dd, J = 8 Hz und J = 1 Hz, 1 H), 8.11 (m, 1 H)

### C. tert-Butyl-1-[3-(4-octylphenoxy)-2-oxopropyl]indol-6-carboxylat

Die Darstellung erfolgt ausgehend von *tert*-Butyl-1-[2-hydroxy-3-(4-octylphenoxy)propyl]indol-6-carboxylat analog der Synthese der Stufe C von Beispiel 9. Davon abweichend beträgt die Reaktionszeit nur 16 h. Das Produkt fällt als Öl an.
C₃₀H₃₉NO₄ (477.6)
¹H-NMR (CDCl₃): δ (ppm) = 0.89 (t, J = 7 Hz, 3H), 1.27-1.31 (m, 10H), 1.57-1.62 (m, 2H), 1.62 (s, 9H), 2.57 (t, J = 8 Hz, 2H), 4.64 (s, 2H), 5.28 (s, 2H), 6.62 (dd, J = 3 Hz und J = 1 Hz, 1 H), 6.84 (d, J = 8 Hz, 2H), 7.14 (d, J = 8 Hz, 2H), 7.16 (d, J = 3 Hz, 1H), 7.63 (dd, J = 8 Hz und J = 1 Hz, 1 H), 7.74 (dd, J = 8 Hz und J = 1 Hz, 1H), 7.89 (m, 1 H)

### D. 1-[3-(4-Octylphenoxy)-2-oxopropyl]indol-6-carbonsäure

59 mg (0.12 mmol) *tert*-Butyl-1-[3-(4-octylphenoxy)-2-oxopropyl]indol-6-carboxylat werden in 15 ml absol. Dichlormethan gelöst und mit 1.05 g (9.20 mmol) Trifluoressigsäure versetzt.
Nach zweieinhalbstündigem Rühren bei Raumtemperatur wird am Rotationsverdampfer bis zur Trockene eingeengt. Dreimaliges Versetzen mit je 10 ml einer Mischung aus Petrolether und Ethylacetat (1:2) sowie jeweiliges Einengen am Rotationsverdampfer bis zur Trockene hinterlassen als Rohprodukt einen Feststoff, der chromatographisch an einer RP-HPLC Säule (stationäre Phase: Kromasil, mobile Phase: Acetonitril/Wasser 9:1) gereinigt wird. Ausbeute: 30 mg (0.07 mmol); 57%
Schmp.: 180°C
C₂₆H₃₁NO₄ (421.5)
¹H-NMR (DMSO-d₆): δ (ppm) = 0.83 (t, J = 7 Hz, 3H), 1.21-1.27 (m, 10H), 1.48-1.62 (m, 2H), 2.48 (t, J = 8 Hz, 2H), 5.00 (s, 2H), 5.48 (s, 2H), 6.55 (dd, J = 3 Hz und J = 1 Hz, 1H), 6.86 (d, J = 9 Hz, 2H), 7.08 (d, J = 9 Hz, 2H), 7.46 (d, J = 3 Hz, 1H), 7.58-7.62 (m, 2H), 8.06 (d,J= 1Hz,1H)

### Beispiel 12

### 1-(5-Methylindol-1-yl)-3-(4-octylphenoxy)propan-2-on

### A. 1-(5-Methylindol-1-yl)-3-(4-octylphenoxy)propan-2-ol

Die Darstellung erfolgt ausgehend von 5-Methylindol analog der Synthese der Stufe B von Beispiel 9. Davon abweichend wird die Umsetzung bei Raumtemperatur durchgeführt. Die Reaktionszeit beträgt 23 h. Die chromatographische Reinigung erfolgt an Kieselgel (Fließmittel: Petrolether/Ethylacetat 95:5). Das Produkt fällt als Öl an.
C₂₆H₃₅NO₂ (393.6)
¹H-NMR (CDCl₃): δ (ppm) = 0.89 (t, J = 7 Hz, 3H), 1.27-1.31 (m, 10H), 1.54-1.58 (m, 2H), 2.36 (s, breit, 1H), 2.45 (s, 3H), 2.55 (t, J = 8 Hz, 2H), 3.85 (dd, J = 10 Hz und J = 5 Hz, 1 H), 3.98 (dd, J = 10 Hz und J = 4 Hz, 1 H), 4.31 (dd, J = 12 Hz und J = 7 Hz, 1H), 4.33-4.38 (m, 1H), 4.40 (dd, J = 12 Hz und J = 5 Hz), 6.42 (dd, J = 3 Hz und J = 1 Hz, 1 H), 6.81 (d, J = 9 Hz, 2H), 7.03 (dd, J = 8 Hz und J = 1 Hz, 1H), 7.08 (d, J = 9 Hz, 2H), 7.09 (d, J = 3 Hz, 1 H), 7.29 (d, J = 8 Hz, 1H), 7.41 (m, 1 H)

### B. 1-(5-Methylindol-1-yl)-3-(4-octylphenoxy)propan-2-on

Die Darstellung erfolgt ausgehend von 1-(5-Methylindol-1-yl)-3-(4-octylphenoxy)propan-2-ol analog der Synthese der Stufe C von Beispiel 9. Davon abweichend beträgt die Reaktionszeit 19 h. Die chromatographische Reinigung an Kieselgel erfolgt mit dem Fließmittel Petrolether/Ethylacetat 97:3. Das Produkt fällt als Feststoff an.
Schmp.: 75°C
C₂₆H₃₃NO₂ (391.6)
¹H-NMR (CDCl₃): δ (ppm) = 0.88 (t, J = 7 Hz, 3H), 1.27-1.31 (m, 10H), 1.55-1.59 (m, 2H), 2.44 (s, 3H), 2.56 (t, J = 8 Hz, 2H), 4.57 (s, 2H), 5.12 (s, 2H), 6.51 (d, J = 3 Hz, 1H), 6.79 (d, J = 9 Hz, 2H), 7.00-7.02 (m, 3H), 7.11 (d, J = 9 Hz, 2H), 7.42-7.43 (m, 1H)

### Beispiel 13

### 1-(5-Chlorindol-1-yl)-3-(4-octylphenoxy)propan-2-on

### A. 1-(5-Chlorindol-1-yl)-3-(4-octylphenoxy)propan-2-ol

Die Darstellung erfolgt ausgehend von 5-Chlorindol analog der Synthese der Stufe B von Beispiel 9. Davon abweichend wird die Umsetzung bei Raumtemperatur durchgeführt. Die Reaktionszeit beträgt 17 h. Die chromatographische Reinigung an Kieselgel erfolgt mit dem Fließmittel Petrolether/Ethylacetat 95:5. Das Produkt fällt als Öl an.
C₂₅H₃₂ClNO₂ (414.0)
¹H-NMR (CDCl₃): δ (ppm) = 0.88 (t, J = 7 Hz, 3H), 1.26-1.31 (m, 10H), 1.55-1.59 (m, 2H), 2.36 (s, breit, 1 H), 2.55 (t, J = 8 Hz, 2H), 3.83 (dd, J = 10 Hz und J = 5 Hz, 1H), 3.92 (dd, J = 10 Hz und J = 4 Hz, 1H), 4.27-4.42 (m, 3H), 6.45 (dd, J = 3 Hz und J = 1 Hz, 1H), 6.80 (d, J = 9 Hz, 2H), 7.09 (d, J = 9 Hz, 2H), 7.13 (dd, J = 9 Hz und J = 2 Hz, 1H), 7.16 (d, J = 3 Hz, 1H), 7.30 (d, J = 9 Hz, 1 H), 7.58 (d, J = 2 Hz, 1 H)

### B. 1-(5-Chlodndol-1-yl)-3-(4-octylphenoxy)propan-2-on

Die Darstellung erfolgt ausgehend von 1-(5-Chlorindol-1-yl)-3-(4-octylphenoxy)propan-2-ol analog der Synthese der Stufe C von Beispiel 9. Davon abweichend beträgt die Reaktionszeit 16 h. Das Produkt fällt als Feststoff an und wird aus Petrolether umkristrallisiert.
Schmp.: 77°C
C₂₅H₃₀ClNO₂ (412.0)
¹H-NMR (CDCl₃): δ (ppm) = 0.89 (t, J = 7 Hz, 3H), 1.28-1.32 (m, 10H), 1.56-1.60 (m, 2H), 2.57 (t, J = 8 Hz, 2H), 4.61 (s, 2H), 5.17 (s, 2H), 6.52 (dd, J = 3 Hz und J = 1 Hz, 1 H), 6.82 (d, J = 9 Hz, 2H), 6.99 (d, J = 9 Hz, 1H), 7.05 (d, J = 3 Hz, 1 H), 7.12-7.15 (m, 3H), 7.60 (d, J = 2Hz, 1H)

### Beispiel 14

### 1-(5-Methoxyindol-1-yl)-3-(4-octylphenoxy)propan-2-on

### A. 1-(5-Methoxyindol-1-yl)-3-(4-octylphenoxy)propan-2-ol

Die Darstellung erfolgt ausgehend von 5-Methoxyindol analog der Synthese der Stufe B von Beispiel 9. Davon abweichend wird die Umsetzung bei Raumtemperatur und unter Lichtausschluss durchgeführt. Die Reaktionszeit beträgt 17 h. Die chromatographische Reinigung erfolgt an Kieselgel (Fließmittel: Petrolether/Ethylacetat 93:7). Das Produkt fällt als Öl an.
C₂₆H₃₅NO₃ (409.6)
¹H-NMR (CDCl₃): δ (ppm) = 0.89 (t, J = 7 Hz, 3H), 1.27-1.31 (m, 10H), 1.55-1.59 (m, 2H), 2.35 (d, J = 5 Hz, 1 H), 2.55 (t, J = 8 Hz, 2H), 3.84 (dd, J = 10 Hz und J=5 Hz, 1H), 3.85 (s, 3H), 3.91 (dd, J = 10 Hz und J = 4 Hz, 1 H), 4.27-4.40 (m, 3H), 6.43 (dd, J = 3 Hz und J = 1 Hz, 1 H), 6.80 (d, J = 9 Hz, 2H), 6.86 (dd, J = 9 Hz und J = 3 Hz, 1 H), 7.08 (d, J = 9 Hz, 2H), 7.09 (d, J = 3 Hz, 1H), 7.11 (d, J = 3 Hz, 1H), 7.28 (d, J = 9 Hz, 1H)

### B. 1-(5-Methoxyindol-1-yl)-3-(4-octylphenoxy)propan-2-on

Die Darstellung erfolgt ausgehend von 1-(5-Methoxyindol-1-yl)-3-(4-octylphenoxy)propan-2-ol analog der Synthese der Stufe C von Beispiel 9. Davon abweichend beträgt die Reaktionszeit 18 h. Die Reaktion wird unter Lichtausschluss durchgeführt. Das Produkt fällt als Feststoff an.
Schmp.: 85°C
C₂₆H₃₃NO₃ (407.6)
¹H-NMR (CDCl₃): δ (ppm) = 0.88 (t, J = 7 Hz, 3H), 1.27-1.31 (m, 10H), 1.55-1.57 (m, 2H), 2.56 (t, J = 8 Hz, 2H), 3.85 (s, 3H), 4.56 (s, 2H), 5.12 (s, 2H), 6.51 (dd, J = 3 Hz und J = 1 Hz, 1H), 6.80 (d, J = 9 Hz, 2H), 6.86 (dd, J = 9 Hz und J = 2 Hz, 1H), 7.01 (d, J = 9 Hz, 1H), 7.02 (d, J = 3 Hz, 1 H), 7.10 (d, J = 2 Hz, 1H), 7.12 (d, J = 9 Hz, 2H)

### Beispiel 15

### 1-(3-(4-Octylphenoxy)-2-oxopropyl]indol-5-carbonitril

### A. 1-[2-Hydroxy-3-(4-octylphenoxy)propyl]indol-5-carbonitril

Die Darstellung erfolgt ausgehend von Indol-5-carbonitril analog der Synthese der Stufe B von Beispiel 9. Davon abweichend beträgt die Reaktionszeit 5 h. Die chromatographische Reinigung erfolgt zunächst an Kieselgel (Fließmittel: Dichlormethan) und danach an RP18-Material (Fließmittel Acetonitril/Wasser 75:25). Das Produkt fällt als Öl an.
C₂₆H₃₂N₂O₂ (404.5)
¹H-NMR (CDCl₃): δ (ppm) = 0.89 (t, J = 7 Hz, 3H), 1.28-1.32 (m, 10H), 1.55-1.61 (m, 2H), 2.47 (s, breit, 1H), 2.56 (t, J = 8 Hz, 2H), 3.85 (dd, J = 9 Hz und J = 5 Hz, 1H), 3.95 (dd, J = 9 Hz und J = 4 Hz, 1 H), 4.33-4.39 (m, 2H), 4.47 (dd, J = 17 Hz und J = 7 Hz, 1 H), 6.60 (dd, J = 3 Hz und J = 1 Hz, 1 H), 6.83 (d, J = 9 Hz, 2H), 7.11 (d, J = 9 Hz, 2H), 7.29 (d, J = 3 Hz, 1H), 7.41 (dd, J=9 Hz und J = 2 Hz, 1 H), 7.46 (d, J = 9 Hz, 1H), 7.97 (s, 1 H)

### B. 1-(3-(4-Octylphenoxy)-2-oxopropyl]indol-5-carbonitril

Die Darstellung erfolgt ausgehend von 1-[2-Hydroxy-3-(4-octylphenoxy)propyljindol-5-carbonitril analog der Synthese der Stufe C von Beispiel 9. Davon abweichend beträgt die Reaktionszeit 15 h. Die chromatographische Reinigung an Kieselgel erfolgt mit dem Fließmittel Petrolether/Ethylacetat 90:10. Das Produkt fällt als Feststoff an und wird aus Petrolether/Ethyiacetat 95:5 umkristallisiert.
Schmp.: 96 °C
C₂₆H₃₀N₂O₂ (402.5)
¹H-NMR (CDCl₃): δ (ppm) = 0.89 (t, J = 7 Hz, 3H), 1.28-1.32 (m, 10H), 1.58-1.62 (m, 2H), 2.59 (t, J = 8 Hz, 2H), 4.67 (s, 2H), 5.27 (s, 2H), 6.67 (dd, J = 3 Hz und J = 1 Hz, 1H), 6.85 (d, J = 9 Hz, 2H), 7.11 (d, J = 9 Hz, 1H), 7.14 (d, J = 3 Hz, 1H), 7.16 (d, J = 9 Hz, 2H), 7.41 (dd, J = 9 Hz und J = 2 Hz, 1 H), 7.99 (dd, J = 2 Hz und J = 1 Hz, 1 H)

### Beispiel 16

### 1-[3-(4-Octylphenoxy)-2-oxopropyl]indol-5-carbaldehyd

### A. 1-[2-Hydroxy-3-(4-octylphenoxy)propyl]indol-5-carbaldehyd

Die Darstellung erfolgt ausgehend von Indol-5-carbaldehyd analog der Synthese der Stufe B von Beispiel 9. Die chromatographische Reinigung erfolgt zunächst an Kieselgel (Fließmittel Dichlormethan) und danach an RP18-Material (Fließmittel: Acetonitril/Wasser 4:1). Das Produkt fällt als Feststoff an.
Schmp.: 79°C
C₂₆H₃₃NO₃ (407.6)
¹H-NMR (CDCl₃): δ (ppm) = 0.88 (t, J = 7 Hz, 3H), 1.26-1.30 (m, 10H), 1.55-1.59 (m, 2H), 2.44 (s, breit, 1 H), 2.55 (t, J = 8 Hz, 2H), 3.86 (dd, J = 10 Hz und J = 5 Hz, 1 H), 3.96 (dd, J = 10 Hz und J = 4 Hz, 1H), 4.34-4.40 (m, 2H), 4.48 (dd, J = 17 Hz und J = 7 Hz, 1H), 6.68 (dd, J = 3 Hz und J = 1 Hz, 1 H), 6.80 (d, J = 9 Hz, 2H), 7.09 (d, J = 9 Hz, 2H), 7.26 (d, J = 3 Hz, 1 H), 7.48 (d, J = 9 Hz, 1 H), 7.76 (dd, J = 9 Hz und J = 2 Hz, 1 H), 8.14 (d, J = 1 Hz, 1 H), 10.00 (s, 1 H)

### B. 1-(3-(4-Octylphenoxy)-2-oxopropyl]indol-5-carbaldehyd

Die Darstellung erfolgt ausgehend von 1-[2-Hydroxy-3-(4-octylphenoxy)propyl]indol-5-carbaldehyd analog der Synthese der Stufe C von Beispiel 9. Davon abweichend beträgt die Reaktionszeit 16 h. Die chromatographische Reinigung an Kieselgel erfolgt mit dem Fließmittel Petrolether/Ethylacetat 9:1. Das Produkt fällt als Feststoff an.
Schmp.: 96 °C
C₂₆H₃₁NO₃ (405.5)
¹H-NMR (CDCl₃): δ (ppm) = 0.88 (t, J = 7 Hz, 3H), 1.28-1.32 (m, 10H), 1.59-1.63 (m, 2H), 2.58 (t, J = 8 Hz, 2H), 4.67 (s, 2H), 5.26 (s, 2H), 6.74 (dd, J = 3 Hz und J = 1 Hz, 1H), 6.85 (d, J = 9 Hz, 2H), 7.12 (d, J = 3 Hz, 1 H), 7.15 (d, J = 9 Hz, 2H), 7.16 (d, J = 9 Hz, 1H), 7.76 (dd, J = 9 Hz und J = 1 Hz, 1H), 8.16-8.17 (m, 1H), 10.02 (s, 1H)

### Beispiel 17

### Methyl-1-(3-(4-octylphenoxy)-2-oxopropyl]indol-5-carboxylat

### A. Methyl-1-[2-hydroxy-3-(4-octylphenoxy)propyl]indol-5-carboxylat

Die Darstellung erfolgt ausgehend von Methylindol-5-carboxylat analog der Synthese der Stufe B von Beispiel 9. Davon abweiched beträgt die Reaktionszeit 6 h. Das Produkt fällt als Feststoff an.
Schmp.: 58°C
C₂₇H₃₅NO₄ (437.6)
¹H-NMR (CDCl₃): δ (ppm) = 0.89 (t, J = 7 Hz, 3H), 1.27-1.30 (m, 10H), 1.55-1.59 (m, 2H), 2.41 (s, breit, 1H), 2.55 (t, J = 8 Hz, 2H), 3.85 (dd, J = 10 Hz und J = 5 Hz, 1H), 3.93 (s, 3H), 3.95 (dd, J = 10 Hz und J = 5 Hz, 1 H), 4.33-4.39 (m, 2H), 4.43-4.49 (m, 1 H), 6.61 (dd, J = 3 Hz und J = 1 Hz, 1H), 6.80 (d, J = 9 Hz, 2H), 7.09 (d, J = 9 Hz, 2H), 7.22 (d, J = 3 Hz, 1H), 7.40 (d, J = 9 Hz, 1 H), 7.90 (dd, J = 9 Hz und J = 2 Hz, 1 H), 8.39 (m, 1 H)

### B. Methyl-1-(3-(4-octylphenoxy)-2-oxopropyl]indol-5-carboxylat

Die Darstellung erfolgt ausgehend von Methyl-1-[2-hydroxy-3-(4-octylphenoxy)propyl]indol-5-carboxylat analog der Synthese der Stufe C von Beispiel 9. Davon abweichend beträgt die Reaktionszeit 19 h. Das Produkt fällt als Feststoff an und wird (ohne chromatographische Reinigung) aus Petrolether/Ethylacetat 94:6 umkristallisiert.
Schmp.: 118 °C
C₂₇H₃₃NO₄ (435.6)
¹H-NMR (CDCl₃): δ (ppm) = 0.88 (t, J = 7 Hz, 3H), 1.27-1.31 (m, 10H), 1.57-1.60 (m, 2H), 2.57 (t, J = 8 Hz, 2H), 3.92 (s, 3H), 4.63 (s, 2H), 5.22 (s, 2H), 6.67 (dd, J = 3 Hz und J = 1 Hz, 1H), 6.83 (d, J = 9 Hz, 2H), 7.08-7.10 (m, 2H), 7.14(d, J = 9 Hz, 2H), 7.89 (dd, J = 9 Hz und J = 2 Hz, 1 H), 8.39 (m, 1 H)

### Beispiel 18

### 3-tert-Butyl-1-[3-(4-octylphenoxy)-2-oxopropyl]indol-5-carbonsäure

Die Verbindung fällt bei der Synthese von 1-[3-(4-Octylphenoxy)-2-oxopropyl]indol-5-carbonsäure (Beispiel 10) als Nebenprodukt an und lässt sich von diesem bei der Reinigung mittels RP-HPLC abtrennen.
Schmp.: 146-147 °C
C₃₀H₃₉NO₄ (477.6)
¹H-NMR (CDCl₃): δ (ppm) = 0.88 (t, J = 6 Hz, 3H), 1.26-1.30 (m, 10H), 1.46 (s, 9H), 1.56-1-60 (m, 2H), 2.57 (t, J = 8 Hz, 2H), 4.62 (s, 2H), 5.15 (s, 2H), 6.82 (m, 3H), 7.09 (d, J = 9 Hz, 1 H), 7.14 (d, J = 9 Hz, 2H), 7.93 (dd, J = 8 Hz und J = 1 Hz, 1 H), 8.65 (s, 1 H)

### Beispiel 19

### 3-Chlor-1-[3-(4-octylphenoxy)-2-oxopropyl]indol-5-carbonsäure

### A. tert-Butyl-3-chlorindol-5-carboxylat

Eine Lösung von 389 mg (1.79 mmol) *tert*-Butylindol-5-carboxylat in 12 ml Methanol wird mit 335 mg (2.50 mmol) *N*-Chlorsuccinimid versetzt und über Nacht bei Raumtemperatur gerührt. Das Methanol wird abrotiert und der erhaltene Rückstand in 15 ml Ethylacetat aufgenommen. Die organische Phase wird zweimal mit 1 M Natriumhydrogencarbonatlösung gewaschen, über Natriumsulfat getrocknet und einrotiert. Säulenchromatographische Aufarbeitung des Rückstandes an Kieselgel (Fließmittel: Hexan/Ethylacetat 9:1) ergibt einen Feststoff.
Ausbeute: 190 mg (0.76 mmol); 42 %).
C₁₃H₁₄ ClNO₂ (251.1)
Schmp.: 120°C
¹H-NMR (CDCl₃): δ (ppm) = 1.64 (s, 9H), 7.22 (d, J = 1 Hz, 1H), 7.35 (d, J = 9 Hz, 1H), 7.90 (d, J = 9 Hz, 1 H), 8.33 (d, J = 1 Hz, 1 H), 8.39 (s, 1 H)

### B. tert-Butyl-3-chlor-1-[2-hydroxy-3-(4-octylphenoxy)propyl]indol-5-carboxylat

Die Darstellung erfolgt ausgehend von *tert-*Butyl-3-chlorindol-4-carboxylat analog der Synthese der Stufe B von Beispiel 9. Davon abweichend beträgt die Reaktionszeit 8 h. Die chromatographische Reinigung an Kieselgel erfolgt mit Dichlormethan als Fließmittel. Das Produkt fällt als Öl an.
C₃₀H₄₀ClNO₄ (514.1)
¹H-NMR (CDCl₃): δ (ppm) = 0.86 (t, J = 7 Hz, 3H), 1.28 (m, 10H), 1.59 (m, 2H), 1.63 (s, 9H), 2.53 (s, breit, 1H), 2.56 (t, J = 8 Hz, 2H), 3.83 (dd, J = 10 Hz und J = 5 Hz, 1H), 3.93 (m, 1H), 4.28-4.40 (m, 2H), 4.28-4.40 (m, 3H), 6.79 (d, J = 9 Hz, 2H), 7.09 (d, J = 9 Hz, 2H), 7.21 (s, 1 H), 7.36 (d, J = 9 Hz, 1H), 7.88 (dd, J = 9 Hz und J = 2 Hz, 1 H), 8.30 (d, J = 2 Hz, 1 H)

### C. tert-Butyl-3-chlor1-[3-(4-octylphenoxy)-2-oxopropyl]indol-5-carboxylat

Die Darstellung erfolgt ausgehend von teft-Butyl-3-chlor-1-[2-hydroxy-3-(4-octylphenoxy)propyl]indol-5-carboxylat analog der Synthese der Stufe C von Beispiel 9. Die chromatographische Reinigung an Kieselgel erfolgt mit dem Fließmittel Petrolether/Ethylacetat 9:1. Das Produkt fällt als Öl an.
C₃₀H₃₈ClNO₄ (512.1)
¹H-NMR (CDCl₃): δ (ppm) = 0.89 (t, J = 7 Hz, 3H), 1.26 (m, 10H), 1.59 (m, 2H), 1.64 (s, 9H), 2.58 (t, J = 8 Hz, 2H), 4.66 (s, 2H), 5.18 (s, 2H), 6.84 (d, J = 8 Hz, 2H), 7.06 (m, 2H), 7.16 (d, J = 8 Hz, 2H), 7.94 (d, J = 9 Hz, 1 H), 8.31 (s, 1 H)

### D. 3-Chlor-1-[3-(4-octylphenoxy)-2-oxopropyl]indol-5-carbonsäure

Die Darstellung erfolgt ausgehend von *tert*-Butyl-3-chlor-1-[3-(4-octylphenoxy)-2-oxopropyl]indol-5-carboxylat analog der Synthese der Stufe D von Beispiel 9. Das Produkt fällt als Feststoff an und wird (ohne chromatographische Reinigung) aus Hexan/Tetrahydrofuran umkristallisiert.
Schmp.: 157°C
C₂₆H₃₀ClNO₄ (456.0)
¹H-NMR (CDCl₃): δ (ppm) = 0.88 (t, J = 6 Hz, 3H), 1.28 (m, 10H), 1.62 (m, 2H), 2.58 (t, J = 8 Hz, 2H), 4.68 (s, 2H), 5.22 (s, 2H), 6.91 (d, J = 8 Hz, 2H), 7.09 (m, 2H), 7.16 (d, J = 8 Hz, 2H), 7.97 (d, J = 9 Hz, 1H), 8.47 (s, 1 H)

### Beispiel 20

### 3-Formyl-1-[3-(4-octylphenoxy)-2-oxopropyl]indol-5-carbonsäure

### A. tert-Butyl-3-formylindol-5-carboxylat

0.4 ml (4.87 mmol) Oxalylchlorid werden in einem Dreihalskolben unter Stickstoff in 15 ml absol. Dichlormethan gelöst und auf 0°C gekühlt. Anschließend gibt man 0.4 ml absol. Dimethylformamid gelöst in 15 ml absol. Dichlormethan hinzu. Die Mischung wird unter Eiskühlung für 20 Minuten gerührt, wobei eine Gasentwicklung und die Bildung eines farblosen Niederschlags beobachtet wird. Danach versetzt man mit 1.00 g (4.64 mmol) *tert-*Butylindol-5-carboxylat und lässt die Reaktionsmischung auf Raumtemperatur erwärmen. Man rührt weitere 20 min, überführt die Suspension mit Tetrahydrofuran in einen Rundkolben und engt am Rotationsverdampfer ein. Der Rückstand wird in 40 ml Tetrahydrofuran aufgenommen, mit 50 ml 20%iger Natriumacetatlösung versetzt und 30 Minuten unter Rückfluss erhitzt. Nach dem Abkühlen gibt man 30 ml 5%ige Natriumhydrogencarbonatlösung zu, extrahiert dreimal mit Ethylacetat, engt das Volumen der vereinigten organischen Phasen auf ca. 150 ml ein und wäscht mit gesättigter NaCl-Lösung. Die organische Phase wird über Natriumsulfat getrocknet und das Lösungsmittel am Rotationsverdampfer entfernt. Umkristallisation des Rohproduktes aus n-Hexan/Ethylacetat liefert das Produkt als Feststoff.
Ausbeute: 0.81 g (3.30 mmol); 71 %
C₁₄H₁₅ NO₃ (245.3)
Schmp.: 190 °C
¹H-NMR (DMSO-d₆): δ (ppm) = 1.55 (s, 9H), 7.56 (dd, J = 9 Hz und J = 1 Hz, 1H), 7.81 (dd, J = 9 Hz und J = 2 Hz, 1 H), 8.39 (d, J = 3 Hz, 1H), 8.67 (d, J = 2 Hz, 1 H), 9.94 (s, breit, 1 H), 12.40 (s, breit, 1 H)

### B. tert-Butyl-3-formyl-1-[2-hydroxy-3-(4-octylphenoxy)propyl]indol-5-carboxylat

Die Darstellung erfolgt ausgehend von *tert*-Butyl-3-formylindol-5-carboxylat analog der Synthese der Stufe B von Beispiel 9. Davon abweichend wird der Reaktionsansatz 12 h bei 120°C erhitzt. Die chromatographische Reinigung an Kieselgel erfolgt mit Petrolether/Ethylacetat 3:2 als Fließmittel. Das Produkt fällt als Öl an.
C₃₁H₄₁NO₅ (507.7)
¹H-NMR (CDCl₃): δ (ppm) = 0.88 (t, J = 7 Hz, 3H), 1.27 (m, 10H), 1.59 (m, 2H), 1.63 (s,9H), 2.55 (t, J = 8 Hz, 2H), 3.07 (s, breit, 1H), 3.98 (m, 2H), 4.37 (dd, J = 14 Hz und J = 7 Hz, 1 H), 4.51 (m, 1 H), 4.54 (dd, J = 14 Hz und J = 4 Hz, 1 H), 6.82 (d, J = 9 Hz, 2H), 7.10 (d, J = 9 Hz, 2H), 7.41 (d, J = 9 Hz, 1H), 7.86 (s, 1 H), 7.94 (d, J = 9 Hz, 1H), 8.81 (s, 1 H), 9.86 (s, 1 H)

### C. tert-Butyl-3-formyl-1-[3-(4-octylphenoxy)-2-oxopropyl]indol-5-carboxylat

Die Darstellung erfolgt ausgehend von *tert*-Butyl-3-formyl-1-[2-hydroxy-3-(4-octylphenoxy)propyl]indol-5-carboxylat analog der Synthese der Stufe C von Beispiel 9. Davon abweichend beträgt die Reaktionszeit 20 h. Die chromatographische Reinigung an Kieselgel erfolgt mit dem Fließmittel Petrolether/Ethylacetat 3:2. Das Produkt fällt als Öl an.
C₃₁H₃₉NO₅ (505.3)
¹H-NMR (CDCl₃): δ (ppm) = 0.88 (t, J = 7 Hz, 3H), 1.29 (m, 10H), 1.63 (m, 11 H), 2.58 (t, J = 8 Hz, 2H), 4.71 (s, 2H), 5.33 (s, 2H), 6.87 (d, J = 8 Hz, 2H), 7.08 (d, J = 9 Hz, 1 H), 7.17 (d, J = 8 Hz, 2H), 7.72 (s, 1 H), 7.95 (d, J = 9 Hz, 1 H), 8.92 (s, 1 H), 10.05 (s, 1 H)

### D. 3-Formyl-1-[3-(4-octylphenoxy)-2-oxopropyl]indol-5-carbonsäure

Die Darstellung erfolgt ausgehend von *tert*-Butyl-3-formyl-1-[3-(4-octylphenoxy)-2-oxopropyl]indol-5-carboxylat analog der Synthese der Stufe D von Beispiel 9. Davon abweichend beträgt die Reaktionszeit nur 3 h. Die chromatographische Reinigung mittels RP-HPLC erfolgt mit dem Fließmittel Acetonitril/Wasser 9:1. Das Produkt fällt als Feststoff an.
Schmp.:193°C
C₂₇H₃₁NO₅ (449.5)
¹H-NMR (DMSO-d₆): δ (ppm) = 0.83 (t, J = 7 Hz, 3H), 1.24 (m, 10H), 1.51 (m, 2H), 2.51 (t, J = 8 Hz, 2H), 5.03 (s, 2H), 5.58 (s, 2H), 6.90 (d, J = 9 Hz, 2H), 7.11 (d, J = 9 Hz, 2H), 7.60 (d, J = 9 Hz, 1H), 7.86 (dd, J = 9 Hz und J = 2 Hz, 1 H), 8.29 (s, 1 H), 8.74 (d, J = 2 Hz, 1 H), 9.97 (s, 1 H)

### Beispiel 21

### 3-Acetyl-1-[3-(4-octylphenoxy)-2-oxopropyl]indol-5-carbonsäure

### A. Methyl-3-acetylindol-5-carboxylat

In einem Dreihalskolben legt man unter Stickstoff 4.20 ml (9.25 mmol) einer 2.2 M ZnCl₂-Lösung in 20 ml absol. Dichlormethan vor und kühlt die Lösung mit einem Eisbad auf 0°C ab. Anschließend tropft man langsam 5.62 ml (8.99 mmol) einer 1.6 M n-Butyllithiumlösung zu. Man erwärmt die Reaktionsmischung auf Raumtemperatur, lässt für eine Stunde rühren, gibt dann die Lösung von 1.50 g (8.56 mmol) Methylindol-5-carboxylat in 20 ml absol. Dichlormethan zu und rührt erneut für eine Stunde bei Raumtemperatur. Anschließend kühlt man den Ansatz auf 0°C ab und gibt vorsichtig 1.28 ml (18.1 mmol) Acetylchlorid zu, wodurch eine orangefarbene Suspension entsteht. Diese wird eine Stunde bei Raumtemperatur gerührt. Anschließend fügt man 0.93 g (7.02 mmol) Aluminiumchlorid zu. Nach einer weiteren Stunde Rühren bei Raumtemperatur erfolgt Hydrolyse mit halbgesättigter NaCl-Lösung und Zugabe von 100 ml Ethylacetat und 20 ml Tetrahydrofuran. Man extrahiert dreimal mit Ethylacetat, wäscht die vereinigten organischen Phasen mit gesättigeter NaCl-Lösung und trocknet die organische Phase über Natriumsulfat. Das Lösungsmittels wird abdestilliert. Chromatographische Reinigung des Rückstandes an Kieselgel (Fließmittel: Hexan/Ethylacetat 3:2) liefert das Produkt als Feststoff.
Ausbeute: 0.70 g (3.23 mmol); 38 %
Schmp.: 235-236°C.
C₁₂H₁₁NO₃ (217.2)
¹H-NMR (DMSO-d₆): δ (ppm) = 2.49 (s, 3H), 3.87 (s, 3H), 7.53 (dd, J = 9 Hz und J = 1 Hz, 1 H), 7.82 (dd, J = 9 Hz und J = 2 Hz, 1H), 8.43 (d, J = 3 Hz, 1H), 8.85 (d, J = 1 Hz, 1H), 12.23 (s, breit, 1H)

### B. 3-Acetylindol-5-carbonsäure

0.686g (3.16 mmol) Methyl-3-acetylindol-5-carboxylat werden in der Hitze in 10 ml Tetrahydrofuran und 10 ml Ethylenglycol gelöst und mit 7.08 g (0.13 mol) Kaliumhydroxid versetzt. Nach dreißigminütigem Rühren unter Rückfluss wird das Reaktionsgemisch auf Raumtemperatur abgekühlt. Man entfernt das Tetrahydrofuran am Rotationsverdampfer und säuert die Lösung unter Eiskühlung mit 20 ml 6N Salzsäure an. Der ausgefallene hellviolette Niederschlag wird abgesaugt und getrocknet.
Ausbeute: 0.64 g (3.16 mmol)
Schmp.: 364°C.
C₁₁H₀₉ NO₃ (203.2)
¹H-NMR (DMSO-d₆): δ (ppm) = 2.47 (s, 3H), 7.50 (dd, J = 9 Hz und J = 1 Hz, 1H), 7.86 (dd, J = 9 Hz und J = 2 Hz, 1H), 8.41 (d, 1H), 8.82 (d, 1 H), 12.17 (s, breit, 1H), 12.57 (s, breit, 1 H)

### C. tert-Butyl-3-acetylindol-5-carboxylat

Die Darstellung erfolgt ausgehend von 3-Acetylindol-5-carbonsäure analog der Synthese der Stufe A von Beispiel 9. Die chromatographische Reinigung an Kieselgel erfolgt mit Hexan/Ethylacetat 3:2 als Fließmittel.
Schmp.: 214°C
C₁₅H₁₇ NO₃ (259.1)
¹ H-NMR (DMSO-*d₆*): δ (ppm) = 1.56 (s, 9H), 2.46 (s, 3H), 7.50 (d, J = 8 Hz, 1 H), 7.76 (d, J = 8 Hz, 1H), 8.40 (s, 1 H), 8.78 (s, 1H), 12.17 (s, breit, 1H)

### D. tert-Butyl-3-acetyl-1-[2-hydroxy-3-(4-octylphenoxy)propyl]indol-5-carboxylat

Die Darstellung erfolgt ausgehend von *tert*-Butyl-3-acetylindol-5-carboxylat analog der Synthese der Stufe B von Beispiel 9. Davon abweichend wird der Reaktionsansatz 26 h bei 120°C erhitzt. Die chromatographische Reinigung an Kieselgel erfolgt mit Petrolether/Ethylacetat 3:2 als Fließmittel. Das Produkt fällt als Öl an.
C₃₂H₄₃NO₅ (521.7)
¹H-NMR (CDCl₃): δ (ppm) = 0.88 (t, J = 7 Hz, 3H), 1.28 (m, 10H), 1.57 (m, 2H), 1.64 (s, 9H), 2.31 (s, 3H), 2.34 (s, breit, 1H), 2.55 (t, J = 8 Hz, 2H), 3.99 (m, 2H), 4.32 (dd, J = 14 Hz und J = 7 Hz, 1H), 4.50 (m, 1H), 4.53 (dd, J = 12 Hz und J = 4 Hz, 1H), 6.75 (d, J = 9 Hz, 2H), 7.10 (d, J = 9 Hz, 2H), 7.38 (d, J = 9 Hz, 1H), 7.83 (s, 1H), 7.88 (dd, J = 7 Hz und J = 3 Hz, 1H), 8.84 (d,J=3 Hz,1 H)

### E. tert-Butyl-3-acetyl-1-[3-(4-octylphenoxy)-2-oxopropyl]indol-5-carboxylat

Die Darstellung erfolgt ausgehend von *tert*-Butyl-3-acetyl-1-[2-hydroxy-3-(4-octylphenoxy)propyl]indol-5-carboxylat analog der Synthese der Stufe C von Beispiel 9. Die chromatographische Reinigung an Kieselgel erfolgt mit dem Fließmittel Petrolether/Ethylacetat 17:3. Das Produkt fällt als Öl an.
C₃₂H₄₁NO₅ (519.3)
¹H-NMR (CDCl₃): δ (ppm) = 0.88 (t, J = 7 Hz, 3H), 1.30 (m, 10H), 1.58 (m, 2H), 1.62 (s, 9H), 2.53 (s, 3H), 2.59 (t, J = 8 Hz, 2H), 4.69 (s, 2H), 5.29 (s, 2H), 6.86 (d, J = 7 Hz, 2H), 7.07 (d, J = 9 Hz, 1H), 7.17 (d, J = 7 Hz; 2H), 7.71 (s, 1H), 7.92 (dd, J = 9 Hz und J = 2 Hz, 1H), 9.00 (d, J = 2 Hz, 1 H)

### F. 3-Acetyl-1-[3-(4-octylphenoxy)-2-oxopropyl]indol-5-carbonsäure

Die Darstellung erfolgt ausgehend von *tert*-Butyl-3-acetyl-1-[3-(4-octylphenoxy)-2-oxopropyl]indol-5-carboxylat analog der Synthese der Stufe D von Beispiel 9. Das Produkt fällt als Feststoff an und wird (ohne chromatographische Reinigung) aus Hexan/Tetrahydrofuran umkristallisiert.
Schmp.:197°C
C₂₈H₃₃NO₅ (463.6)
¹H-NMR (DMSO-d₆): δ (ppm) = 0.83 (t, J = 7 Hz, 3H), 1.23 (m, 10H), 1.51 (m, 2H), 2.47 (m, 2H), 2.51 (s, 3H), 5.03 (s, 2H), 5.53 (s, 2H), 6.89 (d, J = 8 Hz, 2H), 7.10 (d, J = 8 Hz, 2H), 7.56 (d, J = 9 Hz, 1H), 7.81 (dd, J = 9 Hz und J = 1 Hz, 1 H), 8.33 (s, 1 H), 8.83 (d, J = 1 Hz, 1 H)

### Beispiel 22

### 3-Methoxycarbonyl-1-[3-(4-octylphenoxy)-2-oxopropyl]indol-5-carbonsäure

### A. 5-tert-Butyl-3-methylindol-3,5-dicarboxylat

Zu einer Lösung von 0.70 g (2.83 mmol) *tert*-Butyl-3-formylindol-5-carboxylat in 30 ml Methanol gibt man 0.70 g (14.3 mmol) Natriumcyanid. Es entsteht eine gelbe Lösung, zu der spatelweise 4.92 g (57.2 mmol) aktivierter Braunstein zugefügt werden. Man lässt für 48 Stunden bei Raumtemperatur rühren. Nach Zugabe von 80 ml Dichlormethan und 5 g Celite wird die schwarze Suspension über einen Büchnertrichter abgesaugt, der Rückstand mit Dichlormethan gewaschen und das Filtrat zur Komplexierung von überschüssigen Cyanid-lonen mit einer frisch angesetzten Eisen(II)-sulfatlösung ausgeschüttelt. Die organische Phase wird mit gesättigter NaCl-Lösung gewaschen, über Natriumsulfat getrocknet und eingeengt. Man erhält das Produkt als Feststoff.
Ausbeute: 0.743 g (2.69 mmol); 94%
Schmp: 196°C.
C₁₅H₁₇ NO₄ (275.3)
¹H-NMR (CDCl₃): δ (ppm) = 1.58 (s, 9H), 3.94 (s, 3H), 7.42 (d, J = 9 Hz, 1H), 7.92 (d, 1H), 7.99 (d, 1 H), 8.84 (d, J = 1 Hz, 1 H), 9.36 (s, breit, 1 H)

### 8. 5-tert-Butyl-3-methyl-1-[2-hydroxy-3-(4-octylphenoxy)propyl]indol-3,5-dicarboxylat

Die Darstellung erfolgt ausgehend von 5-*tert*-Butyl-3-methylindol-3,5-dicarboxylat analog der Synthese der Stufe B von Beispiel 9. Davon abweichend wird der Reaktionsansatz 14 h bei 120°C erhitzt. Die chromatographische Reinigung an Kieselgel erfolgt mit Petrolether/Ethylacetat 7:3 als fließmittel. Das Produkt fällt als Öl an.
C₃₂H₄₃NO₆ (537.7)
¹H-NMR (CDCl₃): δ (ppm) = 0.89 (t, J = 7 Hz, 3H), 1.28 (m, 10H), 1.57 (m, 2H), 1.62 (s, 9H), 2.55 (t, J = 8 Hz, 2H), 2.78 (s, breit, 1H), 3.90 (m, 4H), 3.96 (dd, 1 H), 4.37 (m, 2H), 4.47 (dd, J = 10 Hz und J = 4 Hz, 1H), 6.80 (d, J = 7 Hz, 2H), 7.10 (d, J = 7 Hz, 2H), 7.40 (d, J = 9 Hz, 1 H), 7.91 (dd, J = 9 Hz und J = 2 Hz, 1 H), 7.96 (s, 1 H), 8.79 (d, J = 2 Hz, 1 H)

### C. 5-tert-Butyl-3-methyl-1-[3-(4octylphenoxy)-2-oxopropyl]indol-3,5-dicarboxylat

Die Darstellung erfolgt ausgehend von 5-*tert*-Butyl-3-methyl-1-[2-hydroxy-3-(4-octylphenoxy)propyl]indol-3,5-dicarboxylat analog der Synthese der Stufe C von Beispiel 9. Die chromatographische Reinigung an Kieselgel erfolgt mit dem Fließmittel Hexan/Ethylacetat 4:1. Das Produkt fällt als Öl an.
C₃₂H₄₁NO₆ (535.7)
¹H-NMR (CDCl₃): δ (ppm) = 0.89 (t, J = 7 Hz, 3H), 1.29 (m, 10H), 1.59 (m, 2H), 1.62 (s, 9H), 2.58 (t, J = 7 Hz, 2H), 3.93 (s, 3H), 4.69 (s, 2H), 5.27 (s, 2H), 6.85 (d, J = 9 Hz, 2H), 7.06 (dd, J = 9 Hz und J = 1 Hz, 1 H), 7.16 (d, J = 9 Hz, 2H), 7.72 (s, 1 H), 7.90 (dd, J = 9 Hz und J = 2 Hz, 1H), 8.83 (dd, J = 2 Hz und J = 1 Hz, 1H)

### D. 3-Methoxycarbonyl-1-[3-(4-octylphenoxy)-2-oxopropyl]indol-5-carbonsäure

Die Darstellung erfolgt ausgehend von 5-*tert*-Butyl-3-methyl-1-[3-(4-octylphenoxy)-2-oxopropyl]indol-3,5-dicarboxylat analog der Synthese der Stufe D von Beispiel 9. Davon abweichend beträgt die Reaktionszeit nur 2 h. Das Produkt fällt als Feststoff an und wird (ohne chromatographische Reinigung) aus Hexan/Tetrahydrofuran umkristallisiert.
Schmp.: 208 °C
C₂₈H₃₃NO₆ (479.6)
¹H-NMR (DMSO-d₆): δ (ppm) = 0.83 (t, J = 7 Hz, 3H), 1.22 (m, 10H), 1.50 (m, 2H), 2.53 (m, 2H), 3.83 (s, 3H), 5.00 (s, 2H), 5.52 (s, 2H), 6.88 (d, J = 9 Hz, 2H), 7.09 (d, J = 9 Hz, 2H), 7.57 (d, J = 9 Hz, 1H), 7.80 (dd, J = 9 Hz und J = 2 Hz, 1H), 8.16 (s, 1 H), 8.65 (d, J = 1 Hz, 1 H)

### Beispiel 23

### 3-tert-Butyl-1-[3-(4-octylphenoxy)-2-oxopropyl]indol-6-carbonsäure

Die Verbindung fällt bei der Synthese von 1-[3-(4-Octylphenoxy)-2-oxopropyl]indol-6-carbonsäure (Beispiel 11) als Nebenprodukt an und lässt sich von diesem bei der Reinigung mittels RP-HPLC abtrennen.
Schmp.: 145-146 °C
C₃₀H₃₉NO₄ (477.6)
¹H-NMR (CDCl₃): δ (ppm) = 0.89 (t, J = 6 Hz, 3H), 1.27-1.30 (m, 10H), 1.46 (s, 9H), 1.58-1.62 (m, 2H), 2.59 (t, J = 8 Hz, 2H), 4.64 (s, 2H), 5.23 (s, 2H), 6.86 (d, J = 9 Hz, 2H), 6.96 (s, 1 H), 7.16 (d, J = 9 Hz, 2H), 7.87 (m, 2H), 7.96 (m, 1 H)

### Beispiel 24

### 1-[3-(4-Decyloxyphenoxy)-2-oxopropyl]indol-5-carbonsäure

### A. tert-Butyl-1-[3-(4-decyloxyphenoxy)-2-hydroxypropyl]indol-5-carboxylat

Die Darstellung erfolgt ausgehend von *tert*-Butylindol-5-carboxylat und 2-(4-Decyloxyphenoxymethyl)oxiran analog der Synthese der Stufe B von Beispiel 9. Davon abweichend beträgt die Reaktionszeit 5 h. Die chromatographische Reinigung erfolgt zunächst an Kieselgel (Fließmittel: Petrolether/Ethylacetat 85:15) und danach an RP18-Material (Fließmittel Acetonitril/Wasser 4:1). Das Produkt fällt als Öl an.
C₃₂H₄₅NO₅ (523.7)
¹H-NMR (CDCl₃): δ (ppm) = 0.88 (t, J = 7 Hz, 3H), 1.27-1.34 (m, 12H), 1.42-1.46 (m, 2H), 1.62 (s, 9H), 1.75 (quin, J = 7 Hz, 2H), 2.45 (s, breit, 1H), 3.79 (dd, J = 10 Hz und J = 5 Hz, 1 H), 3.88-3.91 (m, 3H), 4.32-4.36 (m, 2H), 4.43 (dd, J = 16 Hz und J = 8 Hz, 1H), 6.59 (dd, J = 3 Hz und J = 1 Hz, 1 H), 6.78-6.85 (m, 4H), 7.19 (d, J = 3 Hz, 1 H), 7.36 (d, J = 9 Hz, 1 H), 7.85 (dd, J = 9 Hz und J = 2 Hz, 1 H), 8.32 (m, 1 H)

### B. tert-Butyl-1-[3-(4-decyloxyphenoxy)-2-oxopropyl]indol-5-carboxylat

Die Darstellung erfolgt ausgehend von *tert*-Butyl-1-[3-(4-decyloxyphenoxy)-2-hydroxypropyl]indol-5-carboxylat analog der Synthese der Stufe C von Beispiel 9. Die chromatographische Reinigung an Kieselgel erfolgt mit dem Fließmittel Petrolether/Ethylacetat 9:1. Das Produkt fällt als Feststoff an.
C₃₂H₄₃NO₅ (521.7)
¹H-NMR (CDCl₃): δ (ppm) = 0.88 (t, J = 7 Hz, 3H), 1.27-1.34 (m, 12H), 1.41-1.45 (m, 2H), 1.62 (s, 9H), 1.77 (quin, J = 7 Hz, 2H), 3.91 (t, J = 7 Hz, 2H), 4.59 (s, 2H), 5.21 (s, 2H), 6.67 (d, J = 3 Hz, 1H), 6.82-6.87 (m, 4H), 7.08 (d, J = 3 Hz, 1H), 7.09 (d, J = 9 Hz, 1H), 7.86 (dd, J = 9Hz und J = 2Hz, 1H), 8.34 (d, J = 1Hz, 1H)

### C. 1-[3-(4-Decyloxyphenoxy)-2-oxopropyl]indol-5-carbonsäure

50 mg (0.096 mmol) *tert*-Butyl-1-[3-(4-decyloxyphenoxy)-2-oxopropyl]indol-5-carboxylat werden in 15 ml absol. Dichlormethan gelöst und mit 0.82 g (7.19 mmol) Trifluoressigsäure versetzt. Nach vierstündigem Rühren bei Raumtemperatur wird bis zur Trockene am Rotationsverdampfer eingeengt. Dreimaliges Versetzen mit je 10 ml einer Mischung aus Petrolether und Ethylacetat (4:1) sowie jeweiliges Einengen am Rotationsverdampfer bis zur Trockene hinterlassen als Rohprodukt einen Feststoff, der chromatographisch an einer RP-HPLC Säule (stationäre Phase: Kromasil, Fließmittel: Acetonitril/Wasser 90:10) gereinigt wird.
Ausbeute: 25 mg (0.05 mmol); 56%
Schmp.: 137°C
C₂₈H₃₅NO₅ (465.6)
¹H-NMR (CDCl₃): δ (ppm) = 0.89 (t, J = 7 Hz, 3H), 1.28-1.33 (m, 12H), 1.43-1.49 (m, 2H), 1.75-1.82 (m, 2H), 3.93 (t, J = 7 Hz, 2H), 4.63 (s, 2H), 5.25 (s, 2H), 6.72 (dd, J = 3 Hz und J = 1 Hz, 1 H), 6.84-6.89 (m, 4H), 7.12 (d, J = 3 Hz, 1 H), 7.14 (d, J = 9 Hz, 1H), 7.97 (dd, J = 9 Hz und J = 2 Hz, 1 H), 8.50 (d, J = 1 Hz, 1 H)

### Beispiel 25

### 1-[3-(4-Octylphenoxy)-2-oxopropyl]indol-5-carbamid

### A. 1-[2-Hydroxy-3-(4-octylphenoxy)propyl]indol-5-carbamid

0.18 g (0.44 mmol) 1-[2-Hydroxy-3-(4-octylphenoxy)propyl]indol-5-carbonitril (Beispiel 15A) werden in 15 ml *tert-*Butanol gelöst, mit 0.23 g (3.6 mmol) pulverisiertem, 88%-igem Kaliumhydroxid versetzt und 11 h unter Rückfluss erhitzt. Nach Abkühlen erfolgt Hydrolyse in Wasser und Ansäuern mit 1N-Salzsäure. Dreimalige Extraktion mit Diethylether, Einengen am Rotationsverdampfer auf die Hälfte des Volumens, Waschen mit Wasser sowie mit gesättigter NaCl-Lösung, Trocknen über Natriumsulfat, Filtrieren und erneutes Einengen am Rotationsverdampfer hinterlassen als Rohprodukt einen gelblichen Feststoff, der, säulenchromatographisch an Kieselgel gereinigt (Fließmittel: Petrolether/Ethylacetat 1:1), das Produkt in Form eines Feststoffes liefert.
Ausbeute: 0.13 g (0.31 mmol); 70%
Schmp.: 118-119 °C
C₂₆H₃₄N₂O₃ (422.6)
¹H-NMR (CDCl₃): δ (ppm) = 0.88 (t, J = 7 Hz, 3H), 1.26-1.30 (m, 10H), 1.55-1.58 (m, 2H), 2.52 (m, 2H), 3.85 (dd, J = 10 Hz und J = 5 Hz, 1 H), 3.94 (dd, J = 10 Hz und J = 4 Hz, 1 H), 4.32-4.39 (m, 2H), 4.46 (dd, J = 17 Hz und J = 8 Hz, 1 H), 5.40-6.28 (s, breit, 2H), 6.59 (d, J = 3 Hz, 1H), 6.80 (d, J = 9 Hz, 2H), 7.09 (d, J = 9 Hz, 2H), 7.23 (d, J = 3 Hz, 1H), 7.42 (d, J = 9 Hz, 1 H), 7.66 (d, J = 9 Hz, 1 H), 8.12 (s, 1 H)

### B. 1-[3-(4-Octylphenoxy)-2-oxopropyl]indol-5-carbamid

Die Darstellung erfolgt ausgehend von 1-[2-Hydroxy-3-(4-octylphenoxy)propyl]indol-5-carbamid analog der Synthese der Stufe C von Beispiel 9. Davon abweichend beträgt die Reaktionszeit 19 h. Die chromatographische Reinigung an Kieselgel erfolgt mit dem Fließmittel Petrolether/Ethylacetat 1:1. Das Produkt fällt als Feststoff an.
Schmp.: 162-163 °C
C₂₆H₃₂N₂O₃ (420.5)
¹H-NMR (DMSO-*d*₆): δ (ppm) = 0.84 (t, J = 7 Hz, 3H), 1.23-1.26 (m, 10H), 1.49-1.53 (m, 2H), 2.47-2.51 (m, 2H), 4.97 (s, 2H), 5.39 (s, 2H), 6.54 (d, J = 3 Hz, 1H), 6.85 (d, J = 9 Hz, 2H), 7.07-7.10 (m, 3H), 7.33 (d, J = 3 Hz, 1 H), 7.38 (d, J = 9 Hz, 1 H), 7.65 (d, J = 9 Hz, 1 H), 7.83 (s, breit, 1 H), 8.13 (s, 1 H)

### Beispiel 26

### 1-[3-(4-Heptyloxyphenoxy)-2-oxopropyl]indol-5-carbamid

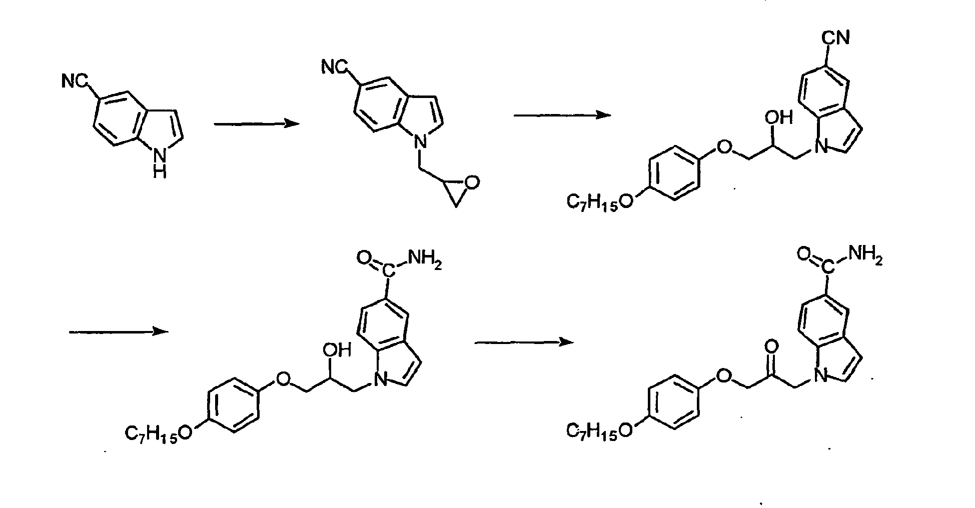

### A. 1-Oxiranylmethylindol-5-carbonitril

2.07 g (42 mmol) pulverisiertes, 88%-iges Kaliumhydroxid, 3.0 g (0.021 mol) Indol-5-carbonitril und 0.68 g (2 mmol) Tetrabutylammoniumbromid werden in der angegebenen Reihenolge zusammen eingewogen und unter Rühren mit 9.9 ml (126 mmol) Epichlorhydrin versetzt. Man lässt über Nacht rühren. Nach Hydrolyse und dreimaliger Extraktion mit Ethylacetat werden die vereinigten organischen Phasen dreimal mit Wasser gewaschen. Nach Trocknen der organischen Phase mit Natriumsulfat und Einengen des Lösungsmittels am Rotationsverdampfer verbleibt ein Feststoff, der durch Säulenchromatographie an Kieselgel (Fließmittel: Hexan/Ethylacetat 7:3) gereinigt wird.
Ausbeute: 3.34 g (17 mmol); 80 %
Schmp.: 92°C
C₁₂H₁₀N₂O (198.2)
¹H-NMR (CDCl₃): δ (ppm) = 2.29 (dd, J = 5 Hz und J = 3 Hz, 1H), 2.77 (m, 1 H), 3.22 (m, 1 H), 4.08 (dd, J = 15 Hz und J = 6 Hz, 1 H), 4.47 (dd, J = 15 Hz und J = 3 Hz, 1 H), 6.54 (d, J = 3 Hz, 1 H), 7.20 (d, J = 3 Hz, 1 H), 7.40 (m, 2H), 7.92 (s, 1 H)

### B. 1-[3-(4-Heptyloxyphenoxy)-2-hydroxypropyl]indol-5-carbonitril

0.13 g (2.47 mmol) Natriumhydrid als 60%ige Dispersion in Mineralöl werden unter Stickstoff in 10 ml absol. DMF suspendiert, 10 min bei Raumtemperatur gerührt und mit der Lösung von 0.5 g (2.39 mmol) 4-Heptyloxyphenol in 5 ml absol. DMF versetzt. Nach einstündigem Rühren gibt man eine Lösung von 475 mg (2.39 mmol) 1-Oxiranylmethylindol-5-carbonitril in 5 ml absol. DMF hinzu und lässt über Nacht bei Raumtemperatur rühren. Anschließend wird die Suspension mit halbgesättigter NaCl-Lösung hydrolysiert und dreimal mit Ethylacetat extrahiert. Die Extrakte werden mit gesättigter NaCl-Lösung gewaschen, über Natriumsulfat getrocknet und das Lösungsmittel abdestilliert. Der Rückstand wird säulenchromatographisch an Kieselgel gereingt (Fließmittel: Hexan/Ethylacetat 1:1), wobei das Produkt als Öl anfällt.
Ausbeute: 0.48 g (1.18 mmol); 49 %
C₂₅H₃₀N₂O₃ (406.5)
MS (El): m/z (%) = 406 (100) M⁺, 309 (20), 308 (92), 199 (62), 181 (25), 156 (36), 155 (60), 110 (69)

### C. 1-[3-(4-Heptyloxyphenoxy)-2-hydroxypropyl]indol-5-carbamid

455 mg (1.12 mmol) 1-[3-(4-Heptyloxyphenoxy)-2-hydroxypropyl]indol-5-carbonitril werden in 10 ml *tert*-Butanol gelöst. Nach Zugabe von 1.10 g (22.4 mmol) pulverisiertem, 88%-igem Kaliumhydroxid wird 3 Stunden auf 100°C erhitzt. Nach Abkühlung der Reaktionslösung wird mit 50 ml destilliertem Wasser hydrolysiert und mit 1 N Salzsäure neutralisiert. Die wässrige Phase wird dreimal mit Ethylacetat extrahiert und die vereinigten organischen Phasen einmal mit Wasser und anschließend zweimal mit gesättigter Natriumchloridlösung gewaschen. Nach dem Trocknen über Natriumsulfat wird das Lösungsmittel am Rotationsverdampfer entfernt. Der Rückstand wird säulenchromatographisch an Kieselgel gereingt (Fließmittel: Ethylacetat), wobei das Produkt als Feststoff anfällt.
Ausbeute: 245 mg (0.58 mmol); 52 %
Schmp.: 114 °C
C₂₅H₃₂N₂O₄ (424.5)
MS (El): m/z (%) = 424 (67) M⁺, 328 (21), 327 (100), 174 (18), 173 (69)

### D. 1-[3-(4-Heptyloxyphenoxy)-2-oxopropyl]indol-5-carbamid

Die Darstellung erfolgt ausgehend von 1-[3-(4-Heptyloxyphenoxy)-2-hydroxypropyl]indol-5-carbamid analog der Synthese der Stufe C von Beispiel 9. Davon abweichend beträgt die Reaktionszeit 20 h. Das Produkt wird aus dem Reaktionsansatz mittels Ethylacetat extrahiert. Die chromatographische Reinigung an Kieselgel erfolgt mit dem Fließmittel Ethylacetat. Das Produkt fällt nach Umkristallisieren aus Hexan/Ethylacetat als Feststoff an.
Schmp.: 157 °C
C₂₅H₃₀N₂O₄ (422.5)
¹H-NMR (DMSO-d₆): δ (ppm) = 0.82 (t, J = 7 Hz, 3H), 1.20-1.36 (m, 6H), 1.37-1.42 (m, 2H), 1.62 (quin, J = 7 Hz, 2H), 3.87 (t, J = 7 Hz, 2H), 4.93 (s, 2H), 5.39 (s, 2H), 6.54 (d, J = 3 Hz, 1 H), 6.82-6.93 (m, 4H), 7.21 (s, 1 H), 7.37 (d, J = 3 Hz, 1 H), 7.41 (d, J = 8 Hz, 1H), 7.66 (dd, J = 8 Hz und J = 2 Hz, 1 H), 7.82 (s, 1 H), 8.19 (d, J = 2 Hz, 1 H)

### Beispiel 27

### 1-[3-(Biphenyl-4-yloxy)-2-oxopropyl]indol-5-carbamid

### A. 1-[3-(Biphenyl-4-yloxy)-2-hydroxypropyl]indol-5-carbonitril

Unter Stickstoff werden 0.34 g (2.02 mmol) 4-Phenylphenol in 5 ml absol. THF gelöst und unter starkem Rühren tropfenweise mit 0.1 ml (1.0 mmol) *tert*-Butyllithium versetzt. Die Mischung wird 5 min bei Raumtemperatur gerührt. Anschließend wird die Lösung von 0.20 g (1.0 mmol) 1-Oxiranylmethylindol-5-carbonitril (Beispiel 26A) in 5 ml absol. THF zugetropft. Der Ansatz wird 24 Stunden bei 100°C erhitzt. Nach dem Abkühlen wird mit 100 ml Diethylether versetzt und dreimal mit Wasser extrahiert. Die wässrige Phase wird zweimal mit Ethylacetat extrahiert und die vereinigten organischen Phasen über Natriumsulfat getrocknet, filtriert und das Lösungsmittel im Vakuum entfernt. Der Rückstand wird säulenchromatographisch an Kieselgel gereinigt (Fließmittel: Hexan/Ethylacetat 7:3). Das Produkt wird als Feststoff erhalten.
Ausbeute: 0.37 g (1.0 mmol); 100%
Schmp.: 133 °C
C₂₄H₂₀N₂O₂ (368.4)
¹H-NMR (DMSO-d₆): δ (ppm) = 3.90-3.92 (m, 2H), 4.20 (m, 1H), 4.27 (dd, J = 15 Hz und J = 7 Hz, 1 H), 4.42 (dd, J = 15 Hz und J = 4 Hz, 1 H), 5.45 (d, J = 5 Hz, 1 H), 6.60 (d, J = 3 Hz, 1H), 7.04 (d, J = 9 Hz, 2H), 7.30 (d, J = 8 Hz, 1H), 7.40 (d, J = 8 Hz, 2H), 7.45 (d, J = 8 Hz, 2H), 7.58 (d, J = 3 Hz, 1H), 7.59 (d, J = 7 Hz, 1H), 7.62 (m, 2H), 7.72 (d, J = 8 Hz, 1H), 8.35 (s, 1 H)

### B. 1-[3-(Biphenyl-4-yloxy)-2-hydroxypropyl]indol-5-carbamid

Die Darstellung erfolgt ausgehend von 1-[3-(Biphenyl-4-yloxy)-2-hydroxypropyl]indol-5-carbonitril analog der Synthese der Stufe C von Beispiel 26. Das Produkt wird als Feststoff erhalten.
Schmp.: 155°C
C₂₄H₂₂N₂O₃ (386.4)
¹H-NMR (DMSO-d₆): δ (ppm) = 3.88-3.92 (m, 2H), 4.19 (s, breit, 1H), 4.27 (dd, J = 14 Hz und J = 7 Hz, 1H), 4.41 (dd, J = 14 Hz und J = 4 Hz, 1H), 5.48 (s, breit, 1 H), 6.51 (d, J = 3 Hz, 1 H), 7.02 (d, J = 9 Hz, 2H), 7.10 (s, breit, 1H), 7.28-7.32 (m, 1 H), 7.36-7.43 (m, 3H), 7.48-7.63 (m, 6H), 7.83 (s, breit, 1H), 8.17 (s, 1 H)

### C. 1-[3-(Biphenyl-4-yloxy)-2-oxopropyl]indol-5-carbamid

Die Darstellung erfolgt ausgehend von 1-[3-(Biphenyl-4-yloxy)-2-hydroxypropyl]indol-5-carbamid analog der Synthese der Stufe D von Beispiel 26. Das Produkt wird als Feststoff erhalten.
Schmp.: 120 °C
C₂₄H₂₀N₂O₃ (384.4)
¹H-NMR (DMSO-*d₆*): δ (ppm) = 5.09 (s, 2H), 5.21 (s, 2H), 6.57 (d, J = 3 Hz, 1H), 7.09 (d, J = 9 Hz, 2H), 7.11 (s, breit, 1H), 7.28-7.33 (m, 1H), 7.35 (d, J = 3 Hz, 1H), 7.38-7.45 (m, 3H), 7.57-7.70 (m, 4H), 7.85 (s, breit, 1 H), 8.17 (s, 1 H)

### Beispiel 28

### 1-[3-(Biphenyl-3-yloxy)-2-oxopropyl]indol-5-carbamid

### A. 1-[3-(Biphenyl-3-yloxy)-2-hydroxypropyl]indol-5-carbonitril

Die Darstellung erfolgt ausgehend von 3-Phenylphenol und 1-Oxiranylmethylindol-5-carbonitril analog der Synthese der Stufe A von Beispiel 27. Das Produkt fällt als Feststoff an.
Schmp.: 120 °C
C₂₄H₂₀N₂O₂ (368.4)
¹H-NMR (DMSO-*d*₆): δ (ppm) = 3.95 (m, 2H), 4.19 (m, 1H), 4.32 (dd, J = 14 Hz und J = 7 Hz, 1 H), 4.48 (dd, J = 14 Hz und J = 4 Hz, 1H), 5.49 (d, J = 5 Hz, 1H), 6.61 (d, J = 3 Hz, 1 H), 6.94 (m, 1 H), 7.18 (d, J = 2 Hz, 1H), 7.24 (d, J = 8 Hz, 1 H), 7.37 (m, 2H), 7.42-7.51 (m, 3H), 7.58 (d, J = 3 Hz, 1 H), 7.65 (m, 2H), 7.72 (d, J = 8 Hz, 1 H), 8.09 (s, 1 H)

### B. 1-[3-(Biphenyl-3-yloxy)-2-hydroxypropyl]indol-5-carbamid

Die Darstellung erfolgt ausgehend von 1-[3-(Biphenyl-3-yloxy)-2-hydroxypropyl]indol-5-carbonitril analog der Synthese der Stufe C von Beispiel 26. Das Produkt wird als Feststoff erhalten.
Schmp.: 162 °C
C₂₄H₂₂N₂O₃ (386.4)
¹H-NMR (DMSO-*d₆*): δ (ppm) = 3.95 (m, 2H), 4.20 (s, breit, 1H), 4.29 (dd, J = 15 Hz und J =
7 Hz, 1H), 4.42 (dd, J = 15 Hz und J = 4 Hz, 1H), 5.43 (s, breit, 1 H), 6.45 (d, J = 3 Hz, 1H), 6.97 (dd, J = 8 Hz und J = 2 Hz, 2H), 7.17 (s, breit, 1 H), 7.18 (d, J = 2 Hz, 1 H), 7.22 (d, J = 8 Hz, 1H), 7.31-7.39 (m, 2H), 7.41-7.51 (m, 3H), 7.60-7.69 (m, 3H), 7.84 (s, breit, 1 H), 8.12 (s, 1 H)

### C. 1-[3-(Biphenyl-3-yloxy)-2-oxopropyl]indol-5-carbamid

Die Darstellung erfolgt ausgehend von 1-[3-(Biphenyl-3-yloxy)-2-hydroxypropyl]indol-5-carbamid analog der Synthese der Stufe D von Beispiel 26. Die chromatographische Reinigung erfolgt zunächst an Kieselgel mit dem Fließmittel Hexan/Ethylacetat 3:7 und danach an einer RP-HPLC-Säule (Kromasil) mit dem Fließmittel Acetonitril/Wasser/ Ameisensäure (70:30:0.02). Das Produkt fällt als Feststoff an.
C₂₄H₂₀N₂O₃ (384.4)
¹H-NMR (DMSO-*d₆*): δ (ppm) = 5.18 (s, 2H), 5.44 (s, 2H), 6.58 (d, J = 3 Hz, 1 H), 6.98 (dd, J = 8 Hz und J = 3 Hz, 1H), 7.18 (s, breit, 1H), 7.20-7.25 (m, 2H), 7.25-7.45 (m, 6H), 7.60-7.71 (m, 3H), 7.83 (s, breit, 1H), 8.18 (s, 1 H)

### Beispiel 29

### 1-[3-(Biphenyl-2-yloxy)-2-oxopropyl]indol-5-carbamid

### A. 1-[3-(Biphenyl-2-yloxy)-2-hydroxypropyl]indol-5-carbonitril

Die Darstellung erfolgt ausgehend von 2-Phenylphenol und 1-Oxiranylmethylindol-5-carbonitril analog der Synthese der Stufe A von Beispiel 27. Das Produkt fällt als Feststoff an.
Schmp.: 117°C
C₂₄H₂₀N₂O₂ (368.4)
¹H-NMR (DMSO-*d*₆): δ (ppm) = 3.84 (dd, J = 10 Hz und J = 6 Hz, 1H), 3.94 (dd, J = 10 Hz und J = 4 Hz, 1 H), 4.01-4.08 (m, 1 H), 4.09-4.13 (m, 1 H), 4.29-4.33 (m, 1H), 5.41 (d, J = 5 Hz, 1H), 6.57 (d, J = 3 Hz, 1H), 7.01-7.11 (m, 2H), 7.21-7.39 (m, 6H), 7.43-7.51 (m, 2H), 7.61 (d, J = 7 Hz, 1 H), 8.03 (s, 1 H)

### B. 1-[3-(Biphenyl-2-yloxy)-2-hydroxypropyl]indol-5-carbamid

Die Darstellung erfolgt ausgehend von 1-[3-(Biphenyl-2-yloxy)-2-hydroxypropyl]indol-5-carbonitril analog der Synthese der Stufe C von Beispiel 26. Das Produkt wird als Feststoff erhalten.
Schmp.: 150 °C
C₂₄H₂₂N₂O₃ (386.4)
¹H-NMR (DMSO-*d₆*): δ (ppm) = 3.85 (dd, J = 9 Hz und J = 6 Hz, 1 H), 3.92 (dd, J = 9 Hz und J = 4 Hz, 1H), 4.00-4.10 (m, 2H), 4.20-4.30 (m, 1H), 5.38 (d, J = 4 Hz, 1H), 6.45 (d, J = 3 Hz, 1H), 6.98-7.12 (m, 4H), 7.23 (d, J = 3 Hz, 1H), 7.25-7.50 (m, 5H), 7.52-7.61 (m, 3H), 7.81 (s, breit, 1 H), 8.11 (s, 1 H)

### C. 1-[3-(Biphenyl-2-yloxy)-2-oxopropyl]indol-5-carbamid

Die Darstellung erfolgt ausgehend von 1-[3-(Biphenyl-2-yloxy)-2-hydroxypropyl]indol-5-carbamid analog der Synthese der Stufe D von Beispiel 26. Das Produkt fällt als Feststoff an.
C₂₄H₂₀N₂O₃ (384.4)
¹H-NMR (DMSO-d₆): δ (ppm) = 5.04 (s, 2H), 5.32 (s, 2H), 6.52 (d, J = 3 Hz, 1H), 7.01-7.83 (m, 13H), 7.91 (s, breit, 1H), 8.18 (d, J = 1 Hz, 1 H)

### Beispiel 30

### 1-[3-(1-Heptylindol-5-yloxy)-2-oxopropyl]indol-5-carbamid

### A. 5-Benzyloxy-1-heptylindol

0.22 g (4.45 mmol) pulverisiertes, 88%-iges Kaliumhydroxid, 0.5 g (2.24 mmol) 5-Benzyloxyindol und 0.07 g (0.22 mmol) Tetrabutylammoniumbromid werden in der angegebenen Reihenfolge eingewogen und unter starkem Rühren mit 1.41 ml (8.96 mmol) 1-Bromheptan versetzt. Die Mischung wird über Nacht bei Raumtemperatur gerührt. Nach Zusatz von 30 ml Wasser wird dreimal mit Diethylether extrahiert. Die organische Phase wird dreimal mit gesättigter NaCl-Lösung gewaschen über Natriumsulfat getrocknet und das Lösungsmittel im Vakuum entfernt. Der Rückstand wird an Kieselgel säulenchromatographisch gereinigt (Fließmittel: Hexan/Ethylacetat 95:5). Das Produkt fällt als Öl an.
Ausbeute: 530 mg (0.16 mmol), 74 %
¹H-NMR (CDCl₃): δ (ppm) = 0.80 (t, J = 7 Hz, 3H), 1.13-1.27 (m, 8H), 1.75 (quin, J = 7 Hz, 2H), 4.00 (t, J = 7 Hz, 2H), 5.05 (m, 2H), 6.31 (d, J = 3 Hz, 1H), 6.87 (dd, J = 9 Hz und J = 3 Hz, 1H), 6.98 (d, J = 3 Hz, 1 H), 7.08 (d, J = 3 Hz, 1 H), 7.16 (d, J = 9 Hz, 1H), 7.24 (t, J = 7 Hz, 1 H), 7.31 (m, 2H), 7.41 (d, J = 7 Hz, 2H)

### B. 1-Heptylindol-5-ol

0.5 g (1.55 mmol) 5-Benzyloxy-1-heptylindol werden in einem Schlenkkolben in 10 ml Methanol und 4 ml Dichlormethan gelöst. Dazu gibt man 0.06 g Pd (10 %)/C und spült 15 Minuten mit Stickstoff. Anschließend wird ein mit Wasserstoff gefüllter Luftballon auf den Schlenkkolben gesetzt und 12 h bei Raumtemperatur hydriert. Der Ansatz wird über eine Fritte filtriert und das Lösungsmittel im Vakuum entfernt. Der Rückstand wird über Kieselgel säulenchromatographisch gereinigt (Fließmittel: Hexan/Ethylacetat 9:1). Das Produkt fällt als Öl an.
Ausbeute: 0.33 g (1.43 mmol); 90 %
C₂₄H₂₀N₂O₃ (384.4)
¹H-NMR (DMSO-*d₆*): δ (ppm) = 0.82 (t, J = 7 Hz, 3H), 1.21 (m, 8H), 1.72 (quin, J = 7 Hz, 2H), 4.05 (t, J = 7 Hz, 2H), 6.18 (d, J = 3 Hz, 1H), 6.61 (dd, J = 9 Hz und J = 2 Hz, 1H), 6.82 (d, J = 2 Hz, 1 H), 7.20 (m, 2H), 8.63 (s, 1 H).

### C. 1-[3-(1-Heptylindol-5-yloxy)-2-hydroxypropyl]indol-5-carbonitril

Die Darstellung erfolgt ausgehend von 1-Heptylindol-5-ol und 1-Oxiranylmethylindol-5-carbonitril analog der Synthese der Stufe B von Beispiel 26. Die chromatographische Reinigung an Kieselgel erfolgt mit dem Fließmittel Hexan/Ethylacetat 4:1. Das Produkt fällt als Öl an.
C₂₇H₃₁N₃O₂ (429.6)
¹H-NMR (DMSO-d₆): δ (ppm) = 0.83 (t, J = 7 Hz, 3H), 1.13-1.28 (m, 8H), 1.71 (quin, J = 7 Hz, 2H), 3.81-3.91 (m, 1H), 4.02 (m, 1H), 4.08 (t, J = 7 Hz, 2H), 4.15 (s, breit, 1 H), 4.31 (dd, J = 14 Hz und J = 7 Hz, 1 H), 4.46 (dd, J = 14 Hz und J = 4 Hz, 1 H), 5.41 (s, breit, 1 H), 6.28 (d, J = 3 Hz, 1H), 6.58 (d, J = 3 Hz, 1H), 6.80 (dd, J = 9 Hz und J = 2 Hz, 1 H), 6.95 (d, J = 2 Hz, 1 H), 7.28 (d, J = 3 Hz, 1 H), 7.34 (d, J = 9 Hz, 1H), 7.42 (dd, J = 9 Hz und J = 2 Hz, 1 H), 7.44 (d, J = 3 Hz, 1 H), 7.68 (d, J = 9 Hz, 1H), 8.06 (d, J = 2 Hz, 1 H)

### D. 1-[3-(1-Heptylindol-5-yloxy)-2-hydroxypropyl]indol-5-carbamid

Die Darstellung erfolgt aus gehend von 1-[3-(1-Heptylindol-5-yloxy)-2-hydroxypropyl]indol-5-carbonitril analog der Synthese der Stufe C von Beispiel 26. Das Produkt fällt als Feststoff an.
Schmp.: 114°C
C₂₇H₃₃N₃O₃ (447.6)
¹H-NMR (DMSO-*d*₆): δ (ppm) = 0.83 (t, J = 7 Hz, 3H), 1.13-1.28 (m, 8H), 1.71 (quin, J = 7 Hz, 2H), 3.81-3.91 (m, 1H), 4.02 (q, J = 7 Hz, 1H), 4.08 (t, J = 7 Hz, 2H), 4.15 (m, 1H), 4.31 (dd, J = 14 Hz und J = 7 Hz, 1H), 4.46 (dd, J = 14 Hz und J = 4 Hz. 1H), 5.40 (d, J = 5 Hz, 1 H), 6.28 (d, J = 3 Hz, 1H), 6.48 (d, J = 3 Hz, 1 H), 6.80 (dd, J = 9 Hz und J = 2 Hz, 1H), 6.95 (d, J = 2 Hz, 1 H), 7.08 (s, breit, 1 H), 7.28 (d, J = 3 Hz, 1 H), 7.34 (d, J = 9 Hz, 1 H), 7.42 (dd, J = 9 Hz und J = 2 Hz, 1H), 7.44 (d, J = 3 Hz, 1 H), 7.68 (d, J = 9 Hz, 1 H), 7.81 (s, breit, 1H), 8.12 (d, J = 2 Hz, 1 H)

### E. 1-[3-(1-Heptylindol-5-yloxy)-2-oxopropyl]indol-5-carbamid

Die Darstellung erfolgt ausgehend von 1-[3-(1-Heptylindol-5-yloxy)-2-hydroxypropyl]indol-5-carbamid analog der Synthese der Stufe C von Beispiel 9. Davon abweichend beträgt die Reaktionszeit 20 h. Das Produkt wird aus dem Reaktionsansatz mittels Ethylacetat extrahiert. Die chromatographische Reinigung an Kieselgel erfolgt mit dem Fließmittel Ethylacetat. Das Produkt fällt nach Umkristallisieren aus Hexan/Ethylacetat als Feststoff an. Schmp.: 171 °C
C₂₇H₃₁N₃O3 (445.6)
¹H-NMR (DMSO-*d*₆): δ (ppm) = 0.83 (t, J = 7 Hz, 3H), 1.13 - 1.28 (m, 8H), 1.71 (quin, J = 7 Hz, 2H), 4.08 (t, J = 7 Hz, 2H), 4.97 (s, 2H), 5.42 (s, 2H), 6.28 (d, J = 3 Hz, 1H), 6.48 (d, J = 3 Hz, 1H), 6.80 (dd, J = 9 Hz und J = 2 Hz, 1 H), 6.95 (d, J = 2 Hz, 1H), 7.12 (s, breit, 1 H), 7.28 (d, J = 3 Hz, 1 H), 7.34 (d, J = 9 Hz, 1H), 7.42 (dd, J = 9 Hz und J 0 2 Hz, 1 H), 7.44 (d, J = 3 Hz, 1 H), 7.68 (d, J = 9 Hz, 1 H), 7.82 (s, breit, 1 H), 8.12 (d, J = 2 Hz, 1 H)

### Beispiel 31

### Pharmakologische Testung

Die Wirksamkeit der erfindungsgemäßen Verbindungen lässt sich anhand der Hemmung der cytosolischen Phospholipase A₂ bestimmen. Die verwendete Testmethode wurde bereits beschrieben (Lehr M. et al., *Arch. Pharm. Pharm. Med. Chem.* 2000, *333*, 312-314). Hierbei wird die durch die cytosolische Phospholipase A₂ in intakten menschlichen Blutplättchen nach Stimulation mit Calcium lonophor A23187 gebildete Arachidonsäure in An- und Abwesenheit von Testsubstanzen erfasst.

Mit diesem Testsystem wurden die erfindungsgemäßen Verbindungen der Beispiele 2, 5, 6, 7, 8 und 9 getestet. Bei einer Konzentration von 10 µM hemmten diese die Aktivität der cytosolischen Phospholipase A₂ um 40% bis 95%, was ihre Wirksamkeit belegt.

## Patentansprüche

1. Verbindungen der Formel I 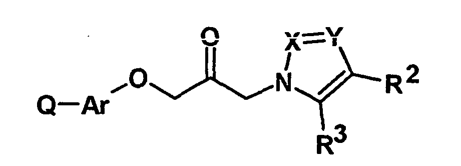 worin
Q für R¹ OR¹, SR¹, SOR¹ SO₂R¹, NR⁹R¹ oder einen geradkettigen C₁₋₃₁-Alkyl- oder C₂₋₃₁-Alkenyl- oder -Alkinylrest steht, der mit 1 oder 2 Resten, unabhängig ausgewählt aus O, S, SO, SO₂, NR⁹ und Aryl, das mit 1 oder 2 Substituenten R⁴ substituiert sein kann, unterbrochen sein kann, und der mit 1-4 C₁₋₆₋Alkylresten und/oder 1 oder 2 Arylresten substituiert sein kann, wobei die Arylreste mit 1 oder 2 Substituenten R⁴ substituiert sein können;
Ar für einen Arylrest steht, der mit 1 oder 2 Substituenten R⁴ substituiert sein kann;
X für N oder CR⁵ steht;
Y für N oder CR⁶ steht;
R¹ für H oder einen Arylrest steht, der mit 1 oder 2 Substituenten R⁴ substituiert sein kann;
R² und R³
a) unabhängig voneinander für H, C₁₋₆-Alkyl, C₂₋₆-Alkenyl, C₂₋₆-Alkinyl oder R⁷-W stehen,
oder
b) zusammen mit den Kohlenstoffatomen, an die sie gebunden sind, für einen 5- oder 6-gliedrigen aromatischen oder heteroaromatischen Ring stehen, der mit 1 oder 2 Substituenten R⁴ substituiert sein kann;
R⁴ für C₁₋₆Alkyl, Halogen, CF₃, CN, NO₂, OR⁹, S(O)ₒR⁹, COR⁹, COOR⁹, CONR⁹R¹⁰, SO₃R⁹, SO₂NR⁹R¹⁰,Tetrazolyl oder R⁷-W steht;
R⁵ für H oder R⁴ steht;
R⁶ für H, C₁₋₆-Alkyl, Halogen, CF₃, CN, NO₂, OR⁹, S(O)ₒR⁹, COR⁹, COOR⁹, CONR⁹R¹⁰, SO₃R⁹, SO₂NR⁹R¹⁰,Tetrazolyl oder R⁸-W steht;
R⁷ für C₁₋₆Alkyl, C₂-₆-Alkeny) oder C₂₋₆-Alkinyl steht;
R⁸ für C₂₋₆Alkyl , C₂₋₆Alkenyl oder C₂₋₆Alkinyl steht;
R⁹ für H, C₁₋₆-Alkyl oder Aryl steht;
R¹⁰ für H oder C₁₋₆-Alkyl steht;
W für COOH, SO₃H oder Tetrazolyl steht; und
und o für 0, 1 oder 2 steht; worin Aryl Phenyl, Naphthyl, Biphenyl sowie 5- oder 6- gliedrige heterocyclische Ringe, die 1 bis 3 Atome ausgewählt aus O, N oder S enthalten umfasst ;
sowie deren pharmazeutisch verträglichen Salze und Ester.

2. Verbindungen nach Anspruch 1, worin Q für
R¹-(CHR¹¹)ₚ-A-Z²-B-Z¹-
steht, worin
A für eine Bindung oder einen geradkettigen C₁₋ₘ-Alkyl- oder C₂₋ₘ-Alkenyl- oder -Alkinylrest steht;
B für eine Bindung oder einen geradkettigen C₁₋ₙ-Alkyl- oder C₂₋ₙ-Alkenyl- oder -Alkinylrest steht;
R¹¹ für H oder einen Arylrest steht, der mit 1 oder 2 Substituenten R⁴ substituiert sein kann;
Z¹ und Z² unabhängig voneinander für eine Bindung, O, S, SO, SO₂, NR⁹, CR⁹R¹⁰ oder einen Arylrest stehen, wobei der Arylrest mit 1 oder 2 Substituenten R⁴ substituiert sein kann;
R¹, R⁴, R⁹ und R¹⁰ wie in Anspruch 1 definiert sind;
p für 0 oder 1 steht;
m für eine ganze Zahl von 0 bis 12 steht;
und
n für eine ganze Zahl von 0 bis 16 steht.

3. Verbindungen nach Anspruch 2, worin m + n ≤ 17 ist.

4. Verbindungen nach Anspruch 2 oder 3, worin Q ausgewählt ist aus
R¹-B-Z¹-
R¹-CHR¹¹-B-Z¹-
R¹-A-Z²-B-Z¹- und
R¹-CHR¹¹-A-Z²-B-Z¹-
und A, B, R¹, R¹¹, Z¹, Z², n und m wie in Anspruch 2 definiert sind.

5. Verbindungen nach Anspruch 4, worin Q für Phenyl oder C₅₋₁₂-Alkyl oder -Alkoxy, vorzugsweise C₇₋₁₀-Alkyl oder -Alkoxy steht.

6. Verbindungen nach einem der vorhergehenden Ansprüche, worin Ar für einen Phenyl- oder Indolylrest steht.

7. Verbindungen nach einem der vorhergehenden Ansprüche, worin R² und R³
a) unabhängig voneinander für H, C₁₋₆-Alkyl, C₂₋₆-Alkenyl, C₂-₆-Alkinyl oder R⁷-W stehen oder
b) zusammen mit den Kohlenstoffatomen, an die sie gebunden sind, einen Benzo-Ring oder einen 6-gliedrigen aromatischen heterocyclischen Ring mit 1-3 Stickstoffatomen bilden, wobei der Benzo-Ring oder der heterocyclische Ring mit 1 oder 2 Substituenten R⁴ substituiert sein kann, und R⁴ wie in Anspruch 1 definiert ist.

8. Verbindungen nach Anspruch 7, worin R² und R³ für H stehen oder zusammen mit den Kohlenstoffatomen, an die sie gebunden sind, einen Benzo-Ring bilden, der mit einem Substituenten R⁴, vorzugsweise COOH, CH₃, Cl, OCH₃, CN, CHO, COOCH₃ oder CONH₂ substituiert sein kann.

9. Verbindungen nach einem der vorhergehenden Ansprüche, worin X = CR⁵ und Y = CR⁶ ist oder X = N und Y = CR⁶ ist, wobei R⁵ und R⁶ wie in Anspruch 1 definiert sind und vorzugsweise für H, COOH, t-Butyl, Cl, CHO, COCH₃ oder COOCH₃ stehen.

10. Pharmazeutisches Mittel umfassend eine Verbindung der allgemeinen Formel I nach einem der Ansprüche 1-9 oder ein pharmazeutisch verträgliches Salz oder Ester davon.

11. Verwendung einer Verbindung der Formel I nach einem der Ansprüche 1-9 oder einem pharmazeutisch verträglichen Salz oder Ester davon zur Herstellung eines pharmazeutischen Mittels zur Prävention oder Behandlung von Erkrankungen, die durch eine erhöhte Aktivität der Phospholipase A₂ verursacht oder mitverursacht werden.

12. Verwendung nach Anspruch 11, worin die Erkrankung ausgewählt ist aus Entzündungen, Schmerz, Fieber, Allergien, Asthma, Psoriasis, cerebrale Ischämie, Alzheimersche Erkrankung, chronische Hauterkrankungen, Schädigung der Haut durch UV-Strahlen, rheumatische Erkrankungen, Thrombose, anaphylaktischer Schock, Urtikaria, akute und chronische Exantheme und Endotoxinschock.

13. Verfahren zur Herstellung einer Verbindung der Formel I nach Anspruch 1, worin eine Verbindung der Formel II 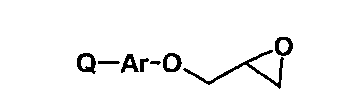 oder eine Verbindung der Formel III 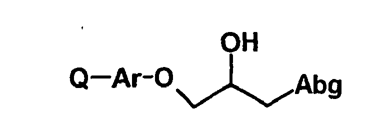 mit einer Verbindung der Formel IV 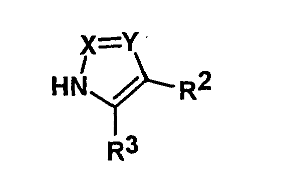 umgesetzt und der gebildete Alkohol zum gewünschten Keton oxidiert wird, wobei Q, Ar, X, Y, R² und R³ wie in Anspruch 1 definiert sind und Abg für eine Abgangsgruppe wie Halogen, insbesondere Brom steht.

## Claims

1. Compounds of formula I 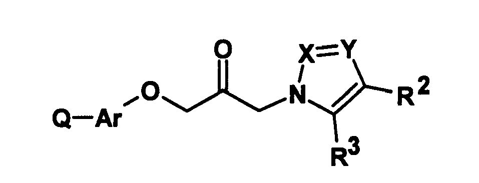 wherein
Q represents R¹, OR¹, SR¹, SOR¹, SO₂R¹, NR⁹R¹ or a straight-chain C₁₋₃₁ alkyl or C₂₋₃₁ alkenyl or alkynyl residue which may be interrupted by 1 or 2 residues, independently selected from O, S, SO, SO₂, NR⁹ and aryl which may be substituted with 1 or 2 substituents R⁴, and which may be substituted with 1-4 C₁₋₆ alkyl residues and/or 1 or 2 aryl residues, wherein the aryl residues may be substituted with 1 or 2 substituents R⁴;
Ar represents an aryl residue which may be substituted with 1 or 2 substituents R⁴;
X represents N or CR⁵;
Y represents N or CR⁶;
R¹ represents H or an aryl residue which may be substituted with 1 or 2 substituents R⁴;
R² and R³
a) independently may represent H, C₁₋₆ alkyl, C₂₋₆ alkenyl, C₂₋₆ alkynyl or R⁷-W,
or
b) together with the carbon atoms to which they are bound, for a 5- or 6-membered aromatic or heteroaromatic ring which may be substituted with 1 or 2 substituents R⁴;
R⁴ represents C₁₋₆ alkyl, halogen, CF₃, CN, NO₂, OR⁹, S(O)ₒR⁹, COR⁹, COOR⁹, CONR⁹R¹⁰, SO₃R⁹, SO₂NR⁹R¹⁰, tetrazolyl or R⁷-W;
R⁵ represents H or R⁴;
R⁶ represents H, C₁₋₆ alkyl, halogen, CF₃, CN, NO₂, OR⁹, S(O)ₒR⁹, COR⁹, COOR⁹, CONR⁹R¹⁰, SO₃R⁹, SO₂NR⁹R¹⁰, tetrazolyl or R⁸-W;
R⁷ represents C₁₋₆ alkyl, C₂₋₆ alkenyl or C₂₋₆ alkynyl;
R⁸ represents C₂₋₆ alkyl, C₂₋₆ alkenyl or C₂₋₆ alkynyl;
R⁹ represents H, C₁₋₆ alkyl or aryl;
R¹⁰ represents H or C₁₋₆ alkyl;
W represents COOH, SO₃H or tetrazolyl; and
o represents 0, 1 or 2;
wherein aryl comprises phenyl, naphthyl, biphenyl and 5- or 6-membered heterocyclic rings which comprise 1 to 3 atoms selected from O, N or S;
and the pharmaceutically compatible salts and esters thereof.

2. Compounds according to claim 1, wherein Q represents
R¹-(CHR¹¹)ₚ-A-Z²-B-Z¹-
wherein
A represents a bond or a straight-chain C₁₋ₘ alkyl residue or C₂₋ₘ alkenyl or alkynyl residue;
B represents a bond or a straight chain C₁₋ₙ alkyl residue or C₂₋ₙ alkenyl or alkynyl residue;
R¹¹ represents H or an aryl residue which may be substituted with 1 or 2 substituents R⁴;
Z¹ and Z² independently represent a bond, O, S, SO, SO₂, NR⁹, CR⁹R¹⁰ or an aryl residue, wherein the aryl residue may be substituted with 1 or 2 substituents R⁴;
R¹, R⁴, R⁹ and R¹⁰ are as defined in claim 1;
p represents 0 or 1;
m represents an integer from 0 to 12;
and
n represents an integer from 0 to 16.

3. Compounds according to claim 2, wherein m + n ≤ 17.

4. Compounds according to claim 2 or 3, wherein Q is selected from
R¹-B-Z¹-
R¹-CHR¹¹-B-Z¹-
R¹-A-Z²-B-Z¹- and
R¹-CHR¹¹-A-Z² -B-Z¹-
and A, B, R¹, R¹¹, Z¹, Z² , n and m are as defined in claim 2.

5. Compounds according to claim 4, wherein Q represents phenyl or C₅₋₁₂ alkyl or alkoxy, preferably C₇₋₁₀ alkyl or alkoxy.

6. Compounds according to any of the preceding claims, wherein Ar represents a phenyl or indolyl residue.

7. Compounds according to any of the preceding claims, wherein R² and R³
a) independently represent H, C₁₋₆ alkyl, C₂₋₆ alkenyl, C₂₋₆ alkynyl or R⁷-W or
b) together with the carbon atoms to which they are bound form a benzo ring or a 6-membered aromatic heterocyclic ring having 1-3 nitrogen atoms,
wherein the benzo ring or the heterocyclic ring may be substituted with 1 or 2 substituents R⁴, and R⁴ is defined as in claim 1.

8. Compounds according to claim 7, wherein R² and R³ represent H or together with the carbon atoms to which they are bound form a benzo ring which may be substituted with a substituent R⁴, preferably COOH, CH₃, Cl, OCH₃, CN, CHO, COOCH₃ or CONH₂.

9. Compounds according to any of the preceding claims, wherein X = CR⁵ and Y = CR⁶ or X = N and Y = CR⁶, wherein R⁵ and R⁶ are as defined in claim 1 and preferably represent H, COOH, t-butyl, Cl, CHO, COCH₃ or COOCH₃.

10. Pharmaceutical preparation comprising a compound of general formula I according to any of claims 1 to 9 or a pharmaceutically compatible salt or ester thereof.

11. Use of a compound of formula I according to any of claims 1 to 9 or a pharmaceutically compatible salt or ester thereof for producing a pharmaceutical preparation for preventing or treating diseases which are caused or contributorily caused by an increased activity of phospholipase A₂.

12. Use according to claim 11, wherein the disease is selected from inflammations, pain, fever, allergies, asthma, psoriasis, cerebral ischemia, Alzheimer's disease, chronic skin diseases, damage to the skin by U.V. rays, rheumatic diseases, thrombosis, anaphylactic shock, urticaria, acute and chronic exanthemas and endotoxic shock.

13. A method of producing a compound of formula I according to claim 1,
wherein a compound of formula II 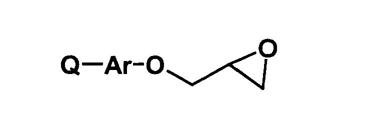 or a compound of formula III 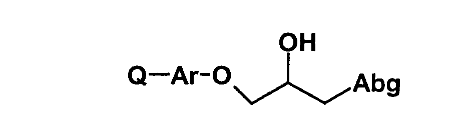 is reacted with a compound of formula IV 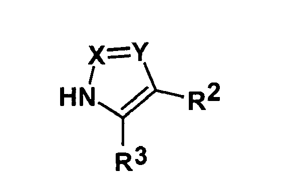 and the alcohol formed is oxidized into the desired ketone, wherein Q, Ar, X, Y, R² and R³ are as defined in claim 1 and Abg represents a leaving group, such as halogen, in particular bromine.

## Revendications

1. Composés de la formule I 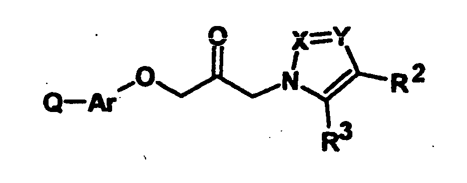 dans laquelle
Q représente R¹, OR¹, SR¹, SOR¹, SO₂R¹, NR⁹R¹ ou un radical alkyle en C₁ à C₃₁, ou alcényle ou alcynyle en C₂ à C₃₁, à chaîne linéaire, qui peut être interrompu par un ou deux radicaux choisis indépendamment parmi O, S, SO, SO₂, NR⁹ et aryle, qui peut être substitué par 1 ou 2 substituants R⁴, et qui peut être substitué par 1 à 4 radicaux alkyle en C₁ à C₆ et / ou 1 ou 2 radicaux aryle, les radicaux aryle pouvant être substitués par 1 ou 2 substituants R⁴,
Ar représente un radical aryle, qui peut être substitué par 1 ou 2 radicaux R⁴,
X représente N ou CR⁵,
Y représente N ou CR⁶,
R¹ représente H ou un radical aryle, qui peut être substitué par 1 ou 2 substituants R⁴,
R² et R³
a) représentent indépendamment l'un de l'autre H ou un radical alkyle en C₁ à C₆, alcényle en C₂ à C₆, alcynyle en C₂ à C₆ ou R⁷-W, ou bien
b) représentent ensemble avec l'atome de carbone auquel ils sont liés un noyau aromatique ou hétéroaromatique à 5 ou 6 membres, qui peut être substitué par 1 ou 2 substituants R⁴,
R⁴ représente un radical alkyle en C₁ à C₆, un halogène, CF₃, CN, NO₂, OR⁹, S(O)ₒR⁹, COR⁹, COOR⁹, CONR⁹R¹⁰, SO₃R⁹, SO₂NR⁹R¹⁰, tétrazolyle ou R⁷-W,
R⁵ représente H ou R⁴
R⁶ représente H ou un radical alkyle en C₁ à C₆, halogène, CF₃, CN, NO₂, OR⁹, S(O)ₒR⁹, COR⁹, COOR⁹, CONR⁹R¹⁰, SO₃R⁹, SO₂NR⁹R¹⁰, tétrazolyle ou R⁸-W,
R⁷ représente un radical alkyle en C₁ à C₆, alcényle en C₂ à C₆ ou alcynyle en C₂ à C₆,
R⁸ représente un radical alkyle en C₂ à C₆, alcényle en C₂ à C₆ ou alcynyle en C₂ à C₆,
R⁹ représente H ou un radical alkyle en C₁ à C₆, ou aryle,
R¹⁰ représente H ou un radical alkyle en C₁ à C₆,
W représente COOH, SO₃H ou tétrazolyle, et
o vaut 0, 1 ou 2,
où aryle couvre des radicaux phényle, naphtyle, biphényle ainsi que des noyaux hétérocycliques à 5 ou 6 membres, contenant de 1 à 3 atomes d'azote,
ainsi que leur sels et esters pharmaceutiquement compatibles.

2. Composés suivant la revendication 1, où Q représente
R¹-(CHR¹¹)ₚ-A-Z²-B-Z¹
dans lequel
A représente une liaison ou un radical alkyle en C₁ à Cₘ ou alcényle ou alcynyle en C₂ à Cₘ, à chaîne linéaire,
B représente une liaison ou un radical alkyle en C₁ à Cₙ ou alcényle ou alcynyle en C₂ à Cₙ, à chaîne linéaire,
R¹¹ représente H ou un radical aryle, qui peut être substitué par 1 ou 2 substituants R⁴,
Z¹ et Z² représentent indépendamment l'un de l'autre une liaison, O, S, SO, SO₂ NR⁹, CR⁹R¹⁰ ou un radical aryle, le radical aryle pouvant être substitué par 1 ou 2 substituants R⁴
R¹, R⁴, R⁹ étant comme définis dans la revendication 1,
p vaut 0 ou 1,
m est un nombre entier de 0 à 12,
et
n est un nombre entier de 0 à 16.

3. Composés suivant la revendication 2, dans lesquels m + n est inférieur ou égal à 17.

4. Composé suivant la revendication 2 ou 3, dans lequel Q est choisi parmi
R¹-B-Z¹
R¹-CHR¹¹-B-Z¹-
R¹-A-B-Z¹- et
R¹-CHR¹¹-A-Z²-B-Z¹-
et A, B, R¹, R¹¹, Z¹, Z², n et m sont tels que définis dans la revendication 2.

5. Composés suivant la revendication 4, où Q représente un radical phényle ou alkyle ou alcoxy en C₅ à C₁₂, de préférence alkyle ou alcoxy en C₇ à C₁₀.

6. Composés suivant l'une quelconque des revendications qui précèdent, dans lesquels Ar représente un radical phényle ou indolyle.

7. Composés suivant l'une quelconque des revendications qui précèdent, dans lesquels R² et R³
a) représentent indépendamment l'un de l'autre H ou un radical alkyle en C₁ à C₆, alcényle en C₂ à C₆, alcynyle en C₂ à C₆ ou R⁷-W ou
b) forment ensemble avec l'atome de carbone auquel ils sont liés un noyau benzénique ou un noyau aromatique hétérocyclique à 6 membres comportant de 1 à 3 atomes d'azote, le noyau benzénique ou le noyau hétérocyclique pouvant être substitué par 1 ou 2 substituants R⁴, et R⁴ étant défini comme dans la revendication 1.

8. Composés suivant la revendication 7, dans lesquels R² et R³ représentent H ou forment ensemble avec l'atome de carbone auquel ils sont liés un noyau benzénique qui peut être substitué par un substituant R⁴, de préférence COOH, CH₃, Cl, OCH₃, CN, CHO, COOCH₃ ou CONH₂.

9. Composés suivant l'une quelconque des revendications qui précèdent, dans lesquels X = CR⁵ et Y = CR⁶, ou bien X = N et Y = CR⁶, où R⁵ et R⁶ sont tels que définis dans la revendication 1 et représentent de préférence H, COOH, t-butyle, Cl, CHO, COCH₃ ou COOCH₃.

10. Agent pharmaceutique comprenant un composé de la formule générale I suivant l'une quelconque des revendications 1 à 9, ou un sel ou ester de celui-ci pharmaceutiquement compatible.

11. Utilisation d'un composé de la formule I suivant l'une quelconque des revendications 1 à 9 ou d'un sel ou ester de celui-ci pharmaceutiquement compatible pour la préparation d'un agent pharmaceutique destiné à la prévention ou au traitement de maladies causées par ou associées à une augmentation d'activité de la phospholipase A2.

12. Utilisation suivant la revendication 11, dans laquelle la maladie est choisie parmi des inflammations, des douleurs, de la fièvre, des allergies, de l'asthme, du psoriasis, de l'ischémie cérébrale, la maladie d'Alzheimer, des maladies cutanées chroniques, des endommagements de la peau par des rayons UV, des maladies rhumatismales, des thromboses, des chocs anaphylactiques, de l'urticaire, des exanthèmes aigus et chroniques et des chocs endotoxiniques.

13. Procédé de préparation d'un composé de la formule I suivant la revendication 1, dans lequel on fait réagir un composé de la formule II 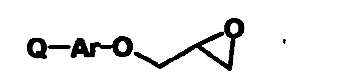 ou un composé de la formule III 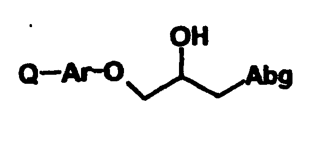 avec un composé de la formule IV 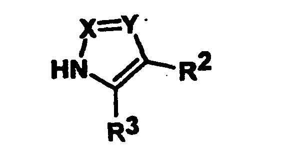 et on oxyde l'alcool formé en la cétone souhaitée, dans laquelle Q, Ar, X, Y, R² et R³ sont tels que définis dans la revendication 1, et Abg représente un groupe partant comme un halogène, en particulier du brome.
